# EUROPEAN PATENT APPLICATION

(11) **EP 4 585 591 A1**
(43) Date of publication of application: **16.07.2025**
(21) Application number: 23863186.5
(22) Date of filing: 05.09.2023
(51) Int. Cl.: C07D 309/10, A61K 9/16, A61K 39/00, A61K 47/10, A61K 47/22, A61K 47/26, A61P 37/04, C07H 17/04, C12N 5/10, C12N 15/11, C12N 15/88

(54) **LIPID NANOPARTICLES**

(30) Priority: 06.09.2022 JP 2022141421
(71) Applicant: Kyushu University, National University Corporation, Nishi-ku Fukuoka-shi Fukuoka 819-0395 (JP)
(72) Inventor: HIRAI, Go, Fukuoka-shi, Fukuoka 819-0395 (JP); MATSUNAGA, Naoya, Fukuoka-shi, Fukuoka 819-0395 (JP); YORITATE, Makoto, Fukuoka-shi, Fukuoka 819-0395 (JP); OHDO, Shigehiro, Fukuoka-shi, Fukuoka 819-0395 (JP); KONDO, Hirosato, Fukuoka-shi, Fukuoka 819-0395 (JP)
(74) Representative: Vossius & Partner Patentanwälte Rechtsanwälte mbB
(86) International application number: PCT/JP2023/032416
(87) International publication number: WO 2024/053648

(57) **Abstract**

The present invention provides candidate molecules for constituent components of various lipid nanoparticles.

The present invention relates to a C-glycoside glycolipid compound represented by formula (I): 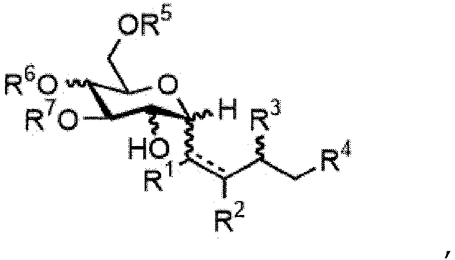 or formula (II): 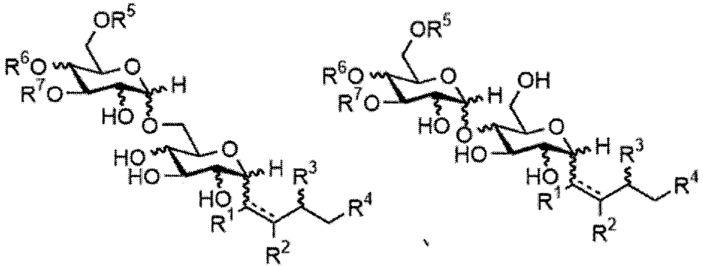 or 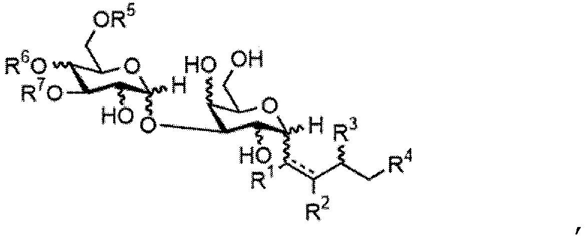 a lipid nanoparticle comprising the same, or a pharmaceutical composition, particularly a vaccine, which comprises the lipid nanoparticle.

## Description

### TECHNICAL FIELD

The present application claims the priority under the Paris Convention with respect to the Japanese Patent Application No. 2022-141421 filed on September 6, 2022, which is incorporated herein by reference in its entirety.

The present invention generally belongs to the field of lipid nanoparticles that can be used for vaccines and the like. In particular, the present invention relates to novel compounds capable of forming lipid nanoparticles, lipid nanoparticles comprising the same, and pharmaceutical compositions including vaccines comprising the lipid nanoparticles.

### BACKGROUND TECHNOLOGY

Nucleic acid medicines are one of the most promising types of pharmaceuticals. For example, antisense nucleic acids (Non-Patent Documents 1 and 2) and siRNA (Non-Patent Document 3) have been known for a long time, and in addition to them, miRNA (Non-Patent Document 4) and lncRNA (Non-Patent Document 5) are attracting attention as new drug targets, since the biological functions and importance of the noncoding RNAs of the latter RNAs have been elucidated in recent years. However, in delivering nucleic acids to target cells, the problems lie in the fact that nucleic acids have low biological stability and poor physical properties due to their negatively-charged and high-molecular structure (approximately 300 to 5000 kDa) (Non-Patent Document 6).

The above problems can be solved by lipid nanoparticles (LNPs) that were used for the transportation of mRNA vaccines, which were first commercialized due to the spread of the novel coronavirus infection (COVID-19) that has caused a pandemic since 2019. LNP is a vesicle consisting of multiple lipids, including neutral (amphiphilic) lipids that assist in particle formation, cholesterol, PEG lipids responsible for particle formation and improved biostability, and cationic lipids that stabilize anionic nucleic acids (Non-Patent Document 7). Those components are able to impart extremely diverse properties to the LNPs since they can design their structures at the molecular level, and also change the physical properties of the LNPs depending on the component ratio of them. Many of the previous researches have focused on the development of cationic lipids that neutralize the negative charge of the nucleic acids, which contributes to selective accumulation in organs and stabilization of the nucleic acids (Patent Documents 1 and 2). In the meantime, in the light that it has been suggested that PEG lipids may be a cause of anaphylactic shock (Non-Patent Document 8), and that neutral lipids have been made from diacyl phospholipids such as 1,2-distearoyl-sn-glycero-3-phosphocholine (DSPC) for a long time, there is still room for research on LNPs. Most of the nucleic acid medicines approved so far have been administered as the nucleic acid itself (in a naked state) or as GalNAc-modified forms, both of which are not encapsulated in any particles such as LNP (Non-Patent Document 9), and therefore there is an urgent demand for developing more practical LNPs and improving the same in function. So far, new LNPs have been developed focusing on cationic lipids responsible for selective accumulation in organs and stabilization of nucleic acids.

On the other hand, glycolipids are components of cell membranes, and the sugar chains thereof protruding on the cell surface recognize specific molecules to cause the intracellular uptake of the molecules and various signal transmissions. For example, viruses such as influenza virus have their own glycan-recognizing proteins, which are known to recognize and bind to the glycans present on the hosts' cells, thereby allowing infection to occur. It can be expected that the glycans, which serve as ligands for specific glycan-recognizing proteins on the cell surface, would be incorporated into LNPs to enhance the organ localization and intracellular uptake of the LNPs. However, glycolipids currently have not yet been applied to Drug Delivery System (DDS) since they are difficult to form vesicular structures on their own, although the self-assembly properties of glycolipids themselves have been studied (Non-patent Document 10). Recently, the properties of LNPs prepared by replacing neutral lipids with plant-derived natural glycolipids was reported (Non-patent Document 11), but the report was limited to the investigation on the glycolipids of plant or biological origin, thus having yet found no novel helper lipids that afford any good transfection efficiency. On the other hand, the replacement of DSPC with other lipids changed the organ localization tendency of the resulting LNPs, suggesting that the selection of helper lipids may be effective for organ- and cell-specific delivery of LNPs. Further, it has been reported that nanoparticles carrying mannose through PEG on the surface of hydroxyethyl starch were taken up into dendritic cells in large quantities (Non-Patent Document 12).

### CITATION LIST

### PATENT DOCUMENTS

Patent Document 1: JPA 2018-532721
Patent Document 2: JPA 2022-501360

### NON-PATENT DOCUMENTS

Non-Patent Document 1: Paterson BM, Roberts BE, Kuff EL. Structural gene identification and mapping by DNA-mRNA hybrid-arrested cell-free translation. Proc Natl Acad Sci U S A. 1977; 74(10): 4370-4374. https://doi.org/10.1073/pnas.74.10.4370
Non-Patent Document 2: Zamecnik PC, Stephenson ML. Inhibition of Rous sarcoma virus replication and cell transformation by a specific oligodeoxynucleotide. Proc Natl Acad Sci U S A. 1978; 75(1): 280-284. https://doi.org/10.1073/pnas.75.1.280
Non-Patent Document 3: Elbashir SM, Harborth J, Lendeckel W, Yalcin A, Weber K, Tuschl T. Duplexes of 21± nucleotide RNAs mediate RNA interference in cultured mammalian cells. n.d.
Non-Patent Document 4: O' Brien J, Hayder H, Zayed Y, Peng C. Overview of MicroRNA Biogenesis, Mechanisms of Actions, and Circulation. Front Endocrinol. 2018; 9: 402. https://doi.org/10.3389/fendo.2018.00402
Non-Patent Document 5: Iyer MK, Niknafs YS, Malik R, et al. The landscape of long noncoding RNAs in the human transcriptome. Nat Genet. 2015; 47(3): 199-208. https://doi.org/10.1038/ng.3192
Non-Patent Document 6: Kowalski PS, Rudra A, Miao L, Anderson DG. Delivering the Messenger: Advances in Technologies for Therapeutic mRNA Delivery. Mol Ther. 2019; 27(4): 710-728. https://doi.org/10.1016/j.ymthe.2019.02.012
Non-Patent Document 7: Buschmann MD, Carrasco MJ, Alishetty S, Paige M, Alameh MG, Weissman D. Nanomaterial Delivery Systems for mRNA Vaccines. Vaccines (Basel). 2021; 9(1). https://doi.org/10.3390/vaccines9010065
Non-Patent Document 8: Sellaturay P, Nasser S, Islam S, Gurugama P, Ewan PW. Polyethylene glycol (PEG) is a cause of anaphylaxis to the Pfizer/BioNTech mRNA COVID-19 vaccine. Clin Exp Allergy. 2021; 51(6):861-863. https://doi.org/10.1111/cea.13874
Non-Patent Document 9: Yamada Y. Nucleic Acid Drugs-Current Status, Issues, and Expectations for Exosomes. Cancers. 2021; 13(19). https://doi.org/10.3390/cancers13195002
Non-Patent Document 10: Hashim R, Zahid NI, Velayutham TS, Aripin NFK, Ogawa S, Sugimura A. Dry Thermotropic Glycolipid Self-Assembly: A Review. J Oleo Sci. 2018; 67(6): 651-668. https://doi.org/10.5650/jos.ess17261
Non-Patent Document 11: Kim J, Jozic A, Sahay G. Naturally Derived Membrane Lipids Impact Nanoparticle-Based Messenger RNA Delivery. Cell Mol Bioeng. 2020; 13(5):463-474. https://doi.org/10.1007/s12195-020-00619-y
Non-Patent Document 12: Landfester K, Wurm FR, et al. Carbohydrate-Based Nanocarriers Exhibiting Specific Cell Targeting with Minimum Influence from the Protein Corona. Angew. Chem. Int. Ed. 2015; 54: 7436-7440. http://dx.doi.org/10.1002/anie.201502398

### SUMMARY OF THE INVENTION

### PROBLEMS TO BE SOLVED BY THE INVENTION

mRNA vaccines for COVID-19 may induce fever, inflammation, and anaphylactic shock, and those side effects are believed to be caused by the constituent components of the LNP that delivers the mRNA. Thus, there is to be improved in the components of LNPs used currently, and, in addition to the fact, these components would not be guaranteed to be equally effective against newly emerging infectious diseases in LNP-mRNA vaccines. Therefore, the development of candidate molecules for various LNP components is essential.

### MEANS TO SOLVE PROBLEMS

The present inventors have discovered that the replacement of the existing neutral lipids represented by DSPC with any specific glycolipids in LNPs consisting of neutral lipids, cholesterol, PEG lipids, and cationic lipids, surprisingly improves the intracellular uptake, stability, organ localization, and cytokine induction ability of mRNA in the LNPs, thereby accomplishing the present invention. According to the present invention, it is suggested that nanoparticles could be modified with any glycolipids structures to deliver to target cells.

Therefore, the present invention encompasses the aspects as follows.

### <C-glycoside glycolipid compounds>

[1] A C-glycoside glycolipid compound represented by formula (I): 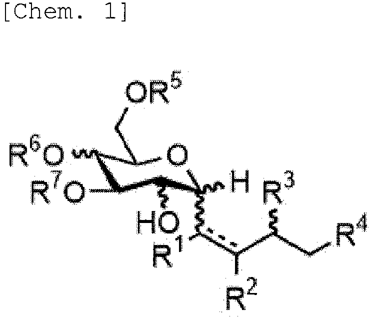 in which
   R1 is hydrogen, a halogen, or a hydroxy;
   R2 is a hydrogen atom, a hydroxy group that may be substituted, or a straight or branched, saturated or unsaturated C11-C15 alkyl group that may be substituted, wherein the alkyl group may be optionally interrupted by one or more ester bonds, amide bonds, and/or ether bonds;
   R3 is a hydrogen atom, a straight or branched, saturated or unsaturated C11-C15 alkyl group that may be substituted, wherein the alkyl group may be optionally interrupted by one or more ester bonds, amide bonds, and/or ether bonds, an amino group that may be secondarily amidated, or a hydroxy group that may be alkylated or esterified;
   R4 is a straight or branched, saturated or unsaturated C11-C15 alkyl group that may be substituted, wherein the alkyl group may be interrupted by one or more ester bonds, amide bonds, and/or ether bonds, an amino group that may be secondarily amidated, or a hydroxy group that may be alkylated or esterified;
   R5, R6 and R7 are each independent a hydrogen atom, or a sugar chain connected by glycosyl bonds;
   the wave lines each independently refer to a single stereochemistry of either S or R configuration when the bonded carbon is an asymmetric carbon atom; and
   the dotted lines each independently indicate the presence or absence of a bond, and refer to either the Z or E isomer when the bond exists as a double bond.
[2] The C-glycoside glycolipid compound according to [1], in which in formula (I),
   R1 is hydrogen, a halogen, or a hydroxy;
   R2 is a straight or branched, saturated C11-C15 alkyl group that may be substituted, wherein the alkyl group may be optionally interrupted by one or more ester bonds, amide bonds, and/or ether bonds;
   R3 is a straight or branched, saturated C11-C15 alkyl group that may be substituted, wherein the alkyl group may be optionally interrupted by one or more ester bonds, amide bonds, and/or ether bonds;
   R4 is a straight or branched, saturated C11-C15 alkyl group that may be substituted, wherein the alkyl group may be interrupted by one or more ester bonds, amide bonds, and/or ether bonds;
   R5, R6 and R7 are each independent a hydrogen atom, or a sugar chain connected by glycosyl bonds;
   the wave lines each independently refer to a single stereochemistry of either S or R configuration when the bonded carbon is an asymmetric carbon atom; and
   the dotted lines each independently indicate the presence or absence of a bond, and refer to either the Z or E isomer when the bond exists as a double bond.
[3] The C-glycoside glycolipid compound according to [2], in which in formula (I),
   R1 is hydrogen, a halogen, or a hydroxy;
   R2 is a straight or branched, saturated C11-C15 alkyl group that may be substituted, wherein the alkyl group is not interrupted by any bond;
   R3 is a straight or branched, saturated C11-C15 alkyl group that may be substituted, wherein the alkyl group is not interrupted by any bond;
   R4 is a straight or branched, saturated C11-C15 alkyl group that may be substituted, wherein the alkyl group is not interrupted by any bond;
   R5, R6 and R7 are each independent a hydrogen atom, or a sugar chain connected by glycosyl bonds;
   the wave lines each independently refer to a single stereochemistry of either S or R configuration when the bonded carbon is an asymmetric carbon atom; and
   the dotted lines each independently indicate the presence or absence of a bond, and refer to either the Z or E isomer when the bond exists as a double bond.
[4] The C-glycoside glycolipid compound according to [3], represented by formula (I''): 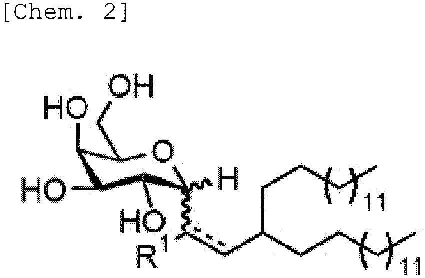 in which
   R1 is hydrogen, a halogen, or a hydroxy group;
   the wave lines each independently refer to a single stereochemistry of either S or R configuration when the bonded carbon is an asymmetric carbon atom; and
   the dotted lines each independently indicate the presence or absence of a bond, and refer to either the Z or E isomer when the bond exists as a double bond.
[5] The C-glycoside glycolipid compound according to [4], in which R1 is fluoro.
[6] The C-glycoside glycolipid compound according to [5], in which the dotted lines represent a double bond of Z isomer.

### <C-glycoside disaccharide compounds>

[7] A C-glycoside glycolipid compound represented by formula (II): 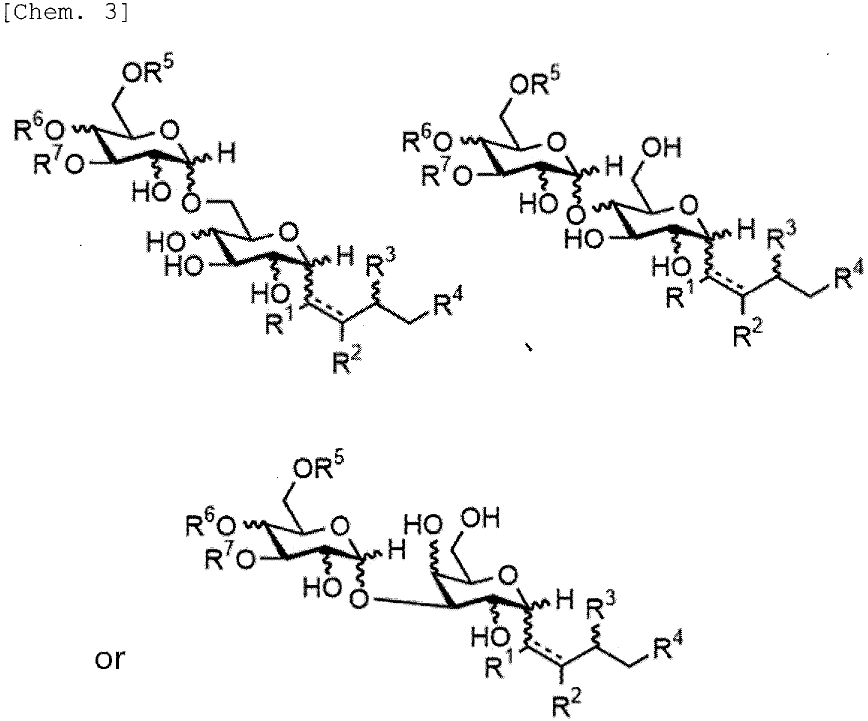 in which
   R1 is hydrogen, a halogen, or a hydroxy;
   R2 is a hydrogen atom, a hydroxy group that may be substituted, or a straight or branched, saturated or unsaturated C11-C15 alkyl group that may be substituted, wherein the alkyl group may be optionally interrupted by one or more ester bonds, amide bonds, and/or ether bonds;
   R3 is a hydrogen atom, a straight or branched, saturated or unsaturated C11-C15 alkyl group that may be substituted, wherein the alkyl group may be optionally interrupted by one or more ester bonds, amide bonds, and/or ether bonds, an amino group that may be secondarily amidated, or a hydroxy group that may be alkylated or esterified;
   R4 is a straight or branched, saturated or unsaturated C11-C15 alkyl group that may be substituted, wherein the alkyl group may be interrupted by one or more ester bonds, amide bonds, and/or ether bonds, an amino group that may be secondarily amidated, or a hydroxy group that may be alkylated or esterified;
   R5, R6 and R7 are each independent a hydrogen atom, or a sugar chain connected by glycosyl bonds;
   the wave lines each independently refer to a single stereochemistry of either S or R configuration when the bonded carbon is an asymmetric carbon atom; and
   the dotted lines each independently indicate the presence or absence of a bond, and refer to either the Z or E isomer when the bond exists as a double bond.
[8] The C-glycoside glycolipid compound according to [7], in which in formula (II),
   R1 is hydrogen, a halogen, or a hydroxy;
   R2 is a straight or branched, saturated C11-C15 alkyl group that may be substituted, wherein the alkyl group may be optionally interrupted by one or more ester bonds, amide bonds, and/or ether bonds;
   R3 is a straight or branched, saturated C11-C15 alkyl group that may be substituted, wherein the alkyl group may be optionally interrupted by one or more ester bonds, amide bonds, and/or ether bonds;
   R4 is a straight or branched, saturated C11-C15 alkyl group that may be substituted, wherein the alkyl group may be interrupted by one or more ester bonds, amide bonds, and/or ether bonds;
   R5, R6 and R7 are each independent a hydrogen atom, or a sugar chain connected by glycosyl bonds;
   the wave lines each independently refer to a single stereochemistry of either S or R configuration when the bonded carbon is an asymmetric carbon atom; and
   the dotted lines each independently indicate the presence or absence of a bond, and refer to either the Z or E isomer when the bond exists as a double bond.
[9] The C-glycoside glycolipid compound according to [8], in which in formula (II),
   R1 is hydrogen, a halogen, or a hydroxy;
   R2 is a straight or branched, saturated C11-C15 alkyl group that may be substituted, wherein the alkyl group is not interrupted by any bond;
   R3 is a straight or branched, saturated C11-C15 alkyl group that may be substituted, wherein the alkyl group is not interrupted by any bond;
   R4 is a straight or branched, saturated C11-C15 alkyl group that may be substituted, wherein the alkyl group is not interrupted by any bond;
   R5, R6 and R7 are each independent a hydrogen atom, or a sugar chain connected by glycosyl bonds;
   the wave lines each independently refer to a single stereochemistry of either S or R configuration when the bonded carbon is an asymmetric carbon atom; and
   the dotted lines each independently indicate the presence or absence of a bond, and refer to either the Z or E isomer when the bond exists as a double bond.
[10] The C-glycoside glycolipid compound according to [9], represented by formula (II''): 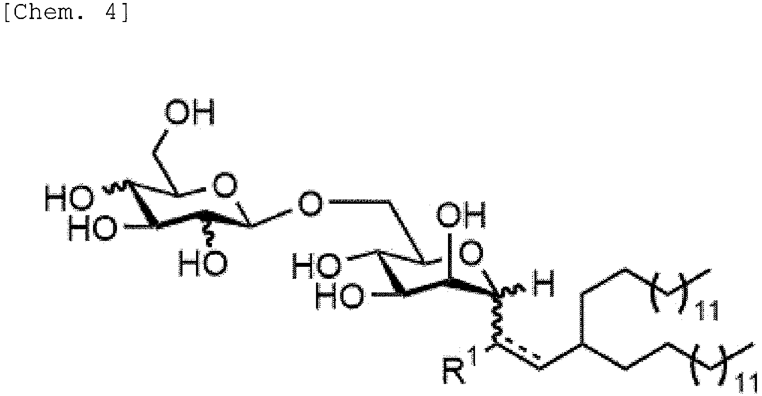 in which
   R1 is hydrogen, a halogen, or a hydroxy group;
   the wave lines each independently refer to a single stereochemistry of either S or R configuration when the bonded carbon is an asymmetric carbon atom; and
   the dotted lines each independently indicate the presence or absence of a bond, and refer to either the Z or E isomer when the bond exists as a double bond.
[11] The C-glycoside glycolipid compound according to [10], in which R1 is fluoro.
[12] The C-glycoside glycolipid compound according to [11], in which the dotted lines represent a double bond of Z isomer.

### <LNPs>

[13] A lipid nanoparticle comprising the C-glycoside glycolipid compound according to any one of [1] to [6] and [7] to [12], as well as (a) a nucleic acid molecule, (b) a cationic lipid, (c) an aggregation inhibitor (such as a polyethylene glycol (PEG) lipid or a PEG-modified lipid), and (d) a sterol.

### <Pharmaceutical compositions>

[14] A pharmaceutical composition comprising the lipid nanoparticle according to [13].
[15] The pharmaceutical composition according to [14], which is a vaccine.

### EFFECT OF INVENTION

LNPs comprising the C-glycoside glycolipid compound of the present invention significantly increase the amount of mRNA delivered into cells and/or the translation ability of proteins encoded by mRNA in comparison with conventional LNPs. This makes it possible to significantly decrease the amount of other LNP components that may cause side effects or adverse reactions such as fever, inflammation, anaphylactic shock, etc. in COVID-19 mRNA vaccines, which is believed to lead to a reduction in those side effects and/or adverse reactions.

### BRIEF DESCRIPTION OF DRAWINGS

[FIG. 1] Figure 1A is a graph showing the effects of cell number on mRNA intracellular delivery by LNP, as indicated by luciferase activity. Figure 1B is a graph showing the effects of LNP doses on mRNA intracellular delivery by LNP, as indicated by luciferase activity.
[FIG. 2] Figure 2 is a graph showing the effects of various C-glycoside glycolipid-LNPs on mRNA intracellular delivery, as indicated by luciferase activity.
[FIG. 3] Figure 3 is a graph showing the effects of various C-glycoside glycolipid-LNPs on live cells, as indicated by ATP activity.
[FIG. 4] Figure 4 shows a photograph of an agarose gel demonstrating the stability at room temperature of various C-glycoside lipid-LNPs.
[FIG. 5A] Figure 5A is a graph showing the effects of various C-glycoside lipid-LNPs on the expression of the cytokine Ccl3 gene associated to adjuvant activity.
[FIG. 5B] Figure 5B is a graph showing the effects of various C-glycoside lipid-LNPs on the expression of the cytokine Ccl22 gene associated with adjuvant activity.
[FIG. 6A] Figure 6A is a graph showing the results of knocking down the Mgl2 gene by transfecting the Mgl2 siRNA listed in Table 2 into cells.
[FIG. 6B] Figure 6B is a graph showing the intracellular uptake amounts of Moderna-type LNP, compound 15α-LNP, and compound 14-Zα-LNP in cells with Mgl2 subjected to knockdown.
[FIG. 7] Figure 7 is a photograph and a graph that shows, after injecting intramuscularly Moderna-type LNP and compound 14-Zα-LNP into animals, the place where the mRNA of the LNPs was delivered to, as indicated by luciferase activity.
[FIG. 8] Figure 8 is a photograph and a graph that shows, after injecting intramuscularly Moderna-type LNP and compound 30-LNP into animals, the place where the mRNA of the LNPs was delivered to, as indicated by luciferase activity.
[FIG. 9] Figure 9 is a photograph and a graph that shows, 24 and 72 hours after injecting intramuscularly Moderna-type LNP and compound 33-LNP into animals, the place where the mRNA of the LNPs was delivered to, as indicated by luciferase activity.
[FIG. 10] Figure 10 shows a photograph and graph that shows the effect of galactose-type C-type lectin 1 (macrophage galactose-type lectin 1: MGL1/CD301a) on the intracellular uptake of compound 14-Zα-LNP.
[FIG. 11] Figure 11 is a graph showing the effect of cell type on the intracellular uptake of compound 14-Zα and compound 30-LNPs.
[FIG. 12] Figure 12 is a graph showing the intracellular mRNA uptake of compound 30-LNP in human leukemia cells THP1, which are human immune cells.
[FIG. 13] Figure 13 is a graph showing the intracellular delivery of luciferase mRNAs of compound 14-Zα, compound 30, and compound 33-LNPs.
[FIG. 14] Figure 14 is a graph showing the mRNA intracellular delivery of compound 30-LNP, as indicated by GFP protein, comparing with Moderna-type LNP. In the figure, "P4 cell population" indicates the part of the cell population with stronger fluorescence (as shown only in the hatched gray zone).
[FIG. 15] Figure 15 is a graph showing the effects of various C-glycoside glycolipid-LNPs on 4°C PBS preservation, as indicated by GFP protein.
[FIG. 16] Figure 16 shows a graph showing the effect of compound 30-LNP on cryopreservation, as indicated by GFP protein. In the figure, "P4 cell population" indicates the part of the cell population with stronger fluorescence (as shown only in the hatched gray zone).
[FIG. 17] Figure 17 is a graph showing the antibody titers of Moderna-type and compound 33-LNPs, as indicated by the receptor-binding domain (RBD).
[FIG. 18] Figure 18 is graphs showing the antibody titers after the first and third weeks of administration of Moderna-type and compound 33-LNPs, as indicated by the receptor-binding domain (RBD).
[FIG. 19] Figure 19 a graph showing the antibody titers of Moderna-type and compound 33-LNPs using the full-length protein of spike protein as an indicator for.

### ASPECTS FOR CARRYING OUT INVENTION

### <Compounds>

In one aspect, the present invention relates to a C-glycoside glycolipid compound represented by formula (I): 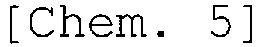 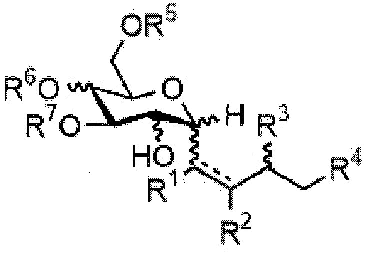 in which
R1 is hydrogen, a halogen, or a hydroxy;
R2 is a hydrogen atom, a hydroxy group that may be substituted, or a straight or branched, saturated or unsaturated C11-C15 alkyl group that may be substituted, wherein the alkyl group may be optionally interrupted by one or more ester bonds, amide bonds, and/or ether bonds;
R3 is a hydrogen atom, a straight or branched, saturated or unsaturated C11-C15 alkyl group that may be substituted, wherein the alkyl group may be optionally interrupted by one or more ester bonds, amide bonds, and/or ether bonds, an amino group that may be secondarily amidated, or a hydroxy group that may be alkylated or esterified;
R4 is a straight or branched, saturated or unsaturated C11-C15 alkyl group that may be substituted, wherein the alkyl group may be interrupted by one or more ester bonds, amide bonds, and/or ether bonds, an amino group that may be secondarily amidated, or a hydroxy group that may be alkylated or esterified;
R5, R6 and R7 are each independent a hydrogen atom, or a sugar chain connected by glycosyl bonds;
the wave lines each independently refer to a single stereochemistry of either S or R configuration when the bonded carbon is an asymmetric carbon atom; and
the dotted lines each independently indicate the presence or absence of a bond, and refer to either the Z or E isomer when the bond exists as a double bond.

In the preferred embodiment of the present aspect, the present invention relates to a C-glycoside glycolipid compound represented by formula (I'): 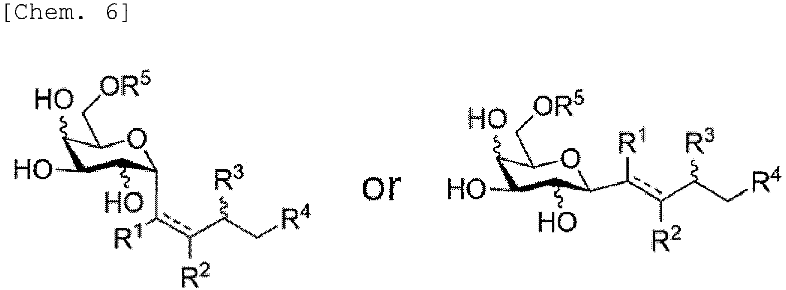 in which
R1 is hydrogen, a halogen, or a hydroxy;
R2 is a hydrogen atom, a hydroxy group that may be substituted, or a straight or branched, saturated or unsaturated C11-C15 alkyl group that may be substituted, wherein the alkyl group may be optionally interrupted by one or more ester bonds, amide bonds, and/or ether bonds;
R3 is a hydrogen atom, a straight or branched, saturated or unsaturated C11-C15 alkyl group that may be substituted, wherein the alkyl group may be optionally interrupted by one or more ester bonds, amide bonds, and/or ether bonds, an amino group that may be secondarily amidated, or a hydroxy group that may be alkylated or esterified;
R4 is a straight or branched, saturated or unsaturated C11-C15 alkyl group that may be substituted, wherein the alkyl group may be interrupted by one or more ester bonds, amide bonds, and/or ether bonds, an amino group that may be secondarily amidated, or a hydroxy group that may be alkylated or esterified;
R5 is a hydrogen atom, or a sugar chain connected by glycosyl bonds;
the wave line refers to a single stereochemistry of either S or R configuration when the bonded carbon is an asymmetric carbon atom; and
the dotted line indicates the presence or absence of a bond, and refer to either the Z or E isomer when the bond exists as a double bond.

As used herein, the term "C-glycoside glycolipid compound" refers to a glycolipid compound in which a two-stranded alkyl group and a sugar moiety are directly linked by a C-glycosyl bond.

The glycolipid compounds of the present invention are those having an extremely simple structure in which a two-chain alkyl and a monosaccharide are directly connected by an α- or β-C-glycosyl bond, whereas the glycolipid compounds that have been reported to use LNP were all O-glycoside glycolipid compounds of diacylglycerol. The C-glycosylation reaction according to the present invention enables to freely change the structure of the glycosidic linkage site. In addition, the structures of sugar chains and lipids can also be extensively modified, thereby making it possible to conduct structure-activity correlation studies that have never been studied so far.

As used herein, the term "halogen" refers to chlorine, bromine, iodine, or fluorine. The preferred halogen is fluorine.

As used herein, the term "hydroxy group that may be substituted" refers to the hydroxy group itself or a hydroxy group substituted with alkyl, ester, or carbonate.

As used herein, the term "C11-C15 alkyl" in "C11-C15 alkyl that may be substituted" refers to a straight or branched hydrocarbon chain group consisting of the indicated number of carbon and hydrogen atoms, which may comprise any unsaturation and which remaining portion of the molecule is connected by a single bond. Examples include, but not limited to, alkyl groups such as n-undecyl, n-dodecyl, n-tridecyl, n-tetradecyl, and n-pentadecyl. When the alkyl group is substituted with one or more substituents, the substituents are defined hereinafter. When the "C11-C15 alkyl group that may be substituted" comprises any unsaturation, it may comprise one or more double bonds. The "C11-C15 alkyl group that may be substituted" according to the present invention may be optionally interrupted by one or more ester bonds, one or more amide bonds, and/or one or more ether bonds, as defined.

As used herein, the term "amino group that may be secondarily amidated" refers to a secondary amide wherein the amino group is condensed with a C11-C15 carboxylic acid, or an unsubstituted amino group, and an ammonium salt thereof.

As used herein, the term "hydroxy group that may be alkylated or esterified" refers to a hydroxy group substituted with an alkyl group of carbon number C11-C15, an ester condensed from a carboxylic acid of carbon number C11-C15, or an unsubstituted hydroxy group.

As used herein, the term "sugar chain connected by glycosyl bonds" refers to a monosaccharide such as glucose, galactose, mannose, or glucosamine, which are linked by α- or β-O-glycosidic bonds, or a disaccharide that comprise the aforementioned monosaccharides as a constituent component.

As used herein, the amino acid residue moiety has the usual stereo-configuration of amino acids, and each of those moieties can exist independently in a stereoisomeric form of either "L" or "D" type.

The term "may be substituted" in each of the above definitions means that a substituent group (including carbon atoms and heteroatoms) may be substituted at any substitutable position with one or more substituents selected from: halogen (fluoro, chloro, bromo); alkoxy, preferably methoxy; alkylcarbonyl, preferably methylcarbonyl; alkoxy-carbonyl, preferably methoxycarbonyl and ethoxycarbonyl; hydroxycarbonyl; methyl that may be substituted, for example, trifluoromethyl; cyano; nitro; methanesulfonyl; isobutyl; tert-butyl; ethanone; acetamidomethyl (-CH₂-NH-CO-CH₃); oxazolyl or isoxazolyl that may be substituted with C1 to C3 alkyl (for example, methyl); pyridyl; hydroxy; phenyl; alkylamide, preferably isobutylamide (-NH-CO-CH-(CH₃)₂), O-alkyl-amino-alkyl, preferably O-(CH₂)₂-N-(CH₂-CH₃)₂; N-acetamide; [1,2,3]thiadiazolyl; dialkylamine, preferably dimethylamine; ethyl; ethyl substituted with an amine (for example, dialkylamine) or dialkylcarboxyamide, preferably dimethylamine and diethylamine), preferably those in which the dialkylamine of the dialkylcarboxyamide forms a 4, 5, 6, or 7-membered ring; isopropyl; N-propionamide, haloacetyl, cyano, formyl, and ketone.

Preferably, the substituent as mentioned above is selected from fluoro, chloro, bromo, methoxy, methylcarbonyl, methoxycarbonyl, hydroxycarbonyl, ethoxycarbonyl, methyl group that may be substituted such as trifluoromethyl, cyano, nitro, methanesulfonyl, pyridine, tert-butyl, acetamido methyl (-CH₂-NH-CO-CH₃), oxazolyl or isoxazolyl that may be substituted with C1 to C3 alkyl (for example, methyl), phenyl, O-(CH₂)₂-N-(CH₂-CH₃)₂, N-acetamide, dimethylamine, isopropyl, N-propionamide, haloacetyl, cyano, formyl, and ketone.

The term "pharmaceutically acceptable salt" refers to a salt that can be (directly or indirectly) provided with the compound described herein when administered to a subject. The preparation of the salt can be carried out by the methods known in the art of the relevant technical field. Preferably, when administered to humans, a "pharmaceutically acceptable salt" provides with a molecular portion that is physiologically tolerable and typically does not cause any adverse reactions such as allergic reactions or similar discomforts (for example, nausea, and dizziness).

For example, pharmaceutically acceptable salts of the compounds provided according to the present specification are synthesized by conventional chemical methods from parent compounds containing a basic or acidic moiety. Generally, such salts are prepared by reacting the free acid or free base form of these compounds with an appropriate salt or acid in stoichiometric amounts, for example, in water or an organic solvent or a mixture thereof. In general, a nonaqueous medium such as ether, ethyl acetate, ethanol, isopropanol, or acetonitrile is preferred. Examples of acid addition salts include mineral acid addition salts (such as hydrochloride, hydrobromide, hydriodic acid, sulfate, nitrate, and phosphate), and organic acid addition salts, such as acetate, maleate, fumarate, citrate, oxalate, succinate, tartrate, malate, mandelate, methanesulfonate, and p-toluenesulfonate. Examples of alkaline addition salts include inorganic salts (for example, salts of sodium, potassium, calcium, ammonium, magnesium, aluminum, and lithium) and organic alkaline salts (for example, salts of ethylenediamine, ethanolamine, N,N-dialkylethanolamine, triethanolamine, glucamine, and basic amino acids).

The compounds according to the present invention may exist in various stereoisomeric forms. Stereoisomers are compounds that differ only in their spatial arrangement. Multiple enantiomers are multiple sets of stereoisomers that cannot be superimposed due to the presence of non-symmetrically substituted carbon atoms, which most commonly function as chiral centers. The enantiomer refers to one of a pair of molecules that are mirror images of each other and cannot be superimposed. Multiple diastereomers are multiple stereoisomers that are not related as mirror images due to the presence of two or more non-symmetrically substituted carbon atoms. The configurations of the substituents around one or more asymmetric carbon atoms are represented by "R" and "S".

In the preferred embodiment, the present invention relates to the C-glycoside glycolipid compound according to the present invention, in which, in formula (I), R1 is hydrogen, a halogen, or a hydroxy;
R2 is a straight or branched, saturated C11-C15 alkyl group, which may be substituted, wherein the alkyl group may be optionally interrupted by one or more ester bonds, amide bonds, and/or ether bonds;
R3 is a straight or branched, saturated C11-C15 alkyl group, which may be substituted, wherein the alkyl group may be optionally interrupted by one or more ester bonds, amide bonds, and/or ether bonds;
R4 is a straight or branched, saturated C11-C15 alkyl group, which may be substituted, wherein the alkyl group may be optionally interrupted by one or more ester bonds, amide bonds, and/or ether bonds;
R5 is a hydrogen atom, or a sugar chain connected by glycosyl bonds;
the wave line refers to a single stereochemistry of either S or R configuration when the bonded carbon is an asymmetric carbon atom; and
the dotted line indicates the presence or absence of a bond, and refer to either the Z or E isomer when the bond exists as a double bond.

In another preferred embodiment, the present invention relates to the C-glycoside glycolipid compound according to the present invention, in which, in formula (I), R1 is hydrogen, a halogen, or a hydroxy group;
R2 is a straight or branched, saturated C11-C15 alkyl group that may be substituted, wherein the alkyl group is not interrupted by any bond;
R3 is a straight or branched, saturated C11-C15 alkyl group that may be substituted, wherein the alkyl group is not interrupted by any bond;
R4 is a straight or branched, saturated C11-C15 alkyl group that may be substituted, wherein the alkyl group is not interrupted by any bond;
R5 is a hydrogen atom, or a sugar chain connected by glycosyl bonds;
the wave line refers to a single stereochemistry of either S or R configuration when the bonded carbon is an asymmetric carbon atom; and
the dotted line indicates the presence or absence of a bond, and refer to either the Z or E isomer when the bond exists as a double bond.

In another preferred embodiment, the present invention relates to the C-glycoside glycolipid compound according to the present invention, represented by formula (I''): 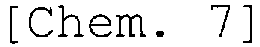 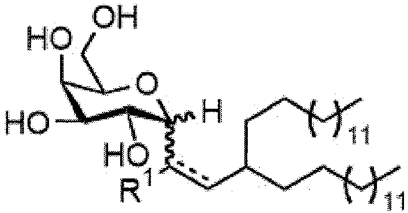 in which
R1 is hydrogen, a halogen, or a hydroxy group;
the wave lines each independently refer to a single stereochemistry of either S or R configuration when the bonded carbon is an asymmetric carbon atom; and
the dotted lines each independently indicate the presence or absence of a bond, and refer to either the Z or E isomer when the bond exists as a double bond.

In further another preferred embodiment, the present invention relates to the C-glycoside glycolipid compound according to the present invention, represented by formula (I‴): 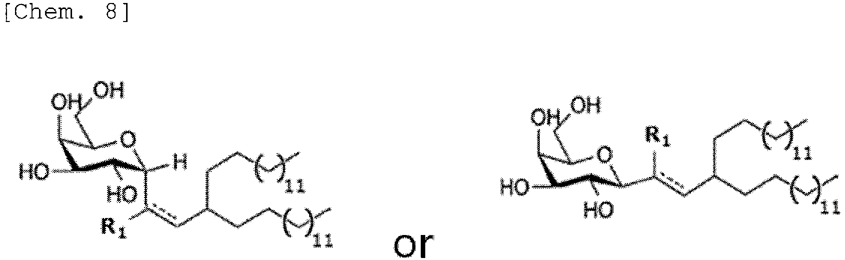 in which
R1 is hydrogen, a halogen, or a hydroxy group; and
the dotted line indicates the presence or absence of a bond, and refer to either the Z or E isomer when the bond exists as a double bond.

In the above preferred embodiments, the C-glycoside glycolipid compounds wherein R1 is fluoro are preferred, and the C-glycoside glycolipid compounds wherein R1 is fluoro, and the dotted line is a double bond of Z isomer are more preferred.

### <General preparation method for C-glycoside monosaccharide lipid compounds according to the present invention>

Hereinafter, a general explanation of the preparation method for C-glycoside monosaccharide lipid compounds according to the present invention is provided.

The C-glycoside monosaccharide lipid compounds of the present invention can be prepared by conducting synthesis of donor compounds and acceptor compounds according to reaction schemes I and II described below, and then conducting photocatalytic reduction-oxidation coupling and deprotection, and optional hydrogenation reaction according to reaction scheme III described below.

### Synthesis of donor compounds and acceptor compounds

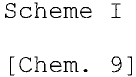

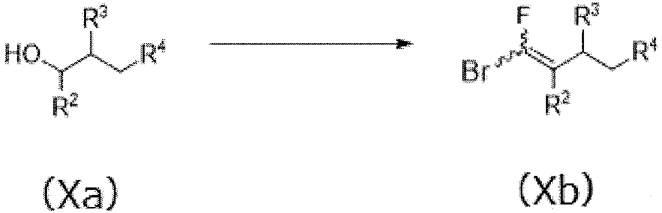

in which
R2 is a hydrogen atom, a hydroxy group that may be substituted, or a straight or branched, saturated or unsaturated C11-C15 alkyl group that may be substituted, wherein the alkyl group may be optionally interrupted by one or more ester bonds, amide bonds, and/or ether bonds;
R3 is a hydrogen atom, a straight or branched, saturated or unsaturated C11-C15 alkyl group that may be substituted, wherein the alkyl group may be optionally interrupted by one or more ester bonds, amide bonds, and/or ether bonds, an amino group that may be secondarily amidated, or a hydroxy group that may be alkylated or esterified;
R4 is a straight or branched, saturated or unsaturated C11-C15 alkyl group that may be substituted, wherein the alkyl group may be interrupted by one or more ester bonds, amide bonds, and/or ether bonds, an amino group that may be secondarily amidated, or a hydroxy group that may be alkylated or esterified.

A haloalkane compound represented by the general formula (Xb) can be obtained By reacting a compound represented by the general formula (Xa) with tribromofluoromethane and triphenylphosphine.

This reaction can be carried out in an appropriate solvent and at an appropriate temperature. A solvent may be any solvent that does not have a negative impact on the reaction, and examples thereof include tetrahydrofuran, diethyl ether, tert-butyl methyl ether, or a mixture of these solvents.

Examples of an activating agent include diethyl zinc and so on.

This reaction proceeds preferably at 25°C, especially between 10 and 40°C. 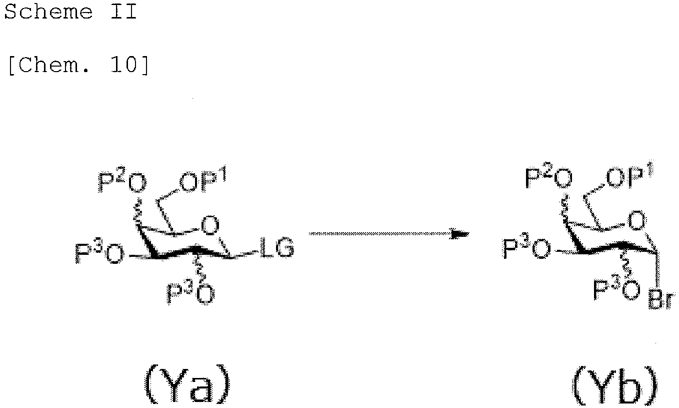 in which
P1 is an ester or acetal when it is a protective group, and when it is a sugar chain, P1 is a sugar chain having an ester, acetal, carbonate, or silyl as a protective group, for example, a monosaccharide such as glucose, galactose, mannose, or glucosamine, or a disaccharide comprising those monosaccharides as a constituent component;
P2 is a protective group which is an ester, acetal, or silyl;
P3 is a protective group which is an ester or carbonate; and
LG is a leaving group, which is a sulfide or an acetoxyl.

A halogenated compound represented by the general formula (Yb) can be obtained by subjecting a compound represented by the general formula (Ya) to a elimination reaction.

This reaction can be carried out in an appropriate solvent and at an appropriate temperature. A solvent may be any solvent that does not have a negative impact on the reaction, and examples thereof include dichloromethane, acetic acid, and a mixture of these solvents.

Examples of an activating agent include hydrogen bromide, iodine bromide and so on.

This reaction proceeds preferably at 25°C, especially between 25 and 60°C.

### Photocatalytic reductive coupling, deprotection, and optional hydrogenation

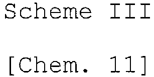

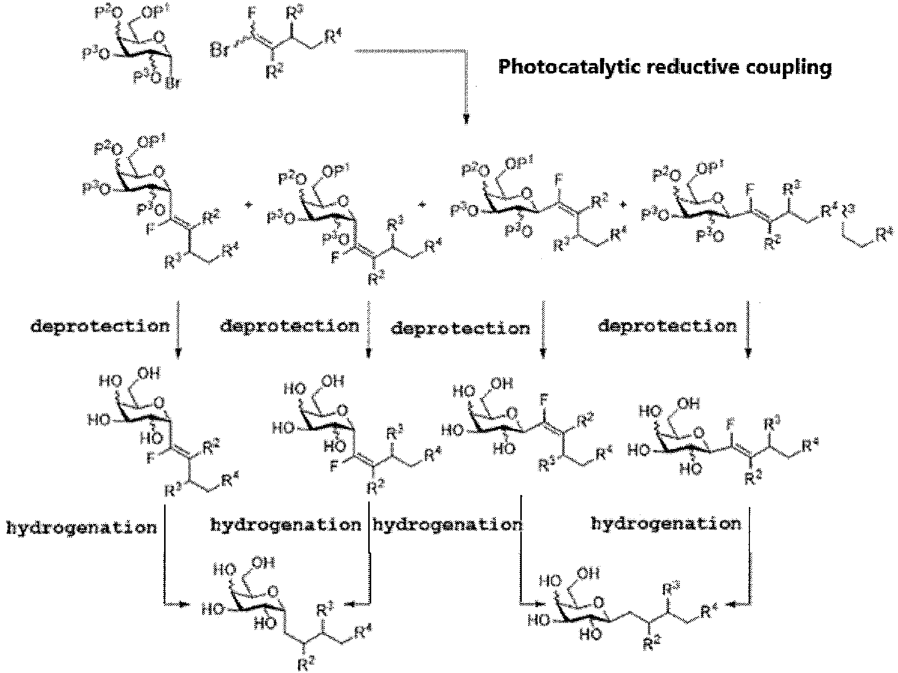

in which
R2, R3, R4, P1, P2, P3, and LG are defined in the same way as in the above schemes.

### Photocatalytic reductive coupling

In accordance with a photocatalytic reductive coupling reaction, a chemical reaction is carried out by irradiating a mixture solution of a substrate and a reagent with a blue LED in the presence of an organic photocatalyst or a transition metal photocatalyst that catalyzes both oxidation and reduction reactions by photoexcitation, and a transition metal catalyst that causes carbon-carbon bond formation.

As an organic photocatalyst or transition metal photocatalyst, 1,2,3,5-tetracarbazole-4,6-dicyanobenzene (4-CzIPN), [4,4"-bis(1,1-dimethyl)-2,2"-bipyridine-N1,N1"]bis[3,5-difluoro-2-[5-(trifluoromethyl)-2-pyridinyl-N]phenyl-C]iridium(III) hexafluorophosphate, and others can be used.

As a transition metal catalysts that causes carbon-carbon bond formation, nickel(II) dimethoxyethane chloride complex, nickel(II) dimethoxyethane bromide complex, nickel(II) bis(2,2,6,6-tetramethyl-3,5-heptanedionate), and others can be used.

A chemical reaction performed by irradiating with a blue LED, for example, can be carried out by irradiating a reaction solution prepared in a Pyrex (registered trademark) glass vial with a blue LED of a peak wavelength of 465 nm, while applying cold air to it from the top with a fan of about 15 cm in diameter.

### Deprotection

In accordance with a deprotection reaction, a protective group is removed, which is installed to render functional groups contained in sugars and lipids such as alcohols, amines, and carboxylic acids unreactive. For example, esters or carbonates can be removed with sodium methoxide, lithium hydroxide, sodium hydroxide, or sodium carbonate, and silyls can be removed with hydrogen fluoride pyridine or tetrabutylammonium fluoride.

### Hydrogenation

A hydrogenation of double bonds and a hydrogenation reaction of fluorine atoms in reaction substrates are carried out using transition metal heterogeneous catalysts such as palladium, platinum, and rhodium, and hydrogen gas.

In another preferred embodiment of the aspect of the present invention, the invention relates to a C-glycoside glycolipid compound represented by formula (II): 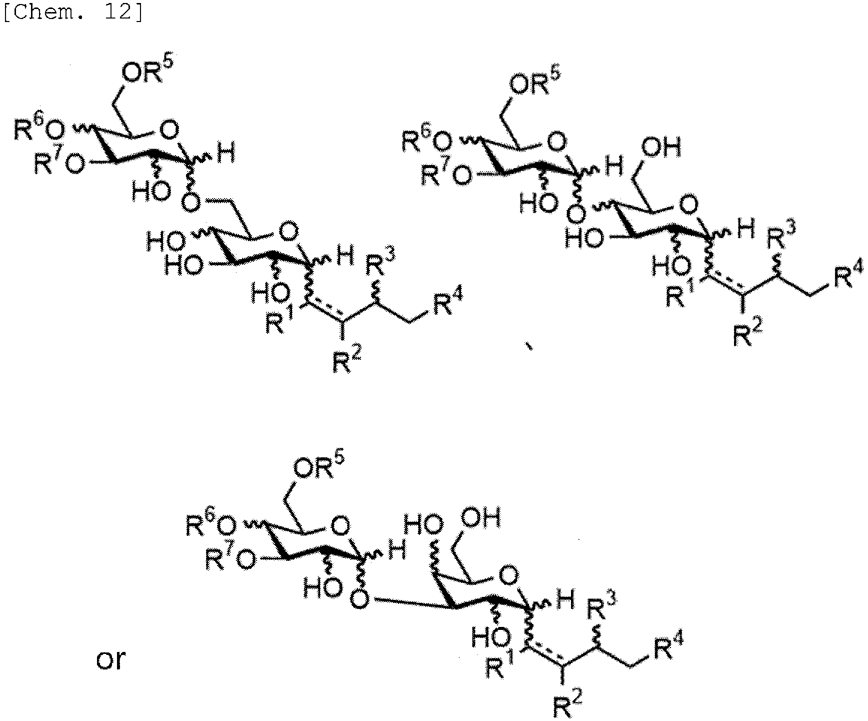 in which
R1 is hydrogen, a halogen, or a hydroxy;
R2 is a hydrogen atom, a hydroxy group that may be substituted, or a straight or branched, saturated or unsaturated C11-C15 alkyl group that may be substituted, wherein the alkyl group may be optionally interrupted by one or more ester bonds, amide bonds, and/or ether bonds;
R3 is a hydrogen atom, a straight or branched, saturated or unsaturated C11-C15 alkyl group that may be substituted, wherein the alkyl group may be optionally interrupted by one or more ester bonds, amide bonds, and/or ether bonds, an amino group that may be secondarily amidated, or a hydroxy group that may be alkylated or esterified;
R4 is a straight or branched, saturated or unsaturated C11-C15 alkyl group that may be substituted, wherein the alkyl group may be interrupted by one or more ester bonds, amide bonds, and/or ether bonds, an amino group that may be secondarily amidated, or a hydroxy group that may be alkylated or esterified;
R5, R6 and R7 are each independent a hydrogen atom, or a sugar chain connected by glycosyl bonds;
the wave lines each independently refer to a single stereochemistry of either S or R configuration when the bonded carbon is an asymmetric carbon atom; and
the dotted lines each independently indicate the presence or absence of a bond, and refer to either the Z or E isomer when the bond exists as a double bond.

In the preferred embodiment of the present aspect, the present invention relates to a C-glycoside glycolipid compound represented by formula (II'): 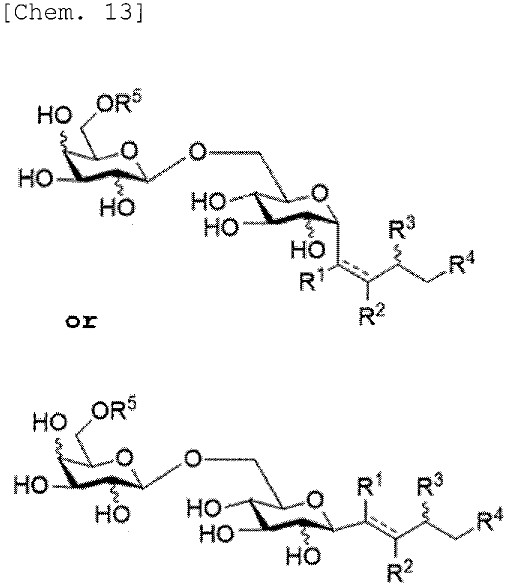 in which
R1 is hydrogen, a halogen, or a hydroxy;
R2 is a hydrogen atom, a hydroxy group that may be substituted, or a straight or branched, saturated or unsaturated C11-C15 alkyl group that may be substituted, wherein the alkyl group may be optionally interrupted by one or more ester bonds, amide bonds, and/or ether bonds;
R3 is a hydrogen atom, a straight or branched, saturated or unsaturated C11-C15 alkyl group that may be substituted, wherein the alkyl group may be optionally interrupted by one or more ester bonds, amide bonds, and/or ether bonds, an amino group that may be secondarily amidated, or a hydroxy group that may be alkylated or esterified;
R4 is a straight or branched, saturated or unsaturated C11-C15 alkyl group that may be substituted, wherein the alkyl group may be interrupted by one or more ester bonds, amide bonds, and/or ether bonds, an amino group that may be secondarily amidated, or a hydroxy group that may be alkylated or esterified;
R5 is a hydrogen atom, or a sugar chain connected by glycosyl bonds;
the wave line refers to a single stereochemistry of either S or R configuration when the bonded carbon is an asymmetric carbon atom; and
the dotted line indicates the presence or absence of a bond, and refer to either the Z or E isomer when the bond exists as a double bond.

The description of the substituent, salt, and stereoisomer in formula (II) is reasonable as an explanation in formula (I).

In other words, in the preferred embodiment of the present embodiment, the present invention relates to a C-glycoside glycolipid compound according to the present invention represented by formula (II) in which:
R1 is hydrogen, a halogen, or a hydroxy;
R2 is a straight or branched, saturated C11-C15 alkyl group that may be substituted, wherein the alkyl group may be optionally interrupted by one or more ester bonds, amide bonds, and/or ether bonds;
R3 is a straight or branched, saturated C11-C15 alkyl group that may be substituted, wherein the alkyl group may be optionally interrupted by one or more ester bonds, amide bonds, and/or ether bonds;
R4 is a straight or branched, saturated C11-C15 alkyl group that may be substituted, wherein the alkyl group may be interrupted by one or more ester bonds, amide bonds, and/or ether bonds;
R5 is a hydrogen atom, or a sugar chain connected by glycosyl bonds;
the wave line refers to a single stereochemistry of either S or R configuration when the bonded carbon is an asymmetric carbon atom; and
the dotted line indicates the presence or absence of a bond, and refer to either the Z or E isomer when the bond exists as a double bond.

In another preferred embodiment of the present invention, the invention relates to a C-glycoside glycolipid compound according to the present invention represented by formula (II) in which:
R1 is hydrogen, a halogen, or a hydroxy;
R2 is a straight or branched, saturated C11-C15 alkyl group that may be substituted, wherein the alkyl group is not interrupted by any bond;
R3 is a straight or branched, saturated C11-C15 alkyl group that may be substituted, wherein the alkyl group is not interrupted by any bond;
R4 is a straight or branched, saturated C11-C15 alkyl group that may be substituted, wherein the alkyl group is not interrupted by any bond;
R5 is a hydrogen atom, or a sugar chain connected by glycosyl bonds;
the wave line refers to a single stereochemistry of either S or R configuration when the bonded carbon is an asymmetric carbon atom; and
the dotted line indicates the presence or absence of a bond, and refer to either the Z or E isomer when the bond exists as a double bond.

In a preferred embodiment of the present aspect, the invention relates to a C-glycoside glycolipid compound according to claim 13 represented by formula (II''): 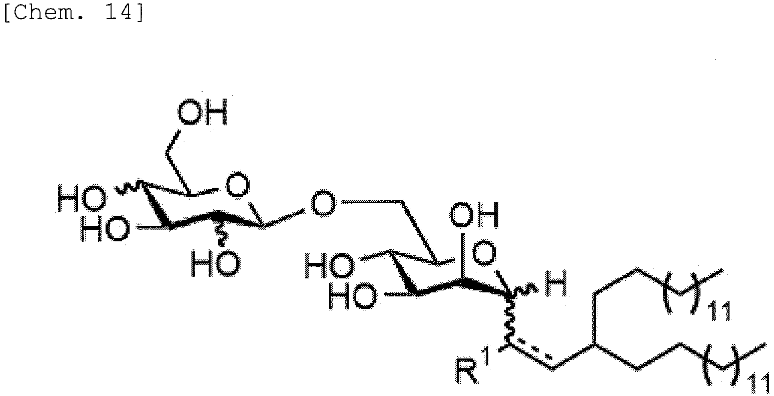 in which
R1 is hydrogen, a halogen, or a hydroxy group;
the wave lines each independently refer to a single stereochemistry of either S or R configuration when the bonded carbon is an asymmetric carbon atom; and
the dotted line indicates the presence or absence of a bond, and refer to either the Z or E isomer when the bond exists as a double bond.

### <General preparation method C-glycoside disaccharide lipid compounds according to the present invention>

The present invention encompasses C-glycoside disaccharide lipid compounds. The C-glycoside disaccharide lipid compounds of the present invention can be prepared according to the preparation method of the above-mentioned C-glycoside monosaccharide lipid compounds.

### <LNP>

In another aspect, the present invention relates to lipid nanoparticles comprising the C-glycoside glycolipid compound of the present invention, as well as (a) a nucleic acid molecule, (b) a cationic lipid, (c) an aggregation inhibitor (such as a polyethylene glycol (PEG) lipid or a PEG-modified lipid), and (d) a sterol.

### (a) Nucleic acid molecules

As used herein, a nucleic acid molecule is a molecule comprising nucleic acid components, preferably consisting of nucleic acid components. The term "nucleic acid molecule" preferably refers to DNA or RNA molecules. The term is preferably used as a synonym for the term "polynucleotide". Preferably, the nucleic acid molecules comprise nucleotide monomers that are covalently bonded to each other through phosphodiester bonds in the sugar/phosphate backbone, or are polymers consisting of such nucleotide monomers. The term "nucleic acid molecule" encompasses modified nucleic acid molecules such as DNA or RNA molecules with a modified base, a modified sugar, or a modified backbone.

The term "RNA" refers to naturally occurring or non-naturally occurring ribonucleic acid. For example, RNA may comprise one or more modified and/or non-naturally occurring components such as nucleic acid bases, nucleosides, nucleotides, or linkers. RNA may comprise a cap structure, a chain termination nucleoside, a stem loop, a poly-A sequence, and/or a polyadenylation signal. RNA may have a nucleotide sequence that codes for a intended polypeptide. For example, RNA can be a messenger RNA (mRNA).

Translation of mRNA that codes for a specific polypeptide, for example, in vivo translation of mRNA in mammalian cells, can generate the encoded polypeptide. RNA can be selected from a group consisting of, but not limited to, small interfering RNA (siRNA), asymmetric interfering RNA (aiRNA), antisense RNA (asRNA), microRNA (miRNA), double-stranded RNA (dsRNA), short hairpin RNA (shRNA), mRNA, and a mixture thereof.

### (b) Cationic lipids

LNP may comprise any cationic lipid suitable for forming lipid nanoparticles. Preferably, cationic lipids carry a net positive charge at about physiological pH.

Cationic lipids may be also amino lipids. The term "amino lipids" as used herein refers to lipids that comprise one or two fatty acids or fatty alkyl chains and an amino head group (including alkylamino or dialkylamino groups) that can be protonated to form cationic lipids at physiological pH.

For example, cationic lipids may be N,N-dioleoyl-N,N-dimethylammonium chloride (DODAC), N,N-stearyl-N,N-dimethylammonium bromide (DDAB), 1,2-dioleoyltrimethylammonium propane chloride (DOTAP) (also known as N-(2,3-dioleoyloxy)propyl-N,N,N-trimethylammonium chloride and 1,2-dioleoyloxy-3-trimethylammonium propane chloride), N-(1-(2,3-dioleoyloxy)propyl)-N,N,N-trimethylammonium chloride (DOTMA), N,N-dimethyl-2,3-dioleyloxypropylamine (DODMA), 1,2-dilinoleyloxy-N,N-dimethylaminopropane (DLinDMA), 1,2-dilinolenyloxy-N,N-dimethylaminopropane (DLenDMA), 1,2-di-y-linolenyloxy-N,N-dimethylaminopropane (gamma-DLenDMA), 1,2-dilinoleyl-carbamoyloxy-3-dimethylaminopropane (DLin-C-DAP), 1,2-dilinoleyloxy-3-(dimethylamino)acetoxylpropane (DLin-DAC), 1,2-dilinoleyloxy-3-morpholinopropane (DLin-MA), 1,2-dilinoleoyl-3-dimethylaminopropane (DLinDAP), 1,2-dilinoleylthio-3-dimethylaminopropane (DLin-S-DMA), 1-linoleoyl-2-linoleyloxy-3-dimethylaminopropane (DLin-2-DMAP), 1,2-dilinoleyloxy-3-trimethylaminopropane chloride (DLin-TMA.Cl), 1,2-Dilinoleoyl-3-trimethylaminopropane chloride (DLin-TAP.Cl), 1,2-Dilinoleyloxy-3-(N-methyl-piperazino) propane (DLin-MPZ), or 3-(N,N-dilinoleylamino)-1,2-propanediol (DLinAP), 3-(N,N-dioleylamino)-1,2-propanediol (DOAP), 1,2-dilinoleyloxo-3-(2-N,N-dimethyl-amino)ethoxypropane (DLin-EG-DMA), 2,2-dilinoleyl-4-dimethylaminomethyl-[1,3]-dioxolane (DLin-K-DMA) or an analog thereof, (3aR,5s,6aS)-N,N-dimethyl-2,2-di((9Z,12Z)-octadeca-9,12-dienyl)tetrahydro-3aH-cyclopenta[d][1,3]dioxol-5-amine, (6Z,9Z,28Z,31Z)-heptatriaconta-6,9,28,31-tetraen-19-yl-4-dimethylamino)butanoate (MC3), 1,1'-(2-(4-(2-((2-(bis(2-hydroxydodecyl)amino)ethyl)(2-hydroxydodecyl)amino)ethyl)piperazin-1-yl)ethylazanediyl)didodecan-2-ol (C12-200), 2,2-dilinoleyl-4-(2-dimethylaminoethyl)-[1,3]-dioxolane (DLin-K-C2-DMA), 2,2-dilinoleyl-4-dimethylaminomethyl-[1,3]-dioxolane (DLin-K-DMA), (6Z,9Z,28Z,31Z)-heptatriaconta-6,9,28,31-tetraen-19-yl-4-(dimethylamino)butanoate (DLin-M-C3-DMA), 3-((6Z,9Z,28Z,31Z)-heptatriaconta-6,9,28,31-tetraen-19-yloxy)-N,N-dimethylpropan-1-amine (MC3 ether), 4-((6Z,9Z,28Z,31Z)-heptatriaconta-6,9,28,31-tetraen-19-yloxy)-N,N-dimethylbutan-1-amine (MC4 ether), or any combination thereof.

Other suitable cationic lipids are disclosed in WO09/086558, WO09/127060, WO10/048536, WO10/054406, WO10/088537, WO10/129709, and WO2011/153493; US patent publication numbers 2011/0256175, 2012/0128760, and 2012/0027803; US patent number 8,158,601; and Love et al., PNAS, 107(5), 1864-69, 2010.

Examples of other suitable amino lipids include amino lipids with different fatty acid groups and other dialkylamino groups. Examples of such amino lipids include those with different alkyl substituents (such as N-ethyl-N-methylamino- and N-propyl-N-ethylamino-). Generally, amino lipids with fewer saturated acyl chains can be more easily adjusted in size, particularly when it is necessary to reduce the size of the complex to less than 0.3 microns for the purpose of filtration sterilization. Amino lipids comprising unsaturated fatty acids with carbon chain lengths ranging from C14 to C22 may be used. In amino lipids, it is also possible to use other skeletons to separate the amino group from the fatty acid or fatty alkyl portion.

In a more preferred embodiment, the LNP comprises a cationic lipid represented by formula (III) described in PCT/EP2017/064066. With regard to this, the description of PCT/EP2017/064066 is also incorporated herein by reference.

The LNP may comprise two or more cationic lipids. The cationic lipids can be selected to contribute to different useful properties. For example, cationic lipids with different properties such as amine pKa, chemical stability, half-life in circulation, half-life in tissues, net accumulation in tissues, or toxicity can be used for LNP. In particular, a cationic lipid may be selected to enhance the properties of LNPs mixed with lipids over LNPs with each single lipid.

### (c) Aggregation inhibitors

Aggregation inhibitor may be a lipid capable of inhibiting aggregation. Examples of such lipids include, but not limited to, polyethylene glycol (PEG)-modified lipids, monosialoganglioside Gml, and polyamide oligomers (PAO) as described in US Patent No. 6,320,017. The description of US Patent No. 6,320,017 is incorporated herein by reference in its entirety.

Examples of the aggregation inhibitor includes, but not limited to, for example, PEG-diacylglycerol (DAG), PEG-dialkylglycerol, PEG-dialkyloxypropyl (DAA), PEG-phospholipid, PEG-ceramide (Cer), or a mixture thereof (PEG-Cerl4 or PEG-Cer20, and so on), which is selected from polyethylene glycol (PEG) lipids. Examples of PEG-DAA complex includes, for example, PEG-dilauryloxypropyl (C12), PEG-dimyristyloxypropyl (C14), PEG-dipalmityloxypropyl (C16), or PEG-distearoyloxypropyl (C18). Examples of other pegylated lipids include, but not limited to, polyethylene glycol-dimyristoyl glycerol (C14-PEG or PEG-C14 (PEG having an average molecular weight of 2000 Da) (PEG-DMG); (R)-2,3-bis(octadecyloxy)propyl-1-(methoxypoly(ethyleneglycol)2000) propylcarbamate) (PEG-DSG); PEG-carbamoyl-1,2-dimyristyloxypropylamine (PEG having an average molecular weight of 2000 Da) (PEG-cDMA); N-acetylgalactosamine-((R)-2,3-bis(octadecyloxy)propyl-1-(methoxypoly(ethylene glycol)2000)propylcarbamate)) (GalNAc-PEG-DSG); mPEG (mw2000)-distearoylphosphatidylethanolamine (PEG-DSPE); and polyethyleneglycol-dipalmitoylglycerol (PEG-DPG).

In some embodiments, the aggregation inhibitor is PEG-DMG. In other embodiments, the aggregation inhibitor is PEG-c-DMA.

In a more preferred embodiment, the LNP comprises PEG lipid substitutes, which comprise no PEG, and/or comprise lipids replaced by phosphatidylcholine (PC) (such as oleic acid or an analog thereof).

In a further more preferred embodiment, the LNP comprises a aggregation inhibitor represented by formula (IV) described in PCT/EP2017/064066.

### (iii) Sterols

Preferably, sterol may be cholesterol. The sterol may be present in a ratio of about 10 mol% to about 60 mol% or about 25 mol% to about 40 mol% in the LNP. In some embodiments, the sterols are present in a ratio of about 10, 15, 20, 25, 30, 35, 40, 45, 50, 55, or approximately 60 mol% of the total lipids present in the LNPs.

### <Pharmaceutical compositions>

In further another embodiment, the present invention relates to a pharmaceutical composition comprising a lipid nanoparticle according to the present invention.

The lipid nanoparticle can be formulated as a whole or as a part in the pharmaceutical composition. The pharmaceutical composition may comprise one or more lipid nanoparticles. For example, the pharmaceutical composition may comprise one or more lipid nanoparticles comprising one or more different therapeutic agents and/or prophylactic agents. The pharmaceutical composition may further comprise one or more pharmaceutically acceptable excipients or adjuvants, including those described herein. Any conventional excipients and adjuvant components can be used in any pharmaceutical composition, except when they are incompatible with one or more components of the nanoparticle composition. Excipients and adjuvant components may be incompatible with the components of the lipid nanoparticles, when the combination thereof may cause any undesirable biological effects or other harmful effects of any kind.

In some embodiments, one or more excipients or adjuvant components may constitute more than 50% of the total weight or volume of a pharmaceutical composition comprising lipid nanoparticles. For example, one or more excipients or adjuvant components may conventionally constitute 50%, 60%, 70%, 80%, 90%, or more of the pharmaceutical products. In some embodiments, the pharmaceutically acceptable excipients are at least 95%, at least 96%, at least 97%, at least 98%, at least 99%, or 100% pure. In some embodiments, the excipients are approved for use in humans and veterinary medicine.

Relative amounts of one or more lipid nanoparticles, one or more pharmaceutically acceptable excipients and/or any further components comprised in a pharmaceutical composition according to the present disclosure vary depending on the properties, size, and/or state of the therapeutic target, and further vary depending on the route of administration of the composition. As an example, the pharmaceutical composition may comprise one or more lipid nanoparticles in an amount ranging from 0.1% to 100% (wt/wt).

The lipid nanoparticles and/or the pharmaceutical compositions comprising one or more lipids nanoparticles can be administered to any patient or subject including those who benefit from therapeutic effects provided by the delivery of therapeutic agents and/or prophylactic agents to one or more specific cells, tissues, organs, or their systems or systems (such as the renal system). The description provided herein for the lipid nanoparticles and/or the pharmaceutical compositions comprising one or more lipids nanoparticles mainly relates to compositions suitable for administration to humans, but those skilled in the art would understand that such compositions should be generally suitable for administration to any other mammal. Typical veterinary pharmacologists fully understand any modification of the composition suitable for administration to human among various animals, and they can design and/or perform such modifications using normal experiments, if any. Examples of the subjects to whom the composition is intended to be administered include, but not limited to, humans and other primates, and other mammals such as cattle, pigs, horses, sheep, cats, dogs, mice, and/or rats that have commercial value.

The pharmaceutical composition comprising one or more lipid nanoparticles can be prepared by the method known in the field of pharmacology, or any future-developed method. Generally, the preparation method comprises a step of combining the active ingredient with the excipients and/or one or more other adjuvant components, and then a step of dividing, shaping, and/or packaging the product into desired single or multiple administration units, if necessary or desirable.

The pharmaceutical composition according to the present disclosure can be prepared, packaged, and/or sold in bulk as a single unit dosage and/or multiple single unit dosages. As used herein, "unit dosage" refers to the specific amount of a pharmaceutical composition comprising a predetermined quantity of active ingredient(s), such as lipid nanoparticles. The amount of the active ingredient is generally equal to the dose of the active ingredient that can be administered to the subject, and/or to a convenient proportion of such a dose, for example, one-half or one-third of such a dose.

The pharmaceutical composition can be prepared in various forms suitable for various administration routes and methods. For example, the pharmaceutical compositions can be formulated into liquid dosage forms (such as emulsions, microemulsions, nano-emulsions, solutions, suspensions, syrups, and elixirs), injectable forms, solid dosage forms (such as capsules, tablets, pills, powders, and granules), forms for local and/or transdermal administration (such as ointments, pastes, creams, lotions, gels, powders, solutions, sprays, inhalants, and patches), suspensions, powders, and other forms.

Examples of the liquid dosage forms for oral and non-oral administrations include, but not limited to pharmaceutically acceptable emulsions, microemulsions, nano-emulsions, solutions, suspensions, syrups, and/or elixirs. In addition to the active ingredient, the liquid dosage forms may comprise inert diluents commonly used in the art, such as water or other solvents, solubilizing agents and emulsifiers, such as ethyl alcohol, isopropyl alcohol, ethyl carbonate, ethyl acetate, benzyl alcohol, benzyl benzoate, propylene glycol, 1,3-butylene glycol, dimethylformamide, oils (especially cottonseed oil, peanut oil, corn oil, germ oil, olive oil, castor oil, and sesame oil), glycerol, tetrahydrofurfuryl alcohol, fatty acid esters of polyethylene glycol and sorbitan, and mixtures thereof. In addition to the inert diluent, the oral compositions may comprise further therapeutic agents and/or prophylactic agents, as well as further agents such as moisturizers, emulsifiers and suspending agents, sweeteners, flavorings, and/or fragrances. In certain embodiments for non-oral administration, the compositions are mixed with solubilizers such as "Cremophor" (registered trademark), alcohols, oils, denatured oils, glycol, polysorbates, cyclodextrins, polymers, and/or combinations thereof.

Injectable formulations, such as sterilized aqueous or oily suspensions, can be formulated using suitable dispersants, wetting agents, and/or suspending agents according to known techniques. Sterile injectable formulations can be in the form of a sterile injectable solution, suspension, and/or emulsion in a non-toxic, non-oral acceptable diluent and/or solvent, such as a solution in 1,3-butanediol. Among the acceptable vehicles and solvents that can be used, water, Lingel solution, and isotonic sodium chloride solution are exemplified. The sterilization fixing oils are usually used as a solvent or suspension medium. For this purpose, any non-irritating fixed oils comprising synthetic mono- or di-glycerides may be used. Fatty acids such as oleic acid can be used in the preparation of injectable medicaments.

Injectable formulations can be sterilized, for example, by filtration through a filter that capture bacteria, and/or by incorporating a sterilizing agent in the form of a sterilized solid composition that can be dissolved or dispersed in sterilized water or other sterilized injection media prior to use.

The pharmaceutical compositions according to the present invention can be used to treat infectious diseases, of neoplasms, such as cancer or tumor diseases, diseases of bloods and hematopoietic organs, diseases of endocrine, nutritional intake and metabolism, diseases of nervous systems, diseases of circulatory systems, diseases of respiratory systems, diseases of digestive systems, diseases of skins and subcutaneous tissues, diseases of musculoskeletal and connective tissues, and genetic or acquired diseases (irrespective of genetic or acquired cause) such as diseases of genitourinary system.

Advantageously, therapeutic (poly)peptides or proteins encoded by nucleic acid molecules include any (poly)peptide or protein that can prevent, improve, or cure genetic or acquired diseases by their expression. In principle, such (poly)peptides or proteins can exhibit their therapeutic functions by exerting any suitable biological action or function. In some embodiments, such (poly)peptides or proteins are not necessary for the reason of replacing by preferably deficient, absent, or mutated proteins, and/or for inducing an immune or allergenic response. For example, (poly)peptides or proteins advantageous in treating genetic or acquired diseases such as infectious diseases or neoplasms may include particularly advantageous and desirable therapeutic proteins for the treatment of acquired or genetic metabolic or endocrine disorders selected from the following.

For example in the case of viral diseases, specific examples of the therapeutic (poly)peptides or proteins encoded by nucleic acid molecules include proteins or peptides characteristic in viruses that cause infectious diseases such as SARS-CoV-2 virus, influenza virus, dengue virus, and so on.

As used herein, the terms "treatment", "treating" and "treat" refer to reversing, alleviating, inhibiting the progression of, or preventing disorders or conditions to which such terms apply, or one or more symptoms of such disorders or conditions, unless otherwise specified. In one embodiment, the terms "treatment", "treating" and "treat" mean that the compound or composition of the present invention is administered to alleviate or eliminate symptoms of viral infections, and/or to reduce viral load in an individual. Treatment may be provided as a prophylactic measure before the onset of the diseases or conditions, or treatment may also be provided after the onset of the diseases.

"Preventing" and "prevent" means any procedure for a, which prevents clinical symptoms of the disease or condition from occurring. The term "prevention" also involves the administration of a compound or composition of the present invention in an effective amount to prevent the onset of disease symptoms and/or to prevent the virus from reaching detectable levels in blood before the individual is exposed to the virus (for example, pre-exposure prophylaxis).

According to the present invention, a pharmaceutical composition usually refers to a medicament for the treatment or prevention of diseases, or for the examination or diagnosis of the same.

Examples of a preferred embodiment can include a vaccine. The vaccine comprises a compound and a preparation capable of providing immunity against one or more conditions related to infectious diseases including coronaviruses such as SARS, SARS-CoV-2 and MARS, influenza viruses, measles viruses, human papillomavirus (HPV), rabies, meningitis, whooping cough, tetanus, hepatitis, and tuberculosis, and may comprise mRNA encoding antigens and/or epitopes that cause infectious diseases. The vaccine commands an immune response against cancer cells, and may also comprise a compound and a preparation that can comprise mRNA encoding antigens, epitopes, and/or neoepitopes derived from tumor cells.

In another embodiment, the present invention relates to a method for treating or preventing infectious diseases including coronaviruses such as SARS, SARS-CoV-2 and MARS, influenza viruses, measles viruses, human papillomavirus (HPV), rabies, meningitis, whooping cough, tetanus, hepatitis, and tuberculosis, as well as various types of cancer, which comprises administering the lipid nanoparticle, the pharmaceutical composition comprising the same, and/or the vaccine according to the present invention to a subject in need of such treatment and so on.

Further, in another embodiment, the present invention relates to the lipid nanoparticle, the pharmaceutical composition comprising the same, and/or the vaccine according to the present invention for treating or preventing infectious diseases including coronaviruses such as SARS, SARS-CoV-2 and MARS, influenza viruses, measles viruses, human papillomavirus (HPV), rabies, meningitis, whooping cough, tetanus, hepatitis, and tuberculosis, as well as various types of cancer.

Furthermore, in another embodiment, the present invention relates to the use of the lipid nanoparticle, the pharmaceutical composition comprising the same, and/or the vaccine according to the present invention in the manufacturing a medicament for treating or preventing infectious diseases including coronaviruses such as SARS, SARS-CoV-2 and MARS, influenza viruses, measles viruses, human papillomavirus (HPV), rabies, meningitis, whooping cough, tetanus, hepatitis, and tuberculosis, as well as various types of cancer.

### Examples

Hereinafter, the present invention will be described in more detail by way of the working examples, but it should be noted that these would not limit the scope of the present invention and merely illustrate the invention.

### Example 1: Compound 14-Zα

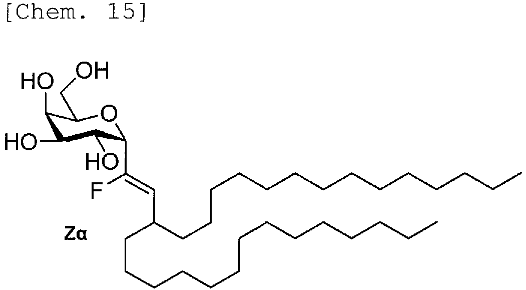

### Example 2: Compound 14E-β

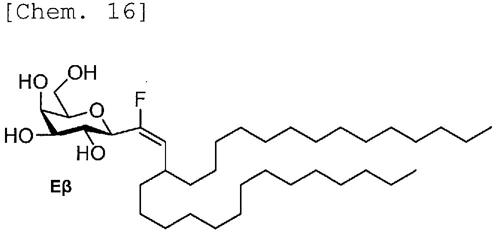

### Example 3: Compound 14-Eα

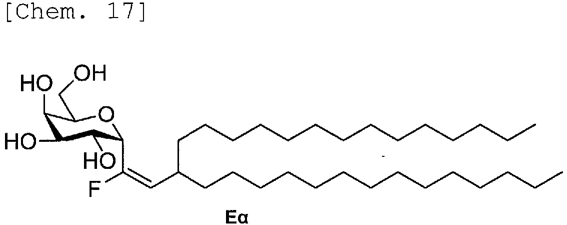

### Example 4-1: Compound 14-Zβ

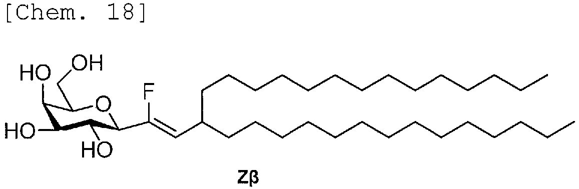

### Preparation Example 1

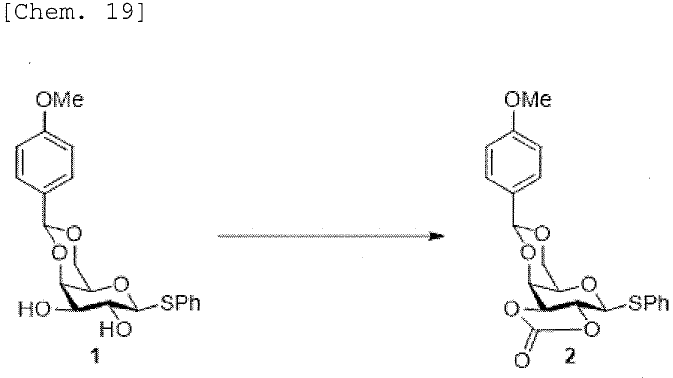

Under a nitrogen atmosphere, a solution of diol 1 (2.55 g, 6.53 µmol), pyridine (3.2 mL, 39 mmol) and N,N-dimethylaminopyridine in CH2Cl2 (30 mL) was cooled on ice, and a solution of triphosgene (1.16 g, 3.92 mmol) in CH2Cl2 (50 mL) was added dropwise thereto. The reaction solution was stirred at 0°C for 30 minutes and then poured into a saturated sodium bicarbonate solution that had been cooled on ice. After the mixture was extracted with CH2Cl2 and washing with saturated saline solution, the extract was dried over anhydrous sodium sulfate. The residue obtained by filtering off the drying agent and evaporating the solvent under reduced pressure was purified by silica gel chromatography (eluent: hexane/ethyl acetate = 1/2) to provide compound 2 (2.14 g, 79%) as white crystals.
¹H NMR (500 MHz, CDCl₃): δ 7.73-7.69 (m, 2H), 7.44-7.40 (m, 1H), 7.38-7.33 (m, 2H), 7.23-7.19 (m, 2H), 6.88-6.84 (m, 2H), 5.48 (s, 1H), 4.94 (d, J = 9.3 Hz, 1H), 4.60-4.58 (m, 1H), 4.52 (dd, J = 11.7, 9.3 Hz, 1H), 4.43 (dd, J = 12.7, 1.0 Hz, 1H), 4.34 (dd, J = 11.7, 2.4 Hz, 1H), 4.08 (dd, J = 12.7, 2.0 Hz, 1H), 3.82 (s, 3H), 3.65-3.63 (m, 1H); HRMS (ESI) Anal. Calcd for C₂₁H₂₀O₇SNa: 439.0827: Found 439.0838 (M + Na)⁺.

### Preparation Example 2

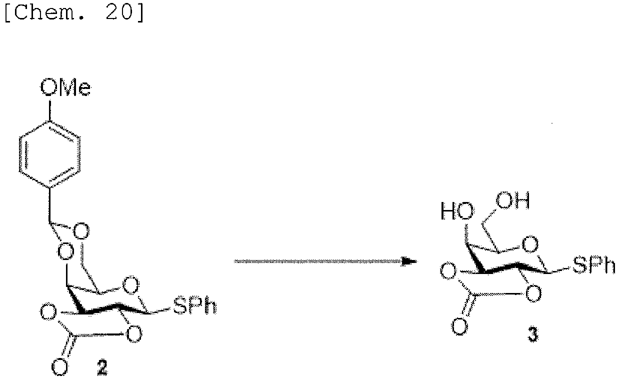

Compound 2 (4.82 g, 11.6 mmol) was added with a mixed solvent of THF (5 mL), acetic acid (40 mL) and water (5 mL) at room temperature, and was dissolved therein. After the reaction solution was stirred at room temperature for 12 hours, and was diluted with ethyl acetate, followed by adding saturated sodium bicarbonate solution thereto. The pH was checked to be 10 or more, and the solution was extracted with ethyl acetate. After washing the organic layer with saturated saline solution, the layer was dried over anhydrous sodium sulfate. The residue obtained by filtering off the drying agent and evaporating the solvent under reduced pressure was purified by silica gel chromatography (eluent: hexane/ethyl acetate = 1/3) to provide compound 3 (2.71 g, 79%) .
¹H NMR (500 MHz, CDCl₃): δ 7.61-7.57 (m, 2H), 7.41-7.33 (m, 3H), 4.94 (d, J = 9.8 Hz, 1H), 4.57-4.55 (m, 1H), 4.53 (dd, J = 11.2, 9.8 Hz, 1H), 4.29 (dd, J = 11.2, 2.4 Hz, 1H), 4.02-3.98 (m, 2H), 3.73-3.69 (m, 1H), 3.12 (d, J = 2.4 Hz, 1H), 2.04 (t, J = 6.3 Hz, 1H); HRMS (ESI) Anal. Calcd for C₁₃H₁₄O₆SNa: 321.0409: Found 321.0413 (M + Na)⁺.

### Preparation Example 3

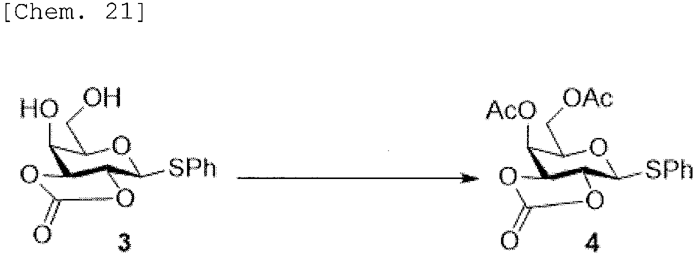

Under a nitrogen atmosphere, a solution of compound 3 (1.70 g, 5.70 mmol) in pyridine (30 mL) was cooled on ice, and anhydrous acetic acid (5.4 mL) was added dropwise thereto. After stirring at room temperature for 30 minutes, the reaction solution was diluted with ethyl acetate and poured into an ice-cold saturated sodium bicarbonate aqueous solution. The solution was extracted with ethyl acetate, and the extract was washed with saturated saline solution, followed by drying the same over anhydrous sodium sulfate. The residue obtained by filtering off the drying agent and evaporating the solvent under reduced pressure was purified by silica gel chromatography (eluent: hexane/ethyl acetate = 1/1) to provide compound 4 (2.14 g, 98%) as a colorless oily substance.
¹H NMR (500 MHz, CDCl₃): δ 7.60-7.58 (m, 2H), 7.41-7.34 (m, 3H), 5.64 (dd, J = 2.8, 1.2 Hz, 1H), 4.94 (d, J = 9.5 Hz, 1H), 4.39 (dd, J = 11.3, 2.8 Hz, 1H), 4.32 (dd, J = 11.3, 9.5 Hz, 1H), 4.18 (d, J = 6.4 Hz, 2H), 4.05 (dt, J = 6.4, 1.2 Hz, 1H), 2.10 (s, 3H), 2.06 (s, 3H); ESI-MS: m/z Anal. Calcd for C₁₇H₁₈O₈SNa: 405.0615: Found 405.0612 (M + Na)⁺.

### Preparation Example 4

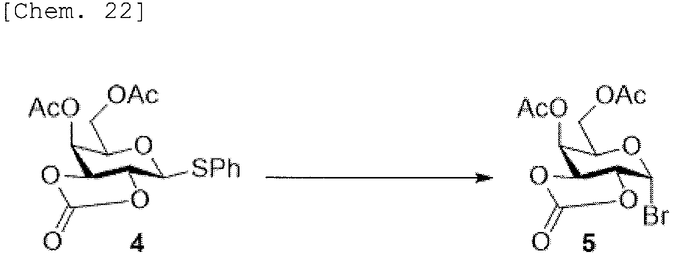

Under a nitrogen atmosphere, a solution of compound 4 (2.67 g, 6.98 mmol) in 1,2-dichloroethane (56 mL) was prepared in a two-neck flask equipped with a stir bar, a septum, and a reflux condenser. After adding activated MS3A (2.67 g) and a dichloromethane solution of HBr (1 M, 17.5 mL, 17.5 mmol) at room temperature thereto, the reaction solution was heated in an oil bath at 90°C. After heating and refluxing for 19 hours, the reaction solution was cooled to room temperature and filtered through Celite, which was then washed with dichloromethane. The filtrate was washed with a 1M sodium thiosulfate aqueous solution and a saturated saline solution, and then it was dried over anhydrous sodium sulfate. The residue obtained by filtering off the drying agent and evaporating the solvent under reduced pressure was purified by silica gel chromatography (eluent: hexane/ethyl acetate = 1/1) to provide compound 5 (1.85 g, 75%) as a white solid.
¹H NMR (500 MHz, CDCl₃): 6.67 (d, J = 3.4 Hz, 1H), 5.74-5.72 (m, 1H), 4.94 (dd, J = 11.2, 2.9 Hz, 1H), 4.67 (dd, J = 11.2, 3.4 Hz, 1H), 4.33 (ddd, J = 6.8, 6.3, 1.5 Hz, 1H), 4.24 (dd, J = 11.7, 6.3 Hz, 1H), 4.13 (dd, J = 11.7, 6.8 Hz, 1H), 2.19 (s, 3H), 2.08 (s, 3H); ESI-MS: m/z Anal. Calcd for C₁₁H₁₃BrO₈Na: 374.9686: Found 374.9685 (M + Na)⁺.

### Preparation Example 5

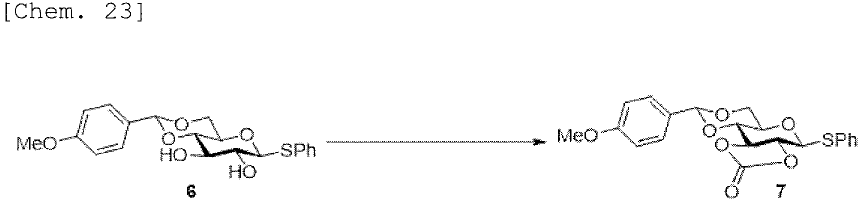

Under a nitrogen atmosphere, a solution of diol 6 (200 mg, 510 µmol) and Et₃N (260 µL, 1.9 mmol) in CH2Cl2 (1.7 mL) was cooled on ice, and a solution of triphosgene (110 mg, 380 µmol) in CH2Cl2 (600 µL) was added dropwise thereto. The reaction solution was stirred at 0°C for 30 minutes and it was poured into a saturated sodium bicarbonate aqueous solution that had been cooled with ice. The solution was extracted with CH2Cl2 and washed with saturated saline solution, and then it was dried over anhydrous sodium sulfate. The residue obtained by filtering off the drying agent and evaporating the solvent under reduced pressure was purified by silica gel chromatography (eluent: hexane/ethyl acetate = 1/2) to provide compound 7 (210 mg, quant.) as white crystals.
¹H NMR (500 MHz, CDCl₃):δ7.59-7.57 (m, 2H), 7.43-7.36 (m, 5H), 6.88 (d, J = 8.9 Hz, 2H), 5.52 (s, 1H), 4.96 (d, J = 9.8 Hz, 1H), 4.48 (t, J = 10.4, 1H), 4.39 (dd, J = 4.9, 10.4 Hz, 1H), 3.95 (dd, 8.9, 9.5 Hz, 1H), 3.88 (td, J = 4.9, 10.1 Hz, 2H), 3.80 (s, 3H), 3.61 (td, J = 4.6, 8.9 Hz, 1H); 13C NMR (125 MHz, CDCl₃): δ 160.3, 152.4, 134.8 (2C), 129.5, 129.3 (2C), 128.6, 128.4. 127.4 (2C), 113.7 (2C), 101.3, 83.5, 80.9, 78.2, 77.2, 72.8, 68.1, 55.3; ESI-MS: m/z Anal. Calcd for C₂₁H₂₀O₇SNa: 439.0827: Found 439.0809 (M + Na)⁺.

### Preparation Example 6

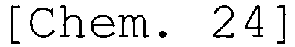

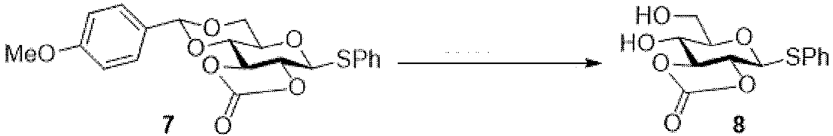

Compound 7 (4.82 g 11.6 mmol) was added to a mixed solvent of THF (5 mL), acetic acid (40 mL), and water (5 mL) at room temperature, and it was dissolved therein. The reaction solution was stirred at room temperature for 12 hours, and was diluted with ethyl acetate, followed by adding a saturated sodium bicarbonate solution thereto. The pH was checked to be 10 or more, and the solution was extracted with ethyl acetate. After washing the organic layer with saturated saline solution, the layer was dried over anhydrous sodium sulfate. The residue obtained by filtering off the drying agent and evaporating the solvent under reduced pressure was purified by silica gel chromatography (eluent: hexane/ethyl acetate = 1/3) to provide compound 8 (2.71 g, 79%).
¹H NMR (500 MHz, CDCl₃) δ 7.62-7.58 (m, 2H), 7.38-7.34 (m, 3H), 5.16 (d, J = 9.8 Hz, 1H), 4.44 (dd, J = 9.8, 11.3 Hz, 1H), 3.96 (dd, J = 9.8, 11.3 Hz, 1H), 3.91-3.86 (m, 2H), 3.75 (dd, J = 5.6, 12.2 Hz, 1H), 3.47 (ddd, J = 2.1, 5.2, 8.9 Hz, 1H); 13C NMR (125 MHz, CDCl₃): δ 155.2, 134.3 (2C), 132.3, 130.1 (2C), 129.5, 86.4, 84.0, 83.8, 78.0, 68.6, 61.8; HRMS (ESI) Anal. Calcd for C₁₃H₁₄O₆SNa: 321.0409: Found 321.0389 (M + Na)⁺.

### Preparation Example 7

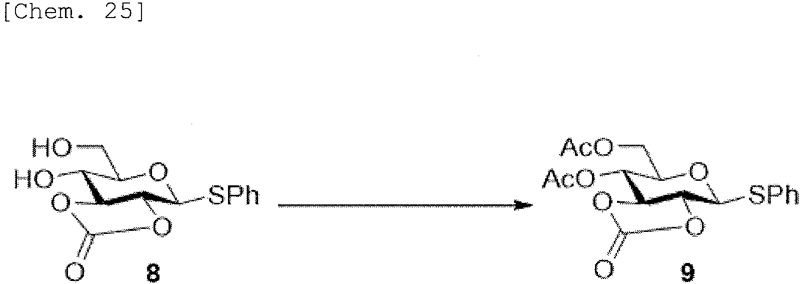

Under a nitrogen atmosphere, a solution of compound 8 (1.70 g, 5.70 mmol) in pyridine (30 mL) was cooled on ice, and anhydrous acetic acid (5.4 mL) was added dropwise thereto. After stirring at room temperature for 30 minutes, the reaction solution was diluted with ethyl acetate and poured into an ice-cold saturated sodium bicarbonate aqueous solution. The solution was extracted with ethyl acetate, and the extract was washed with saturated saline solution, followed by drying the same over anhydrous sodium sulfate. The residue obtained by filtering off the drying agent and evaporating the solvent under reduced pressure was purified by silica gel chromatography (eluent: hexane/ethyl acetate = 1/1) to provide compound 9 (2.14 g, 98%) as a colorless oily substance.
¹H NMR (500 MHz, CDCl₃) δ 7.61-7.58 (m, 2H), 7.44-7.40 (m, 1H), 7.38-7.34 (m, 2H), 5.20 (dd, J = 9.2, 10.1 Hz, 1H), 4.88 (d, J = 9.5 Hz, 1H), 4.34 (dd, J = 10.1, 11.0 Hz, 1H), 4.29 (dd, J = 2.1, 12.5 Hz, 1H), 4.20 (dd, J = 4.6, 12.5 Hz, 1H), 3.85 (dd, J = 9.5, 11.0, 1H), 3.78 (ddd, J = 2.1, 4.6, 9.2 Hz, 1H), 2.11 (s, 3H), 2.09 (s, 3H) ; 13C NMR (125 MHz, CDCl₃): δ 170.4, 168.9, 152.1, 135.3 (2C), 129.6, 129.1 (2C), 128.1, 82.4, 82.0, 77.2, 75.7, 67.1, 61.6, 20.7, 20.6; ESI-MS: m/z Anal. Calcd for C₁₇H₁₈O₈SNa: 405.0615: Found 405.0618 (M + Na)⁺.

### Preparation Example 8

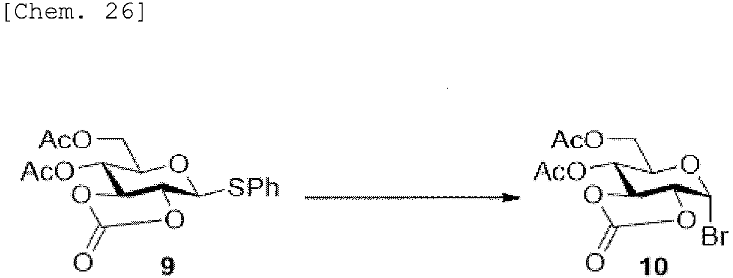

Under a nitrogen atmosphere, a solution of compound 9 (2.92 g, 7.64 mmol) in 1,2-dichloroethane (100 mL) was prepared in a two-neck flask equipped with a stir bar, a septum, and a reflux condenser. After adding activated MS3A (3.0 g) and a dichloromethane solution of HBr (1 M, 38.2 mL, 38.2 mmol) at room temperature thereto, the reaction solution was heated in an oil bath at 85°C. After heating and refluxing for 4 hours, the reaction solution was cooled to room temperature and filtered through Celite, which was then washed with dichloromethane. The filtrate was washed with a 1M sodium thiosulfate aqueous solution and a saturated saline solution, and then the same was dried over anhydrous sodium sulfate. The residue obtained by filtering off the drying agent and evaporating the solvent under reduced pressure was purified by silica gel chromatography (eluent: hexane/ethyl acetate = 1/1) to provide compound 10 (2.16 g, 80%) as a white solid.
¹H NMR (500 MHz, CDCl₃): is 6.61 (d, J = 3.4 Hz, 1H), 5.46 (t, J = 9.8 Hz, 1H), 4.34 (t, J = 3.4 Hz, 1H), 4.31 (dd, J = 1.8, 4.0 Hz, 1H), 4.19 (dd, J = 12.8, 2.1 Hz, 1H), 4.8 (ddd, J = 1.8, 4.0, 9.5 Hz, 1H), 2.15 (s, 3H), 2.10 (s, 3H); 13C NMR (125 MHz, CDCl₃): δ 170.3, 168.8, 151.6, 80.5, 78.5, 76.6, 73.7, 66.6, 60.5, 20.6, 20.5; ESI-MS: m/z Anal. Calcd for C₁₁H₁₃BrO₈Na: 374.9686: Found 374.9683 (M + Na)⁺.

### Preparation Example 9

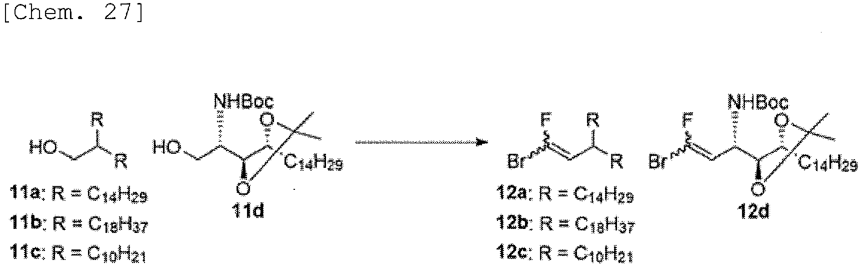

Under a nitrogen atmosphere, compound 11a (1.02 g, 2.32 mmol) was dissolved in 1,2-dichloroethane (11 mL) and dimethyl sulfoxide (11 mL) in a 100 mL flask equipped with a star bar and a septum, and 2-iodoxybenzoic acid (IBX, 847 mg, 3.02 mmol) was added thereto at room temperature. After the reaction solution was stirred at room temperature for 3 hours, hexane (30 mL) and purified water (30 mL) were added sequentially to the solution, and the obtained white solid was filtered through celite, which celite was washed with hexane. After the aqueous layer of the filtrate was extracted with hexane, the extract was dried over anhydrous sodium sulfate. The residue obtained by filtering off the drying agent and evaporating the solvent under reduced pressure was transferred to a 100 mL flask, and the solvent was removed, followed by drying the residue under vacuum for 1 hour using a vacuum pump. To a 100 mL flask equipped with a stir bar and a septum, THF (24 mL), tribromofluoromethane (1.2 mL, 4.6 mmol), and triphenylphosphine (1.22 g, 4.65 mmol) were added under a nitrogen atmosphere. A diethylzinc THF solution (1 M, 4.6 mL, 4.6 mmol) was added dropwise over 10 minutes to the adjusted solution. After stirring the reaction solution at room temperature for 2 hours, methanol (10 mL) was slowly added to the solution. After the gas generation was completed, purified water (30 mL) was added thereto and the solution was extracted with hexane. After drying the organic layer with anhydrous sodium sulfate, the drying agent was separated by filtration. The residue obtained by evaporating the solvent under reduced pressure was purified by silica gel chromatography (eluent: hexane) to provide compound 12a (1.05 g, 85%) as a white solid.

### Compound 12a:

¹H NMR (500 MHz, CDCl₃) : 5.20 (dd, J = 14.0, 10.4 Hz, 1/2H, Z), 4.74 (dd, J = 31.4, 10.4 Hz, 1/2H, E), 2.44 (m, 1/2H, E), 2.10 (m, 1/2H, Z), 1.45-1.10 (m, 52H), 0.88 (t, J = 6.9 Hz, 6H).

In accordance with a similar method to above, compound 11b (500 mg, 907 µmol) was converted to compound 12b (444 mg, 76%), compound 11c (770 mg, 2.36 mmol) was converted to compound 12c (779 mg, 79%), and compound 11d (2.84 g, 6.21 mmol) was converted to compound 12d (1.78 g, 52%).

Compound 12b:
   ¹H NMR (500 MHz, CDCl₃): δ 5.20 (dd, J = 14.0, 10.1 Hz, 1/2H, Z), 4.74 (dd, J = 31.4, 10.4 Hz, 1/2H, E), 2.48-2.39 (m, 1/2H, E), 2.15-2.05 (m, 1/2H, Z), 1.45-1.08 (m, 68H), 0.88 (t, J = 7.0 Hz, 6H).
Compound 12c:
   ¹H NMR (500 MHz, CDCl₃) : 5.21 (dd, J = 13.9, 10.0 Hz, 1/2H, Z), 4.75 (dd, J = 31.7, 10.2 Hz, 1/2H, E), 2.44 (m, 1/2H, E), 2.11 (m, 1/2H, E), 1.40-1.17 (m, 36H), 0.88 (t, J = 7.1 Hz, 6H).
Compound 12d:
   ¹H NMR (500 MHz, CDCl₃): δ 5.70 (t, J = 10.2 Hz, 1/2H), 5.22 (dd, J = 31.0, 7.8 Hz, 1/2H), 4.87 (s, 1H), 4.51 (s, 1/2H), 4.28 (s, 1/2H), 4.19-4.07 (m, 2H),
   1.64-1.49 (m, 4H), 1.46 (s, 3H), 1.44 (s, 3H), 1.34-1.20 (m, 22), 1.32 (br, 9H), 0.86 (t, J = 7.0 Hz, 3H); 19F NMR (471 MHz, CDCl₃): -66.87 (d, J = 10.2 Hz, Z-isomer), -68.26 (d, J = 31.2 Hz, E-isomer); ESI-MS (m/z) Anal. Calcd for C₂₇H₄₉BrFNNaO₄: 572.2727; Found 572.2738 (M + Na)⁺.

### Example 1 to Example 4

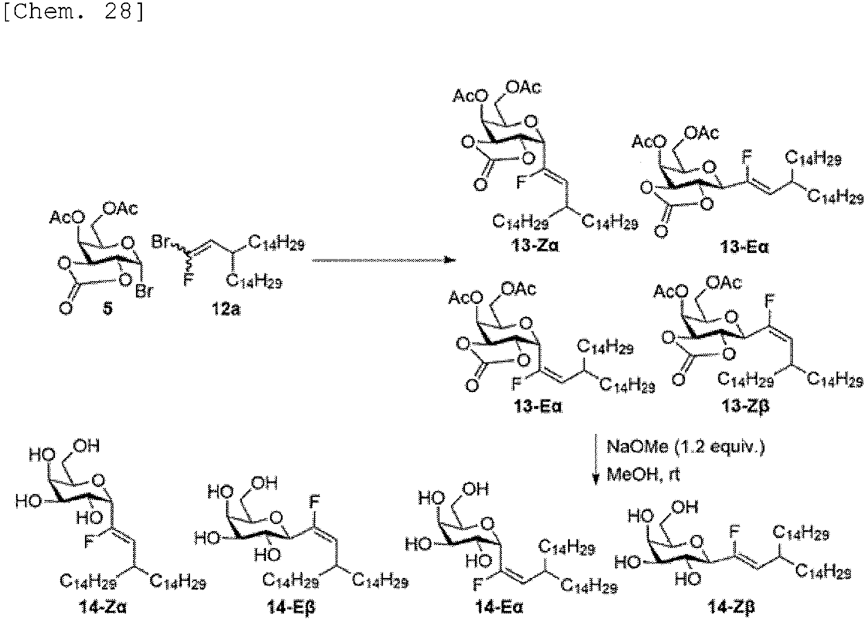

Under a nitrogen atmosphere, a solution of nickel (II) dimethoxyethane complex (6.2 mg, 28.2 µmol) and 2,10-di-tert-butylbipyridine (dtbpy, 8.3 mg, 31.0 µmol) in MeCN (3 mL) was prepared in a 20 mL screw cap vial equipped with a stir bar, which was then sealed, and the solution was sonicated for 5 minutes. Under a nitrogen stream, the MeCN was removed, and compound 5 (299 mg, 846 µmol), compound 12a (E/Z = 1:1.1, 150 mg, 282 µmol), and (Ir[dF(CF3)ppy]2(dtbpy))PF6 (15.8 mg, 14.1 µmol) were added thereto, followed by adding ethyl acetate (2.8 mL), 2,4,6-collidine (110 µL, 850 µmol), and tris(trimethylsilyl)-silanol (260 µL, 850 µmol) in sequence, and sealing the vial. The reaction solution was stirred for 24 hours under blue LED light (Kessil Tuna Blue at 465 nm), and after the light was turned off, the reaction solution was filtered through celite, which celite was then washed with a mixed solvent of ethyl acetate/hexane = 1/3. The residue obtained by evaporating the solvent under reduced pressure was purified using a recycling gel filtration purification system (HP-GPC) (eluent: chloroform) to obtain coupling product 13 as an isomeric mixture. The mixture was purified by silica gel chromatography (eluent: hexane/ethyl acetate = 90/10 -> 65/35) to obtain each of compound 13-Eα (31.4 mg, 32%), a mixture of compounds 13-Eβ and 13-Zα (99.9 mg, Eβ:Zα = 22:78, Eβ (23%), Zα (73%)), and compound 13-Zβ (7.5 mg, 7.0%).

Under a nitrogen atmosphere, a solution of compound 13-Eα (31.4 mg, 43.3 µmol) in methanol (4 mL) was prepared in a 20 mL flask equipped with a star bar, and sodium methoxide (2.8 mg, 52 µmol) was added thereto. After stirring for 16 hours, the reaction solution was added with Amberlite IRC-120, and the mixture was stirred for 1 minute, followed by filtering off the solid. The filtrate was concentrated, and the residue was purified by silica gel chromatography (eluent: hexane/ethyl acetate/methanol = 10/20/1) to provide compound 14-Eα (21.9 mg, 82%) as a white solid.

Similarly, a mixture of compound 13-Eβ and compound 13-Zα (99.9 mg, 140 µmol, Eβ:Zα = 22:78) was solvent-solubilized with sodium methoxide, and the mixture was purified by HPLC (eluent: hexane/ethyl acetate/methanol = 10/40/1) to provide compound 14-Eβ (16.6 mg, 90%) and compound 14-Zα (59.4 mg, 88%), respectively. Further, compound 13-Eβ (7.5 mg, 10 µmol) was solvent-solubilized with sodium methoxide, and the mixture was purified by silica gel chromatography (eluent: chloroform/methanol = 80:20) to provide compound 14-Zβ (6.1 mg, 100%).

### Example 3: Compound 14-Eα

¹H NMR (500 MHz, CDCl₃/CD3OD = 9/1): 5.07 (dd, J = 25.0, 11.0 Hz, 1H), 4.78 (dd, J = 36.0, 6.7 Hz, 1H), 4.03-3.97 (m, 2H), 3.94-3.89 (m, 1H), 3.83-3.78 (m, 1H), 3.74 (dd, J = 11.6, 4.9 Hz, 1H), 3.69 (dd, J = 11.6, 5.8 Hz, 1H), 2.11-2.00 (m, 1H), 1.40-1.10 (m, 52H), 0.83 (t, J = 7.0 Hz, 6H); ESI-MS: m/z Anal. Calcd for C₃₇H₇₁FNaO₅: 637.5183: Found 637.5151 (M + Na)⁺.

### Example 2: Compound 14-Eβ

¹H NMR (500 MHz, CDCl₃/CD3OD = 9/1): 5.10 (dd, J = 22.3, 10.7 Hz, 1H), 3.99-3.96 (m, 1H), 3.94-3.86 (m, 2H), 3.80 (dd, J = 11.9, 5.8 Hz, 1H), 3.73 (dd, J = 11.9, 5.2 Hz, 1H), 3.51-3.46 (m, 2H), 2.12-2.02 (m, 1H), 1.40-1.10 (m, 52H), 0.84 (t, J = 7.0 Hz, 6H); ESI-MS: m/z Anal. Calcd for C₃₇H₇₁FNaO₅: 637.5183: Found 637.5150 (M + Na)⁺.

### Example 1: Compound 14-Zα

¹H NMR (500 MHz, CDCl₃/CD3OD = 9/1): 4.74 (dd, J = 38.5, 10.4 Hz, 1H), 4.46 (dd, J = 24.4, 6.1 Hz, 1H), 4.01-3.97 (m, 2H), 3.87 (ddd, J = 1.2, 3.4, 13.1 Hz, 1H), 3.84-3.80 (m, 1H), 3.76 (dd, J = 11.6, 5.5 Hz, 1H), 3.69 (dd, J = 11.3, 5.5 Hz, 1H), 2.49-2.39 (m, 1H), 1.38-1.08 (m, 52H), 0.83 (t, J = 6.7 Hz, 6H); ESI-MS: m/z Anal. Calcd for C₃₇H₇₁FNaO₅: 637.5183: Found 637.5149 (M + Na)⁺.

### Example 4-1: Compound 14-zβ

¹H NMR (500 MHz, CDCl₃/CD3OD = 9/1): 4.66 (dd, J = 37.2, 10.1 Hz, 1H), 3.91-3.89 (m, 1H), 3.79-3.73 (m, 2H), 3.69 (dd, 11.6, 5.5 Hz, 1H), 3.57 (dd, J = 21.7, 9.8 Hz, 1H), 3.49-3.46 (m, 1H), 3.43 (dd, J = 9.5, 3.4 Hz, 1H), 2.49-2.40 (m, 1H), 1.40-1.10 (m, 52H), 0.83 (t, J = 7.2 Hz, 6H); ESI-MS: m/z Anal. Calcd for C₃₇H₇₁FNaO₅: 637.5183: Found 637.5151 (M + Na)⁺.

### Example 4-2 and Example 4-3

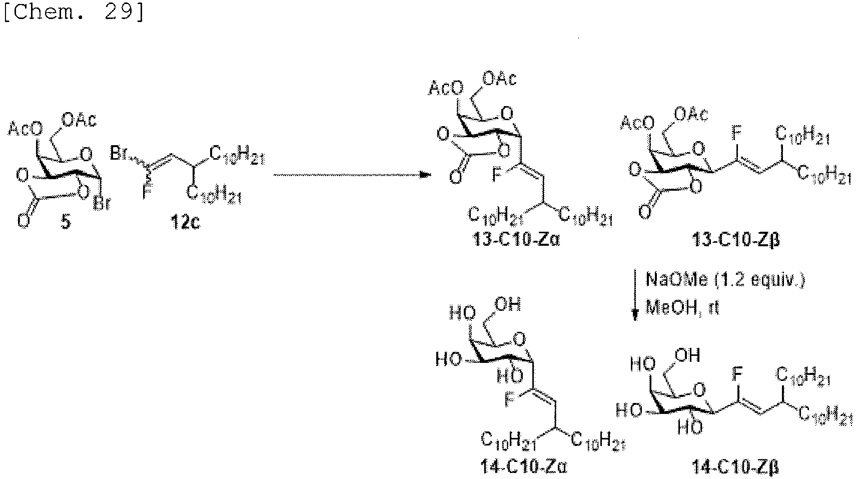

Under a nitrogen atmosphere, a solution of nickel (II) dimethoxyethane complex (5.2 mg, 24 µmol) and 2,10-di-tert-butylbipyridine (dtbpy, 7.0 mg, 26 µmol) in MeCN (3 mL) was prepared in a 20 mL screw cap vial equipped with a stir bar, which was then sealed, and the solution was sonicated for 5 minutes. Under a nitrogen stream, the MeCN was removed, and a stir bar, compound 5 (130 mg, 360 µmol), compound 12c (E/Z = 1:1, 50 mg, 120 pmol), and (Ir[dF(CF₃)ppy₂(dtbpy))PF₆ (6.7 mg, 6.0 µmol) were added thereto, followed by adding ethyl acetate (1.2 mL), 2,4,6-collidine (47 µL, 360 µmol), and tris(trimethylsilyl)-silanol (110 µL, 360 µmol) in sequence, and sealing the vial. The reaction solution was stirred for 24 hours under blue LED light (Kessil Tuna Blue at 465 nm), and after the light was turned off, the reaction solution was filtered through celite, which celite was then washed with a mixed solvent of ethyl acetate/hexane = 1/3. The residue obtained by evaporating the solvent under reduced pressure was purified using a recycling gel filtration purification system (HP-GPC) (eluent: chloroform) to obtain compound 13-C10 of the coupling product as an isomer mixture (171 mg), and compound 13-C10-zβ (21.7 mg) with impurities. 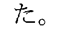

Under a nitrogen atmosphere, a solution of the isomer mixture of compound 13-C10 (171 mg) in methanol (8 ml) was prepared in a 10 mL flask equipped with a star bar, and sodium methoxide (25.5 mg, 471 µmol) was added thereto. After stirring for 16 hours, the reaction solution was added with Amberlite IRC-120, and the mixture was stirred for 1 minute, followed by filtering off the solid. The filtrate was concentrated, and the residue was purified by silica gel chromatography (eluent: hexane/ethyl acetate/methanol = 10/20/1) to provide compound 14-C10-Zα (80.5 mg, 64%) as a white solid.

Similarly, the compound 13-C10-Zβ (21.7 mg) with impurities was solvent-solubilized with sodium methoxide (3.2 mg, 60 µmol), and the mixture was purified by HPLC (eluent: chloroform/methanol = 90:10) to provide compound 14-C10-Zβ (5.3 mg, 35%).

### Example 4-2: Compound 14-C10-Zα

¹H NMR (500 MHz, CDCl₃/CD3OD = 9/1): 5.07 (dd, J = 25.0, 11.0 Hz, 1H), 4.78 (dd, J = 36.0, 6.7 Hz, 1H), 4.03-3.97 (m, 2H), 3.94-3.89 (m, 1H), 3.83-3.78 (m, 1H), 3.74 (dd, J = 11.6, 4.9 Hz, 1H), 3.69 (dd, J = 11.6, 5.8 Hz, 1H), 2.11-2.00 (m, 1H), 1.40-1.10 (m, 52H), 0.83 (t, J = 7.0 Hz, 6H); ESI-MS: m/z Anal. Calcd for C₂₉H₅₅FNaO₅: 525.3931: Found 525.3925 (M + Na)⁺.

### Example 4-3: Compound 14-C10-Z β

¹H NMR (500 MHz, CDCl₃/CD3OD = 9/1): 4.67 (dd, J = 37.4, 10.2 Hz, 1H), 3.95 (d, J = 3.4 Hz, 1H), 3.84-3.77 (m, 2H), 3.74 (dd, J = 11.9, 4.9 Hz, 1H), 3.58 (dd, J = 21.7, 9.8 Hz, 1H), 3.51-3.45 (m, 2H), 2.50-2.41 (m, 1H), 1.34-1.13 (m, 36H), 0.84 (t, J = 6.9 Hz, 6H); ESI-MS: m/z Anal. Calcd for C₂₉H₅₅FNaO₅: 525.3931: Found 525.3927 (M + Na)⁺.

### Example 5: Compound 15α

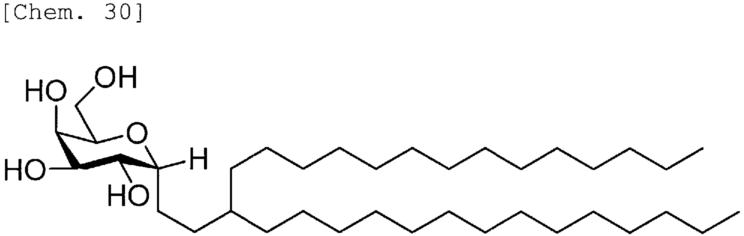

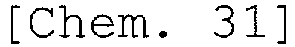

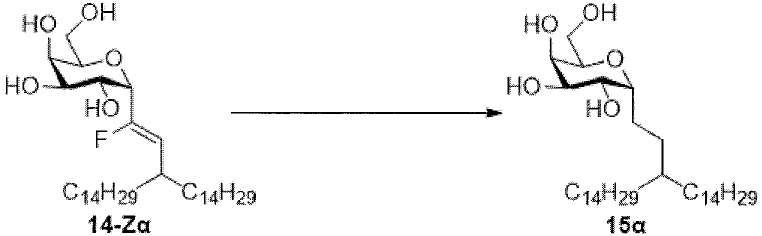

In a two-necked flask equipped with a star bar and a septum, a solution of compound 14-Zα (31.2 mg, 50.7 µmol) and triethylamine (14 µL, 100 µmol) in ethyl acetate (2.0 mL) was prepared. Platinum carbon (Pt 5 wt%, 62.4 mg) was added thereto at room temperature, and the septum was replaced with a three-way cock. After subjecting the reaction vessel to hydrogen substitution, the reaction mixture was stirred for 24 hours, and the solution was filtered through celite, which celite was then washed with chloroform/methanol = 9/1. The residue obtained by evaporating the solvent under reduced pressure was purified by silica gel chromatography (eluent: chloroform/methanol = 9/1) to provide compound 15α (25.4 mg, 84%) as a white solid.
¹H NMR (500 MHz, CDCl₃/CD3OD = 20:1): 4.00-3.92 (m, 3H), 3.83 (dd, J = 11.6, 5.5 Hz, 1H), 3.75 (dd, J = 11.6, 4.9 Hz, 1H), 3.64 (dd, J = 8.9, 3.4 Hz, 1H), 3.61-3.57 (m, 1H), 1.58-1.14 (m, 57H), 0.85 (t, J = 6.7 Hz, 6H); ESI-MS: m/z Anal. Calcd for C₃₇H₇₄NaO₅: 621.5428: Found 621.5402 (M + Na)⁺.

### Example 6: Compound 15β

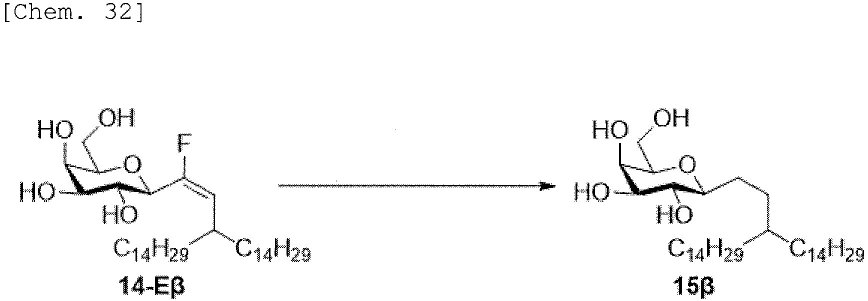

In a two-necked flask equipped with a star bar and a septum, a solution of compound 14-Eβ (3.2 mg, 5.2 µmol) and triethylamine (3.6 µL, 26 µmol) in ethyl acetate (1.0 mL) was prepared. Rhodium alumina (Rh 5 wt%, 7.0 mg) was added thereto at room temperature, and the septum was replaced with a three-way cock. After subjecting the reaction vessel to hydrogen substitution, the reaction mixture was stirred for 17 hours, and the solution was filtered through celite, which celite was then washed with chloroform/methanol = 9/1. The residue obtained by evaporating the solvent under reduced pressure was purified by silica gel chromatography (eluent: chloroform/methanol = 9/1) to provide compound 15β (1.7 mg, 55%) as a white solid.
¹H NMR (500 MHz, CDCl₃/CD3OD = 20:1): 3.92 (d, J = 2.1 Hz, 1H), 3.78 (dd, J = 11.6, 5.8 Hz, 1H), 3.70 (dd, J = 11.6, 5.2 Hz, 1H), 3.42-3.37 (m, 2H), 3.08-3.01 (m, 1H), 1.84-1.76 (m, 1H), 1.60-1.50 (m, 2H), 1.48-1.35 (m, 2H), 1.29-1.15 (m, 52H), 1.05-0.93 (m, 1H), 0.83 (t, J = 7.0 Hz, 6H); ESI-MS: m/z Anal. Calcd for C₃₇H₇₄NaO₅: 621.5428: Found 621.5406 (M + Na)⁺.

### Examples 7 to 10

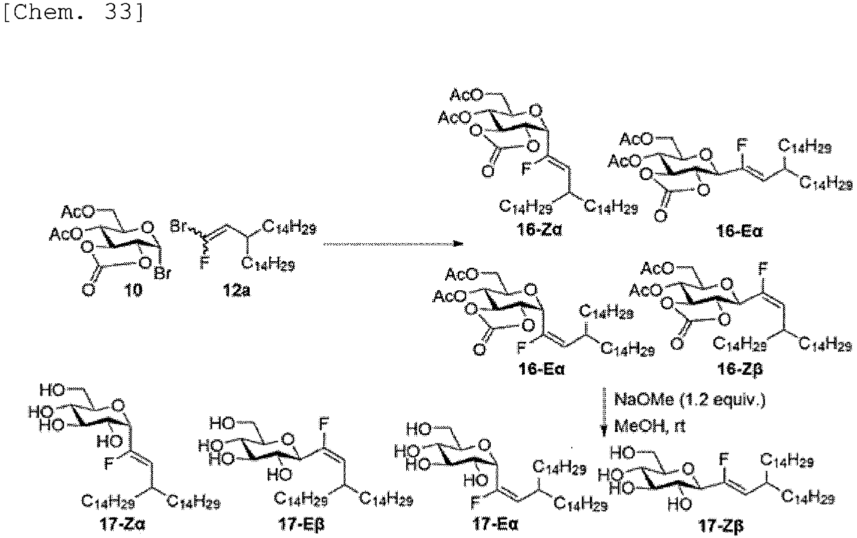

Under a nitrogen atmosphere, a solution of nickel (II) dimethoxyethane complex (4.1 mg, 19 µmol) and 2,10-di-tert-butylbipyridine (dtbpy, 5.5 mg, 20 µmol) in MeCN (2 mL) was prepared in a 3 mL screw cap vial equipped with a stir bar, which was then sealed, and the solution was sonicated for 5 minutes. Under a nitrogen stream, the MeCN was removed, and compound 10 (100 mg, 280 µmol), compound 12a (E/Z = 1.1:1, 50 mg, 94 µmol), and (Ir[dF(CF3)ppy]2(dtbpy))PF6 (5.3 mg, 4.7 µmol) were added thereto, followed by adding ethyl acetate (1.9 mL), triethylamine (29 µL, 280 µmol), and tris(trimethylsilyl)-silanol (75 µL, 280 µmol) in sequence, and sealing the vial. Six similar vials were prepared (using a total of 300 mg 12), and were irradiated with blue LED light (Kessil Tuna Blue at 465 nm). After stirring the reaction solutions for 24 hours, and turning off the light, all of the reaction solutions were collected and the collection was filtered through celite, which celite was then washed with a mixed solvent of ethyl acetate/hexane = 1/3. The residue obtained by evaporating the solvent under reduced pressure was purified by silica gel chromatography (eluent: hexane/ethyl acetate = 82/18 -> 60/40) to obtain a mixture of compound 16-Eα with impurities, compound 16-Eβ, and compound 16-Zα with impurities (218 mg), and compound 16-Zβ with impurities (33.5 mg) respectively.

Under a nitrogen atmosphere, a solution of a mixture of compounds 16-Eα, 16-Eβ, and 16-Zα in methanol (15 mL) was prepared in a 20 mL flask equipped with a star bar, and sodium methoxide (32.5 g, 601 µmol) was added thereto. After stirring for 16 hours, the reaction solution was added with Amberlite IRC-120, and the mixture was stirred for 1 minute, followed by filtering off the solid. The filtrate was concentrated, and the residue was purified by silica gel by HPLC (eluent: hexane/ethyl acetate/methanol = 20/40/1) to provide compound 17-Eα (13.8 mg, 8%, 2 steps), compound 17-Eβ (9.0 mg, 6%, 2 steps), and compound 17-Zα (114 mg, 63%, 2 steps) as white solids.

Similarly, compound 16-Zβ with impurities was solvent-solubilized with sodium methoxide (5.0 mg, 92 µmol), and the mixture was purified by silica gel chromatography (eluent: chloroform/methanol = 9/1) to provide compound 17-Zβ (10.9 mg, 6%, 2 steps).

### Example 7: Compound 17-Eα

¹H NMR (500 MHz, CDCl₃/CD3OD = 20:1): 5.08 (dd, J = 24.7, 10.7 Hz, 1H), 4.72 (dd, J = 35.1, 7.0 Hz, 1H), 3.85 (ddd, J = 9.5, 9.5, 3.4 Hz, 1H), 3.75-3.67 (m, 3H), 3.62-3.56 (m, 1H), 3.45-3.39 (m, 1H), 2.05 (brs, 1H), 1.40-1.08 (m, 52H), 0.83 (t, J = 7.0 Hz, 6H); ESI-MS: m/z Anal. Calcd for C₃₇H₇₁FNaO₅: 637.5183: Found 637.5151 (M + Na)⁺.

### Example 8: Compound 17-Eβ

¹H NMR (500 MHz, CDCl₃/CD3OD = 20:1): 5.09 (dd, J = 22.3, 11.0 Hz, 1H), 3.96 (dd, J = 25.9, 9.8 Hz, 1H), 3.78 (dd, J = 12.2, 3.1 Hz, 1H), 3.72 (dd, J = 12.2, 4.0 Hz, 1H), 3.59 (dd, J = 8.5 Hz, 1H), 3.49-3.40 (m, 2H), 3.26 (ddd, J = 8.5, 4.0, 3.1 Hz, 1H), 2.06 (brs, 1H), 1.40-1.10 (m, 52H), 0.83 (t, J = 6.7 Hz, 6H); ESI-MS: m/z Anal. Calcd for C₃₇H₇₁FNaO₅: 637.5183: Found 637.5141 (M + Na)⁺.

### Example 9: Compound 17-Zα

¹H NMR (500 MHz, CDCl₃/CD3OD = 20:1): 4.75 (dd, J = 38.2, 10.1 Hz, 1H), 4.41 (dd, J = 25.6, 6.7 Hz, 1H), 3.85-3.80 (m, 1H), 3.75-3.73 (m, 2H), 3.71 (dd, J = 9.2, 6.7 Hz, 1H), 3.64-3.58 (m, 1H), 3.42 (dd, J = 9.0, 9.0 Hz, 1H), 2.50-2.40 (m, 1H), 1.40-1.10 (m, 52H), 0.84 (t, J = 6.7 Hz, 6H); ESI-MS: m/z Anal. Calcd for C₃₇H₇₁FNaO₅: 637.5183: Found 637.5140 (M + Na)⁺.

### Example 10: Compound 17-Zβ

¹H NMR (500 MHz, CDCl₃/CD3OD = 20:1): 4.67 (dd, J = 37.5, 10.1 Hz, 1H), 3.83 (dd, J = 12.2, 3.1 Hz, 1H), 3.73 (dd, J = 12.2, 4.6 Hz, 1H), 3.66 (dd, J = 21.7, 9.8 Hz, 1H), 3.53 (dd, J = 8.9, 8.9 Hz, 1H), 3.48-3.41 (m, 2H), 3.32-3.27 (ddd, J = 8.9, 4.6, 3.1 Hz, 1H), 2.51-2.42 (m, 1H), 1.40-1.13 (m, 52H), 0.85 (t, J = 6.7 Hz, 6H); ESI-MS: m/z Anal. Calcd for C₃₇H₇₁FNaO₅: 637.5183: Found 637.5139 (M + Na)⁺.

### Example 11: Compound 18a

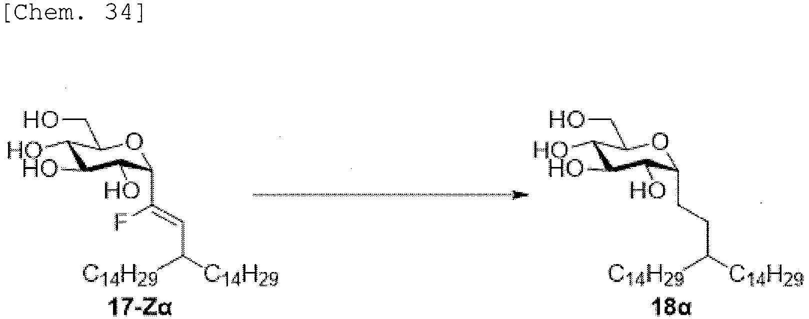

A solution of compound 17-Zα (50.0 mg, 81.0 µmol) and triethylamine (57 µL, 410 µmol) in ethyl acetate (16 mL) was prepared in a two-neck flask equipped with a star bar and a septum. Rhodium alumina (Pt 5 wt%, 62.4 mg) was added thereto at room temperature, and the septum was replaced with a three-way cock. After subjecting the reaction vessel to hydrogen substitution, the reaction mixture was stirred for 24 hours, and the solution was filtered through celite, which celite was then washed with chloroform/methanol = 9/1. The residue obtained by evaporating the solvent under reduced pressure was purified by silica gel chromatography (eluent: chloroform/methanol = 9/1) to provide compound 18α (18.9 mg, 39%) as a white solid.
¹H NMR (500 MHz, CDCl₃/CD3OD = 20:1): 3.90-3.83 (m, 1H), 3.73 (m, 2H), 3.62 (dd, J = 9.5, 5.8 Hz, 1H), 3.56 (dd, J = 9.5, 7.9 Hz, 1H), 3.41-3.35 (m, 2H), 1.62-1.48 (m, 2H), 1.40-1.30 (m, 2H), 1.30-1.15 (m, 53H), 0.84 (t, J = 6.4 Hz, 6H); ESI-MS: m/z Anal. Calcd for C₃₇H₇₄NaO₅: 621.5428: Found 621.5420 (M + Na)⁺.

### Examples 12 to 15

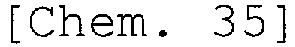

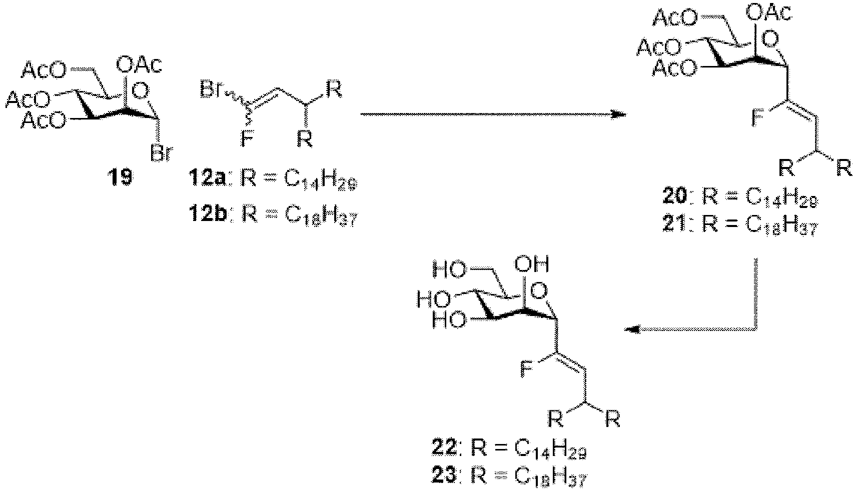

Under a nitrogen atmosphere, a solution of nickel (II) dimethoxyethane complex (6.2 mg, 28.2 µmol) and 2,10-di-tert-butylbipyridine (dtbpy, 8.3 mg, 31.0 µmol) in MeCN (3 mL) was prepared in a 10 mL screw cap vial, which was then sealed, and the solution was sonicated for 5 minutes. Under a nitrogen stream, the MeCN was removed, and a stir bar, compound 19 (464 mg, 1.13 mmol), compound 12a (E/Z = 1:1, 300 mg, 564 µmol), and (Ir[dF(CF₃)ppy]₂(dtbpy))PF₆ (6.3 mg, 5.6 µmol) were added thereto, followed by adding a degassed ethyl acetate (5.6 mL), 2,4,6-collidine (150 µL, 1.1 µmol), and tris(trimethylsilyl)silanol (350 µL, 1.1 µmol) in sequence, and sealing the vial. The reaction solution was stirred for 24 hours under blue LED light (Kessil Tuna Blue at 465 nm), and after the light was turned off, the reaction solution was filtered through celite, which celite was then washed with a mixed solvent of ethyl acetate/hexane = 1/3. The residue obtained by evaporating the solvent under reduced pressure was purified using a recycling gel filtration purification system (HP-GPC) (eluent: chloroform). The obtained mixture was purified by silica gel chromatography (eluent: hexane/ethyl acetate = 90/10 -> 65/35) to provide 20 (192 mg, 87%).

Under a nitrogen atmosphere, a solution of 20 (155 mg, 198 µmol) in a mixed solvent of methanol (5 mL) and dichloromethane (5 mL) was prepared in a 20 mL flask equipped with a star bar,, and sodium methoxide (2.1 g, 40 µmol) was added thereto. After stirring for 12 hours, the reaction solution was added with Amberlite IRC-120, and the mixture was stirred for 1 minute, followed by filtering off the solid. The filtrate was concentrated, and the residue was purified by silica gel (Iatrobeads (registered trademark) chromatography (eluent: chloroform/methanol = 19/1) to provide 22 (115 mg, 94%) as a white solid.

Similarly, compound 19 (128 mg, 311 µmol) and compound 12b (E/Z = 1.1:1, 100 mg, 155 µmol) were reacted to provide compound 21 (53 mg, 72%). Further, compound 21 (13.6 mg, 15.2 µmol) was reacted with sodium methoxide to provide compound 23 (9.9 mg, 90%).

### Example 12: Compound 20:

¹H NMR (500 MHz, CDCl₃): 5.52 (t, J = 3.2 Hz, 1H), 5.27 (dd, J = 8.6, 3.4 Hz, 1H), 5.23 (t, J = 7.9 Hz, 1H), 4.81 (dd, J = 37.5, 10.4 Hz, 1H), 4.55 (dd, J = 11.0, 3.1 Hz, 1H), 4.40 (dd, J = 12.2, 6.4 Hz, 1H), 4.09 (dd, J = 12.2, 2.8 Hz, 1H), 3.97-3.93 (m, 1H), 2.59-2.49 (m, 1H), 2.14 (s, 3H), 2.09 (s, 3H), 2.06 (s, 3H), 2.03 (s, 3H), 1.45-1.18 (m, 54H), 0.88 (t, J = 6.7 Hz, 6H); ESI-MS (m/z) Anal. Calcd for C₄₅H₇₉FNaO₉: 805.5606; Found: 805.5612 (M + Na)⁺.

### Example 13: Compound 21:

¹H NMR (500 MHz, CDCl₃/CD3OD = 9/1): 4.53 (dd, J = 37.7, 10.2 Hz, 2H), 4.06 (d, J = 1.5 Hz, 1H), 3.76-3.69 (m, 3H), 3.64 (dd, J = 9.3, 3.2 Hz, 1H), 3.44 (d, J = 9.2 Hz, 1H), 1.30-1.16 (m, 57H), 0.79 (t, J = 7.0 Hz, 6H); ESI-MS (m/z) Anal. Calcd for C₃₇H₇₁FNaO₅: 637.5183; Found : 637.5165 (M + Na)⁺.

### Example 14: Compound 22:

¹H NMR (500 MHz, CDCl₃): 5.51 (dd, J = 3.4, 3.4 Hz, 1H), 5.28-5.20, (m, 2H), 4.80 (dd, J = 37.5, 10.4 Hz, 1H), 4.55 (dd, J = 11.0, 3.4 Hz, 1H), 4.40 (dd, J = 12.2, 6.4 Hz, 1H), 4.08 (dd, J = 12.2, 3.1 Hz, 1H), 3.97-3.92 (m, 1H), 2.58-2.49 (m, 1H), 2.13 (s, 3H), 2.09 (s, 3H), 2.06 (s, 3H), 2.03 (s, 3H), 1.45-1.08 (m, 76H), 0.87 (t, J = 6.9 Hz, 7H); ESI-MS (m/z) Anal. Calcd for C₅₃H₉₅FNaO₉: 917.6858; Found : 917.6825 (M + Na)⁺.

### Example 15: Compound 23:

¹H NMR (500 MHz, CDCl₃/CD3OD = 19/1): 4.56 (dd, J = 38.8, 10.4 Hz, 1H), 4.52 (d, J = 9.8 Hz, 1H), 4.10-4.08 (m, 1H), 3.80-3.71 (m, 3H), 3.67 (dd, J = 9.2, 3.1 Hz, 1H), 3.47 (d, J = 9.5 Hz, 1H), 2.44 (q, J = 4.5 Hz, 1H), 1.34-1.20 (m, 74H), 0.82 (t, J = 6.9 Hz, 6H); ESI-MS (m/z) Anal. Calcd for C₄₅H₈₇FNaO₅: 749.6435; Found : 749.6420 (M + Na)⁺.

### Example 16

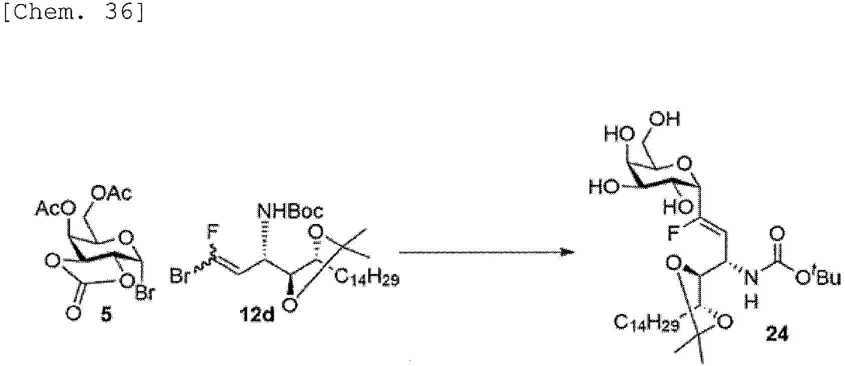

Under a nitrogen atmosphere, a solution of nickel (II) dimethoxyethane complex (12.0 mg, 54.5 µmol) and 2,10-di-tert-butylbipyridine (dtbpy, 16.1 mg, 59.9 µmol) in MeCN (5 mL) was prepared in a 10 mL screw cap vial, which was then sealed, and the solution was sonicated for 5 minutes. The MeCN was evaporated under reduced pressure, and compound 5 (481 mg, 1.36 µmol), compound 12d (E/Z = 1:1, 300 mg, 545 µmol), and (Ir[dF(CF₃)ppy]₂(dtbpy))PF₆ (30.6 mg, 27.2 µmol) were added thereto, followed by adding a degassed 1,4-dioxane (5.5 mL), 2,4,6-collidine (180 µL, 1.4 mmol), and tris(trimethylsilyl)silanol (420 µL, 1.4 mmol) in sequence, and sealing the vial. The reaction solution was stirred for 24 hours under blue LED light (Kessil Tuna Blue at 465 nm), and after the light was turned off, the reaction solution was filtered through celite, which celite was then washed with a mixed solvent of ethyl acetate/hexane = 1/3. The residue obtained by evaporating the solvent under reduced pressure was purified using a recycling gel filtration purification system (HP-GPC) (eluent: chloroform). The obtained mixture was dissolved in methanol (5 mL) under a nitrogen atmosphere in a 20 mL flask, and sodium methoxide (58.9 mg, 1.09 mmol) was added thereto. After stirring for 12 hours, the reaction solution was added with Amberlite IRC-120, and the mixture was stirred for 1 minute, followed by filtering off the solid. The filtrate was concentrated, and the residue was purified by silica gel chromatography (eluent: hexane/ethyl acetate/methanol = 10/20/1) (eluent: ethyl acetate/hexane/methanol = 4/1/0.1) to provide compound 24 (91.3 mg, 54%) as a white solid.

### Compound 24:

¹H NMR (500 MHz, CDCl₃/CD3OD = 2/1): 5.14 (dd, J = 37.8, 9.5 Hz, 1H), 4.77 (dd, J = 9.5, 3.4 Hz, 1H), 4.51 (dd, J = 17.4, 4.9 Hz, 1H), 4.00 (dd, J = 3.4, 3.4 Hz, 1H), 3.98-3.95 (m, 1H), 3.94-3.89 (m, 1H), 3.89 (dd, J =8.2, 3.4 Hz, 1H), 3.85 (dd, J = 11.9, 7.0 Hz, 1H), 3.69 (dd, J = 11.9, 4.0 Hz, 1H), 3.44 (dd, J = 7.9, 3.4 Hz, 1H), 3.36 (ddd, J = 8.5, 7.9, 2.4 Hz, 1H), 1.74-1.66 (m, 1H), 1.55-1.47 (m, 1H), 1.42 (s, 9H), 1.37-1.19 (m, 24H), 1.30 (s, 6H), 0.85 (t, J = 6.9 Hz, 3H); ESI-MS (m/z) Anal. Calcd for C₃₃H₆₀FNNaO₉: 656.4150; Found: 656.4148 (M + Na)⁺.

### Example 17 and 18

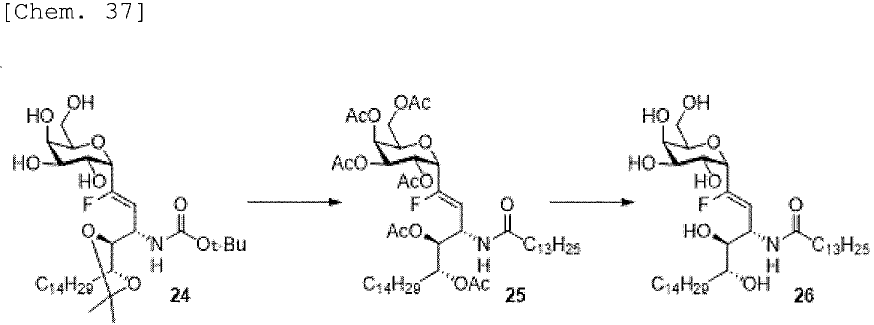

In a 4 mL screw cap vial equipped with a star bar, a solution of compound 24 (13.3 mg, 21.0 µmol) prepared in Example 16 in a mixed solvent of trifluoroacetic acid (150 µL) and a distilled water (50 µL). After the solution was stirred at room temperature for 3 hours, TFA and water was removed under a nitrogen stream, and the residue was dissolved in methanol (100 µL). Under a nitrogen stream, methanol was removed, and the residue was dissolved in THF (300 µL), followed by adding myristic acid-N-hydroxysuccinimide ester (10.2 mg, 31.5 µmol) and saturated sodium bicarbonate solution (100 µL) were added thereto. The reaction solution was stirred at room temperature for 12 hours, and 100 mg of silica gel was added to the reaction solution, followed by evaporating THF under reduced pressure. The residue was filtered through a column packed with silica gel (1 cc), and the silica gel was washed with chloroform/methanol = 9/1. The residue obtained by evaporating the solvent under reduced pressure was dissolved in pyridine (300 µL), and anhydrous acetic acid (100 µL) was added thereto at 0°C. After the reaction solution was stirred at room temperature for 12 hours, the solution was diluted with ethyl acetate and a saturated aqueous solution of sodium hydrogen carbonate was poured into the diluted solution. The mixture was extracted with ethyl acetate, and the extract was washed with saturated saline solution, followed by drying the same over anhydrous sodium sulfate. The residue obtained by filtering off the drying agent and evaporating the solvent under reduced pressure was purified by silica gel chromatography (eluent: hexane/ethyl acetate = 3/1) to provide compound 25 (7.1 mg, 35%) a white solid.

In a 4 mL screw cap vial equipped with a star bar, a solution of compound 25 (4.1 mg, 4.3 µmol) in methanol (300 µL) was prepared, and sodium methoxide (1.2 mg, 21 µmol) was added thereto. After the reaction solution was stirred at room temperature for 12 hours, Amberlite IRC-120 was added thereto and the mixture was stirred for 1 minute, followed by filtering off the solid. The filtrate was purified by silica gel (Iatrobeads (registered trademark)) chromatography (eluent: chloroform/methanol/distilled water = 19/1/0.05) to provided compound 26 (3.0 mg, 100%) as a white solid.

### Example 17: Compound 25:

¹H NMR (500 MHz, CDCl₃): 5.91 (d, J = 8.9 Hz, 1H), 5.48 (dd, J = 3.7, 1.5 Hz, 1H), 5.42 (dd, J = 10.1, 3.7 Hz, 1H), 5.21 (dd, J = 10.1, 6.4 Hz, 1H), 5.12-5.04 (m, 2H), 4.94-4.89 (m, 1H), 4.87 (dd, J = 31.7, 8.9 Hz, 1H), 4.78 (dd, J = 27.8, 6.4 Hz, 1H), 4.27 (m, 1H), 4.11-4.08 (m, 2H), 2.18-2.10 (m, 2H), 2.13 (s, 3H), 2.12 (s, 3H), 2.07 (s, 3H), 2.044 (s, 3H), 2.040 (s, 3H), 2.01 (s, 3H), 1.67-1.50 (m, 4H), 1.32-1.23 (m, 44H), 0.88 (t, J = 6.9 Hz, 6H); ESI-MS (m/z) Anal. Calcd for C₅₁H₈₆FNNaO₁₄: 978.5930; Found: 978.5897 (M + Na)⁺.

### Example 18: Compound 26:

¹H NMR (500 MHz, CDCl₃): 8.77 (d, J = 7.9 Hz, 1H), 6.92-6.81 (m, 2H), 6.78 (brd, J = 4.3 Hz, 1H), 6.53 (brd, J = 2.4 Hz, 1H), 6.45 (brs, 1H), 6.35 (dd, J = 39.2, 9.9 Hz, 1H), 6.24 (td, J = 8.9, 2.5 Hz, 1H), 6.12 (s, 1H), 5.21 (dd, J = 12.8, 4.3 Hz, 1H), 4.81-4.75 (m, 2H), 4.68-4.59 (m, 3H), 4.54-4.48 (m, 1H), 4.39-4.34 (m, 1H), 4.28-4.21 (m, 1H), 2.40 (t, J = 7.6 Hz, 2H), 2.32-2.23 (m, 1H), 1.96-1.84 (m, 2H), 1.80 (quin, J = 7.6 Hz, 2H), 1.65-1.61 (m, 1H), 1.46-1.17 (m, 47H), 0.88 (t, J = 6.9 Hz, 6H); ESI-MS (m/z) Anal. Calcd for C₃₉H₇₄FNNaO₈: 726.5296; Found: 726.5282 (M + Na)⁺.

### Example 19

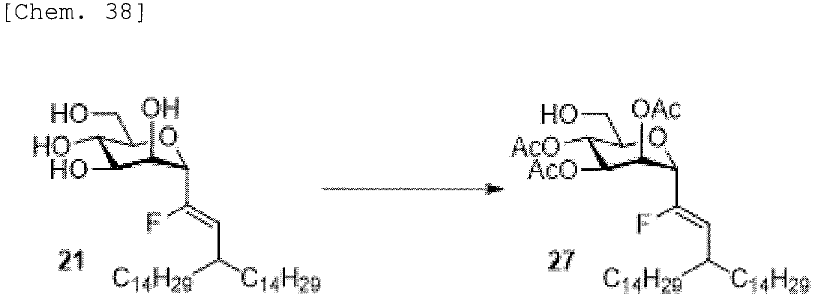

Under an argon atmosphere, a solution of compound 21 (47.7 mg, 77.6 µmol) in THF (1.3 mL) was prepared in a 30 mL flask equipped with a star bar, and triethylamine (260 µL), rityl chloride (65 mg, 230 µmol), and dimethylaminopyridine (4.7 mg, 38 µmol) were added thereto at room temperature. After the reaction vessel was heated to 60°C in an oil bath, the reaction solution was stirred for 24 hours, and then the solution was cooled to room temperature, followed by adding saturated sodium bicarbonate solution (10 mL) thereto. The solution was extracted with dichloromethane, and the extract was dried over anhydrous sodium sulfate. The drying agent was filtered off, and the solvent was evaporated under reduced pressure, followed by drying the residue under high vacuum. Under an argon atmosphere, a solution of the residue in pyridine (1.2 mL) was prepared in a 30 mL flask equipped with a star bar, and anhydrous acetic acid (160 µL) was added thereto at 0°C. After the solution was stirred for 14 hours at room temperature, saturated sodium bicarbonate solution (10 mL) was added thereto. The solution was extracted with dichloromethane and the extract was dried over anhydrous sodium sulfate. The drying agent was filtered off, and the solvent was evaporated under reduced pressure, followed by drying the residue under high vacuum. Under an argon atmosphere, a solution of the residual in dichloromethane (1.2 mL) was prepared in a 30 mL flask equipped with a star bar, and a 5 M solution of hydrogen bromide in acetic acid (70 µL, 390 µmol) was added thereto at 0°C. After the mixture was stirred for 30 seconds at 0°C, the mixture was diluted with dichloromethane (10 mL) and distilled water (10 mL), and the diluted mixture was poured into saturated sodium bicarbonate solution (20 mL). After the mixture was extracted with dichloromethane, and the extract was washed with saturated saline solution, followed by drying the same over anhydrous sodium sulfate. The residue obtained by filtering off the drying agent and evaporating the solvent under reduced pressure was purified by silica gel chromatography (eluent: hexane/ethyl acetate = 70/30) to provide compound 27 (37.7 mg, 66%) as a colorless oily product.

### Compound 27:

¹H NMR (500 MHz, CDCl₃): δ 5.56 (dd, J = 2.4, 2.1 Hz, 1H), 5.28 (dd, J = 9.8, 2.8 Hz, 1H), 5.26 (dd, J = 9.8, 9.8 Hz, 1H), 4.83 (dd, J = 37.8, 10.4 Hz, 1H), 4.58 (dd, J = 8.5, 2.1 Hz, 1H), 3.74-3.67 (m, 2H), 3.62 (ddd, J = 12.8, 5.2, 4.9 Hz, 1H), 2.60-2.50 (m, 1H), 2.35 (dd, J = 8.5, 5.2 Hz, 1H), 2.15 (s, 3H), 2.07 (s, 3H), 2.01 (s, 3H), 1.43-1.24 (m, 52H), 0.87 (t, J = 7.0 Hz, 6H); ESI-MS (m/z): Anal. Calcd for C₄₃H₇₇FNNaO₈: 763.5500, Found : 763.5523 (M + Na)⁺.

### Examples 20 and 21

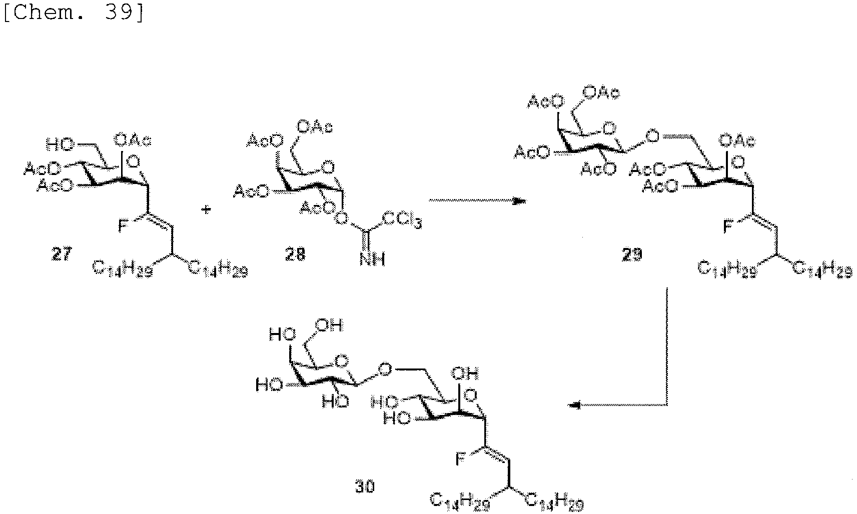

First, compound 28 was prepared according to the method described in J. Med. Chem. 2005, 48, 645-652.

Second, under an argon atmosphere, a suspension of compound 27 (15.0 mg, 20.2 µmol), compound 28 (12.0 mg, 24.3 µmol) and molecular sieve 4A (25 mg) in dichloromethane (670 µL) was prepared in a 10 mL flask equipped with a star bar, and was cooled to -20°C, followed by adding a solution of 35 µM trimethylsilyl trifluoromethanesulfonate in dichloromethane (170 µL, 6 µmol) thereto at -20°C. After the suspension was stirred for 13 hours at -20°C, triethylamine (0.2 mL) and saturated sodium bicarbonate solution (10 mL) were added thereto. The mixture was extracted with dichloromethane and the extract was dried over anhydrous sodium sulfate. The residue obtained by filtering off the drying agent and evaporating the solvent under reduced pressure was purified by silica gel chromatography (eluent: hexane/ethyl acetate = 70/30) to provide compound 29 (15.4 mg, 71%) as a colorless oily product.

A solution of compound 29 (8.0 mg, 7.5 µmol) in methanol (150 µL) was prepared in a 4 mL screw cap vial equipped with a star bar, and sodium methoxide (1.5 mg, 28 µmol) was added thereto at room temperature. After the reaction solution was stirred at room temperature for 1 hour, Amberlite IRC-120 was added to the solution, and the mixture was stirred for 1 minute, followed by filtering off the solid. The filtrate was concentrated and the residue was purified by silica gel (Iatrobeads (registered trademark) chromatography (eluent: chloroform/methanol = 4/1) to obtain 30 (3.4 mg, 59%) as a white solid.

### Example 20: Compound 29:

¹H NMR (500 MHz, CDCl₃): δ 5.53 (dd, J = 2.6, 2.6 Hz, 1H), 5.39 (dd, J = 3.4, 1.1 Hz, 1H), 5.25 (dd, J = 10.4, 7.9 Hz, 1H), 5.21 (dd, J = 9.2, 2.6 Hz, 1H), 5.18 (dd, J = 9.2, 9.2 Hz, 1H), 5.02 (dd, J = 10.4, 3.4 Hz, 1H), 4.81 (dd, J = 38.1, 9.5 Hz, 1H), 4.57 (dd, J = 6.7, 2.6 Hz, 1H), 4.54 (d, J = 7.9 Hz, 1H), 4.19 (dd, J = 11.3, 6.4 Hz, 1H), 4.11 (dd, J = 11.3, 7.2 Hz, 1H), 3.98 (dd, J = 11.3, 2.4 Hz, 1H), 3.91-3.88 (m, 2H), 3.57 (dd, J = 11.3, 6.3 Hz, 1H), 2.58-2.48 (m, 1H), 2.17 (s, 3H), 2.14 (s, 3H), 2.07 (s, 3H), 2.049 (s, 3H), 2.047 (s, 3H), 2.01 (s, 3H), 1.97 (s, 3H), 1.48-1.18 (m, 60H), 0.87 (t, J = 6.9 Hz, 6H); ESI-MS (m/z): Anal. Calcd for C₅₇H₉₅FNaO₁₇: 1093.6451, Found: 1093.6373 (M + Na)⁺.

### Example 21: Compound 30:

¹H NMR (500 MHz, CDCl₃/CD3OD = 4/1): δ 4.60 (dd, J = 38.5, 10.1 Hz, 1H), 4.50 (brd, J = 9.5 Hz, 1H), 4.23 (d, J = 7.6 Hz, 1H), 4.11-4.03 (m, 2H), 3.87-3.81 (m, 3H), 3.79 (dd, J = 11.6, 6.7 Hz, 1H), 3.70 (dd, J = 11.6, 5.0 Hz, 1H), 3.66 (dd, J = 9.5, 3.4 Hz, 1H), 3.60-3.55 (m, 1H), 3.55 (dd, J = 9.8, 7.9 Hz, 1H), 3.49-3.44 (m, 2H), 2.50-2.41 (m, 1H), 1.38-1.08 (m, 52H), 0.83 (t, J = 7.0 Hz, 6H); ESI-MS (m/z): Anal. Calcd for C₄₃H₈₁FNaO₁₀: 799.5711, Found : 799.5677 (M + Na)⁺.

### Examples 22 and 23

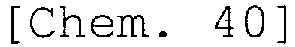

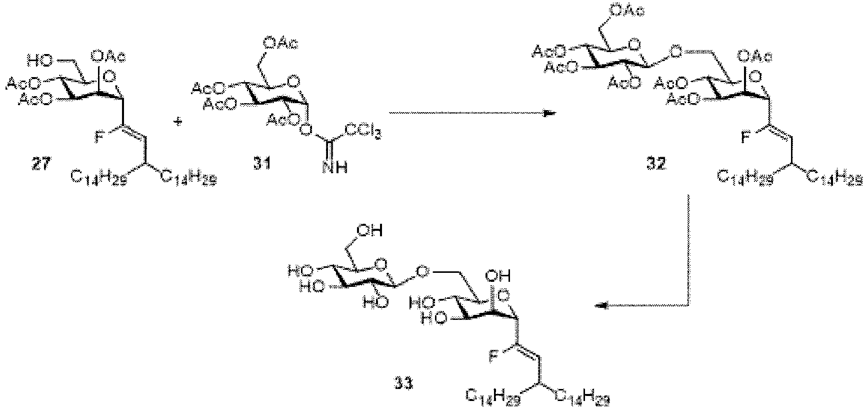

First, compound 31 was prepared according to the method described in J. Med. Chem. 2005, 48, 645-652.

Second, under an argon atmosphere, a suspension of compound 27 (13.8 mg, 18.6 µmol) prepared in Example 19, compound 31 (13.8 mg,27.9 µmol) and molecular sieve 4A (27 mg) in dichloromethane (600 µL) was prepared in a 10 mL flask equipped with a star bar, and was cooled to -20°C, followed by adding a solution of 35 µM trimethylsilyl trifluoromethanesulfonate in dichloromethane (150 µL, 5,2 µmol) thereto at -20°C. After the suspension was stirred for 13 hours at -20°C, triethylamine (0.2 mL) and saturated sodium bicarbonate solution (10 mL) were added thereto. The mixture was extracted with dichloromethane and the extract was dried over anhydrous sodium sulfate. The residue obtained by filtering off the drying agent and evaporating the solvent under reduced pressure was purified by a recycling gel filtration purification apparatus (HP-GPC) (eluent: chloroform), and the resulting mixture was further purified by silica gel chromatography (eluent: hexane/ethyl acetate = 60/40) to provide compound 32 (10.8 mg, 54%) as a colorless oily substance.

In a 4 mL screw cap vial equipped with a star bar, a solution of compound 32 (10.8 mg, 10.1 µmol) in a mixed solvent of dichloromethane (600 µL) and methanol (600 µL) was prepared, and sodium methoxide (2.2 mg, 40 µmol) was added thereto at room temperature. After the reaction solution was stirred at room temperature for 4 hours, Amberlite IRC-120 was added to the solution, and the mixture was stirred for 1 minute followed by filtering off the solid. The filtrate was concentrated, and the residue was purified by silica gel (Iatrobeads^{®}) chromatography (eluent: chloroform/methanol = 4/1) to obtain 33 (7.2 mg, 92%) as a white solid.

### Example 22: Compound 32:

¹H NMR (500 MHz, CDCl_{3/}CD3OD = 4/1): δ 5.52 (t, J = 2.7 Hz, 1H), 5.21 (dd, J = 9.8, 9.5 Hz, 1H), 5.22-5.18 (m, 1H), 5.18 (dd, J = 9.2, 9.2 Hz, 1H), 5.07 (dd, J = 10.1, 9.5 Hz, 1H), 5.04 (dd, J = 9.8, 7.9 Hz, 1H), 4.81 (dd, J = 37.8, 9.8 Hz, 1H), 4.57 (d, J = 7.9 Hz, 1H), 4.56 (brdd, J = 7.0, 2.7 Hz, 1H), 4.28 (dd, J = 12.5, 4.6 Hz, 1H), 4.13 (dd, J = 12.5, 2.4 Hz, 1H), 3.97 (dd, J = 11.3, 2.4 Hz, 1H), 3.90-3.85 (m, 1H), 3.69 (ddd, J = 10.1, 4.6, 2.4 Hz, 1H), 3.58 (dd, J = 11.3, 6.1 Hz, 1H), 2.57-2.48 (m, 1H), 2.16 (s, 3H), 2.09 (s, 3H), 2.06 (s, 3H), 2.05 (s, 3H), 2.02 (s, 3H), 2.01 (s, 3H), 2.00 (s, 3H), 1.43-1.25 (m, 52H), 0.88 (t, J = 7.0 Hz, 6H); ESI-MS (m/z): Anal. Calcd for C₅₇H₉₅FNaO₁₇: 1093.6451, Found: 1093.6382 (M + Na)⁺.

### Example 23: Compound 33:

¹H NMR (500 MHz, CDCl_{3/}CD3OD = 4/1): δ 4.61 (dd, J = 38.5, 10.4 Hz, 1H), 4.51 (d, J = 9.5 Hz, 1H), 4.28 (d, J = 8.2 Hz, 1H), 4.10 (dd, J = 2.4, 2.4 Hz, 1H), 4.06 (dd, J = 11.1, 2.4 Hz, 1H), 3.84-3.80 (m, 3H), 3.71-3.65 (m, 2H), 3.62-3.57 (m, 1H), 3.39 (dd, J = 8.9, 8.5 Hz, 1H), 3.34 (dd, J = 9.2, 8.9 Hz, 1H), 3.27 (dd, J = 8.5, 8.2 Hz, 1H), 3.28-3.23 (m, 1H), 2.51-2.42 (m, 1H), 1.40-1.08 (m, 52H), 0.84 (t, J = 6.9 Hz, 6H); ESI-MS (m/z): Anal. Calcd for C₄₃H₈₁FNaO₁₀: 799.5711, Found : 799.5693 (M + Na)⁺.

### Method:

### <Preparation of LNP>

The LNP of Moderna was prepared from a mixture of SM102 (50 mol%), GM-020 (1.5 mol%), cholesterol (38.5 mol%) and DSPC (10 mol%), and mRNA (0.695 µg) using a LNP preparation device, Nanospark. Hereinafter, this is referred to as Moderna-type LNP. Further, various C-glycoside glycolipid-LNPs were prepared from a mixture of SM102 (50 mol%), GM-020 (1.5 mol%), cholesterol (38.5 mol%), and various C-glycoside glycolipids (10 mol%) and mRNA (0.695 µg) using the LNP preparation device Nanospark. C-glycoside glycolipid-LNP means an LNP comprising C-glycoside glycolipid compound according to the present invention as a component.
The sources of each lipid are as follows:
- SM102: Medchemexpress (MCE), Product No.: HY-134541
- GM-020: SUNBRIGHT GM-020 (DMG-PEG2000)
- Cholesterol: Nippon Oil
- DSPC (1,2-distearoyl-sn-glycero-3-phosphocholine): Sigma (Avanti)
- RNA (GFP): Fujifilm (TriLink Bio Technologies, Inc.)
- mRNA (luciferase): Fujifilm (TriLink Bio Technologies, Inc.)

### <Cultured cells>

Cultured cells (mouse macrophage-like cells Raw 264.7 (American Type Culture Collection) were cultured in Dulbecco's Modified Eagle Medium (DMEM; Sigma Aldrich, St. Louis, MO) supplemented with 5% fetal bovine serum (FBS; Moregate Biotech, Bulimba, Australia) and 0.5% penicillin/streptomycin (Invitrogen, Grand Island, NY) at 37°C in 5% CO₂/95% atmospheric conditions.

### <Quantitative real-time RT-PCR method>

After the DMEM medium (Sigma Aldrich) was removed from the cultured cells, they were washed with PBS and total RNA was extracted. The extraction of RNA was carried out using RNAiso (Takara Bio Co., Ltd., Shiga, Japan), and reverse transcription from total RNA to cDNA was carried out using ReverTra Ace qPCR RT kit (Toyobo, Osaka, Japan). The quantitative real-time PCR reaction was carried out using THUNDERBIRDTM SYBR qPCR Mix (Toyobo) and the 7500 Real-time PCR system (Applied Biosystems, Foster City, CA), and was analyzed by the calibration curve method. 18S was used as an internal standard. Table 1 shows the primer sequences used for measuring each target gene.

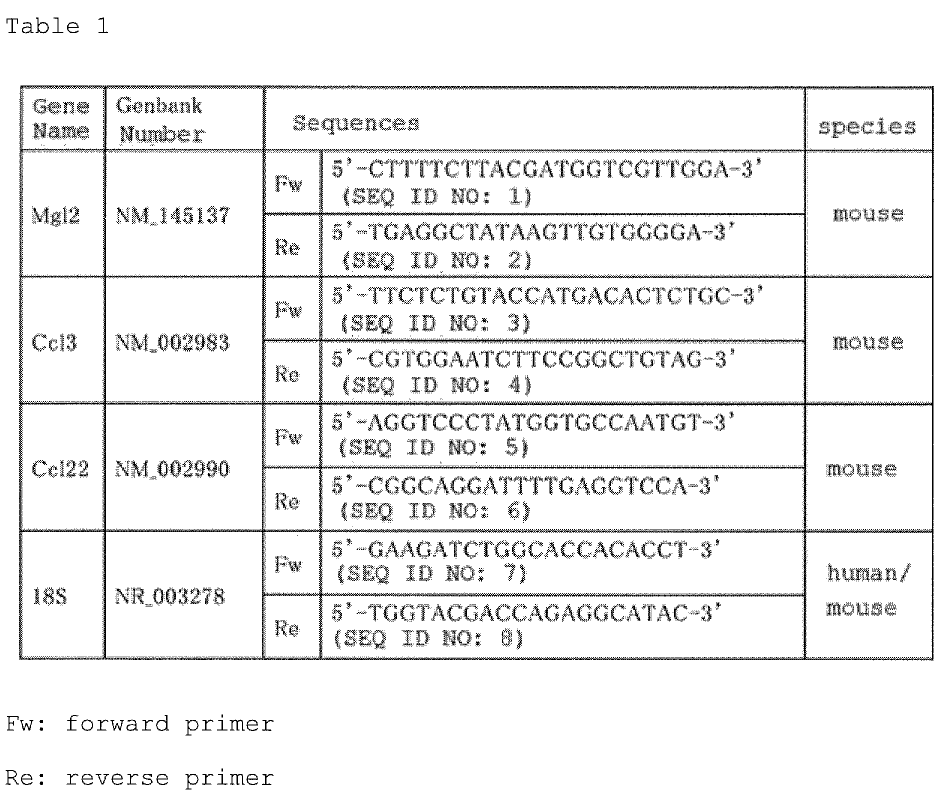

### <Measurement of cell proliferation activity>

Raw 264.7 cells were seeded onto a 96-well black plate (Cat No. 655086, Greiner), and at 24 hours after cell adhesion, various LNPs at a volume of 10 µL were added to the plates. After 24 hours, ATP activity was then measured. For ATP assay, Cell Titer-GloTM Luminescent Cell Viability Assay (Promega) was used, and chemiluminescence intensity was measured by EnSpire Multimode plate Reader (Perkin Elmer) .

### <Measurement of mRNA intracellular uptake by LNP>

Raw 264.7 cells were seeded onto a 96-well black plate (Cat No. 655086, Greiner), and at 24 hours after cell adhesion, various LNPs at a volume of 10 µL were added to the plates. Luciferase activity was measured 8-24 hours after the addition of LNPs. After removing the media, the cells exposed to the LNPs were added with 40 µl of Passive Lysis Buffer (Promega), and the mixture was shaken for 10 minutes to lyse the cells. Then, 10 µl of luciferin was added thereto, and the mixture was shaked for 1 minute, followed by measuring the chemiluminescence intensity using EnSpire Multimode Plate Reader (Perkin Elmer).

### <Measurement of the amount of intracellular uptake by LNP in animals>

Various LNPs (10 µl) were administered to the calf muscle of the ICR mouse. At 24 hours after the administration, Luciferin at a dose of 100 mg/kg was subcutaneously administered, and 5 minutes later, chemiluminescence intensity was measured using an in vivo imaging device (FUSION FX7.EDGE) under isoflurane anesthesia. The quantitative measurement of the luminous flux was performed using the FUSION application software.

### <Evaluation of LNP decomposition>

Five µl of each LNP was electrophoresed on the 2% agarose (WAKO) prepared, to confirm decompositions at each time point (0, 3, 7 days).

### <Mgl1 and 2 genes knockdown>

To knockdown the Mgl1 gene, the Invitrogen miRNA expression vector pcDNA6.2 introduced with the sequence shown in Table 2 below was added to the cells at a concentration of 10 µg, and introduced into the cells using a gene introduction device NEPA21 electroporation. After 24 hours, G418 was exposed on the cells at 4000 ng/ml, and at 72 hours, the DMEM medium was changed so as to prepare the cells with stable knockdown of Mgl1. The expression level of the MGL1 protein was confirmed by Western blotting using the MGL1 antibody (R&D: AF4938).

The Mgl2 gene was knock downed using the Invitrogen siRNA. The Mgl2 siRNA and the control siRNA (Silencer (registered trademark) Select Negative Control) at a concentration of 28.5 pmol were added to Raw 264.7 cells (1X10⁶ cells/100 µl), and introduced into to the same using a gene introduction device NEPA21 electroporation. The knockdown effect of the Mgl2 gene was confirmed by measuring the expression level of Mgl2 mRNA at 48 hours after the cells were collected. Table 2 shows the siRNA sequences for each target gene.

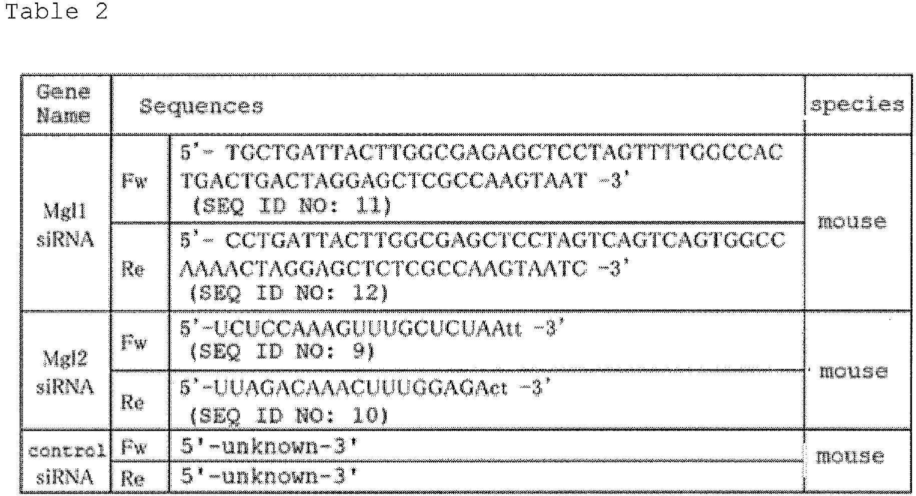

### <Interaction between lectin proteins and LNPs>

The various lectin protein-biotin compounds (Con A, DBA, PNA, SBA, UEA-1, and WGA (Vector Laboratories)) were adjusted to 1 mg/ml in PBS, and they were reacted with 20 µl of LNPs at 4°C for 16 hours, followed by immunoprecipitating them with avidin magnetic beads (50 µl). Subsequently, the mRNAs were collected from the LNPs bound to the avidin magnetic beads using ReliaPrep (registered trademark) RNA Miniprep Systems (Promega), and the mRNAs were converted to cDNAs using the ReverTra Ace (registered trademark) qPCR RT Kit (TOYOBO). Then, the luciferase mRNA levels were detected by real-time PCR using THUNDERBIRD (registered trademark) qPCR Mix and the following primers.

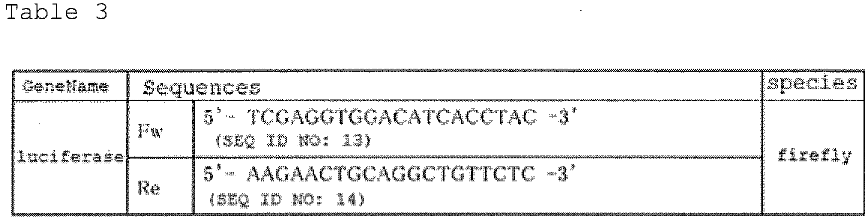

### <Effect on Preservation at -80 °C>

Various LNPs were suspended in either PBS or 20% sucrose PBS solution, preserved at -80°C or 4°C, and the fluorescence intensity of GFP proteins generated by GFP mRNA (TriLink CleanCap (registered trademark) mRNA) were measured using a flow cytometer CytoFLEX (BECKMANN) after the freeze-thawing, thereby verifying their mRNA cellular uptake activities.

### <Statistical Analysis>

One-way ANOVA and Tukey-Kramer post-hoc test were used for comparison between independent other groups. In addition, for comparison between two independent groups, the unpaired t-test was used. Significant difference was defined as 5% or lee significance level.

### Test Example 1

### Functional evaluation of LNP for mRNA intracellular delivery as indicated by luciferase activity

The function of LNPs for mRNA intracellular delivery was evaluated as indicated by luciferase activity.

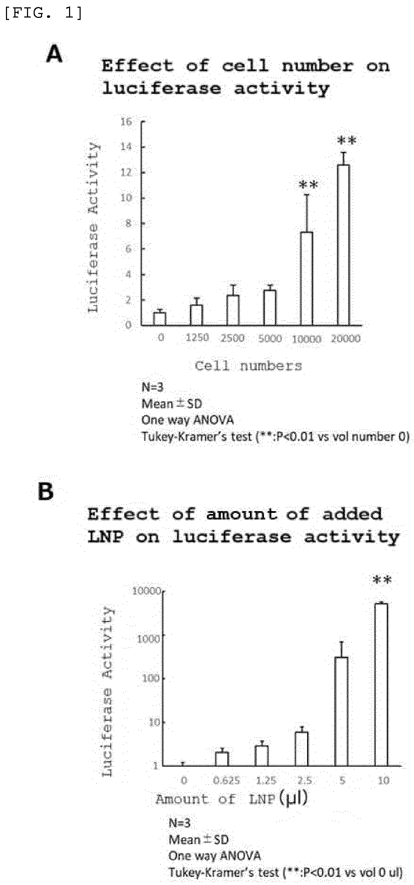

The Moderna-type LNP was used as the basic model LNP to determine the influence of LNP addition amount and cell number on mRNA intracellular delivery. Raw 264.7 cells were seeded onto a 96-well plate at each cell number (50 µl), and 10 µl of the Moderna-type LNP was added thereto. After 16 hours, luciferase activity was measured to investigate the effect of cell number on mRNA uptake in LNPs. The result is shown in Figure 1A. Figure 1A shows that mRNA intracellular delivery increases in a cell number-dependent manner, with 20,000 cells (50µl) resulting in the highest mRNA uptake.

In addition, Raw 264.7 cells were seeded onto a 96-well plate at a density of 20,000 cells (50 µl), and the LNP was added to the cells in a dose-dependent manner, followed by measuring Luciferase activity, 16 hours later. The result is shown in Figure 1B. Figure 1B shows that mRNA is taken up in a dose-dependent manner, with 10 µl resulting in the maximum uptake.

Based on the above investigations, the subsequent studies were determined to be conducted by seeding Raw 264.7 cells at 20,000 cells (50µl) using a 96-well plate, and measuring the luciferase activity 16 hours after 10 µl of LNP exposure was added.

### Test Example 2

### Functional evaluation of various C-glycoside glycolipid-LNPs on mRNA intracellular delivery as indicated by luciferase activity

The function of various C-glycoside glycolipid-LNPs for mRNA intracellular delivery was evaluated as indicated by luciferase activity.

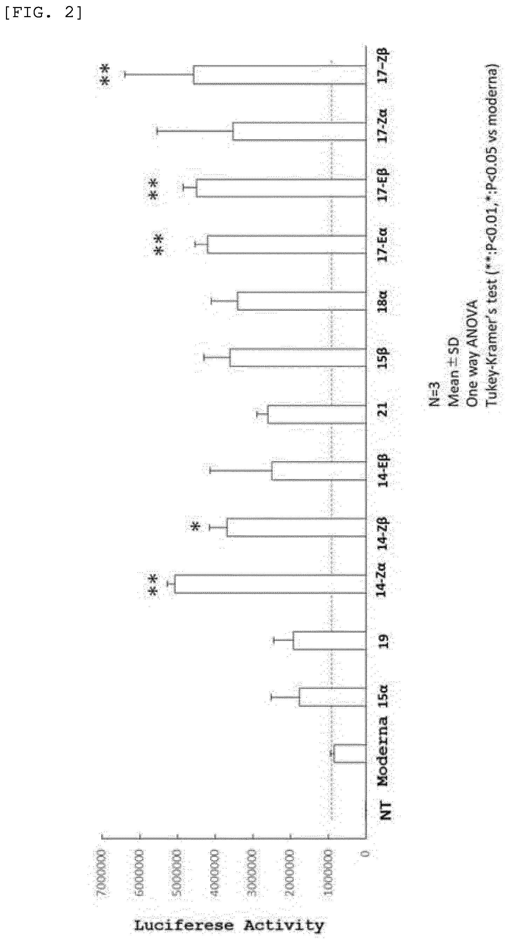

The Moderna-type LNP was used as the basic model LNP to measure the mRNA uptake amount as indicated by luciferase activity. The Raw 264.7 cells were seeded onto a 96-well plate at each cell number (50 µl), and various LNPs (10 µl) were added thereto. The luciferase activity was measured after 16 hours. The results are shown in Figure 2. Figure 2 shows that the C-glycoside glycolipid LNPs (compounds 14-Zα, 14-Zβ, 17-Eα, 17-Eβ, 17-Zα, and 17-Zβ) significantly increased the luciferase activities in the cells treated with the LNPs in comparison with the Moderna-type LNP.

The above investigations reveal that the C-glycoside glycolipid-LNPs (compound 14-Zα, compound 14-Zβ, compound 17-Eα, compound 17-Eβ, compound 17-Zα, and compound 17-Zβ) increase the amount of mRNA uptake or translation ability into cells in comparison with the Moderna-type LNP.

### Test Example 3

### Effects of various C-glycoside glycolipid-LNPs on live cells as indicated by ATP activity

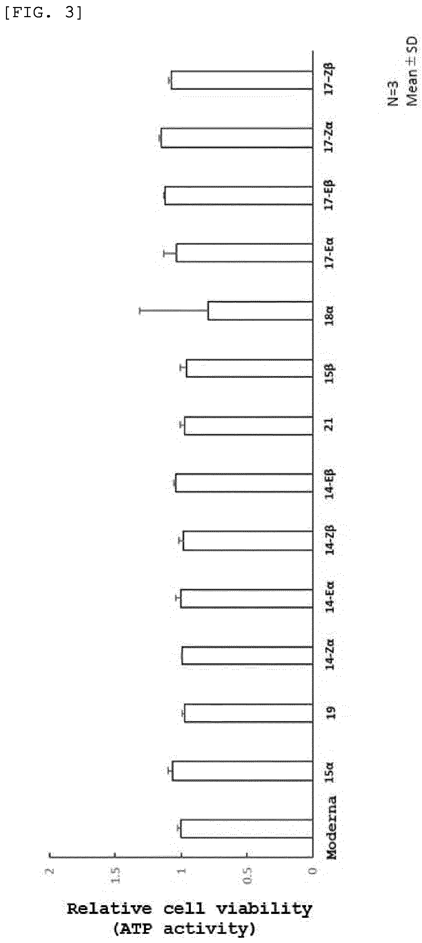

The effects of various LNPs on living cells were evaluated as indicated by ATP activity. The Moderna-type LNP was used as the basic model LNP to determine the effects of various C-glycoside glycolipid-LNPs. The Raw 264.7 cells were seeded onto a 96-well plate at each cell number (50 µl), and various LNPs (10 µl) were added thereto. After 24 hours, ATP activity was measured. The results are shown in Figure 3. Figure 3 shows that none of all C-glycoside glycolipid-LNPs is significantly different in comparison with the Moderna-type LNP.

The above investigations reveal that the various C-glycoside glycolipid-LNPs do not affect the number of viable cells, similarly to the Moderna-type LNP.

### Test Example 4

### Evaluation of the stability at room temperature in various C-glycoside glycolipid-LNPs

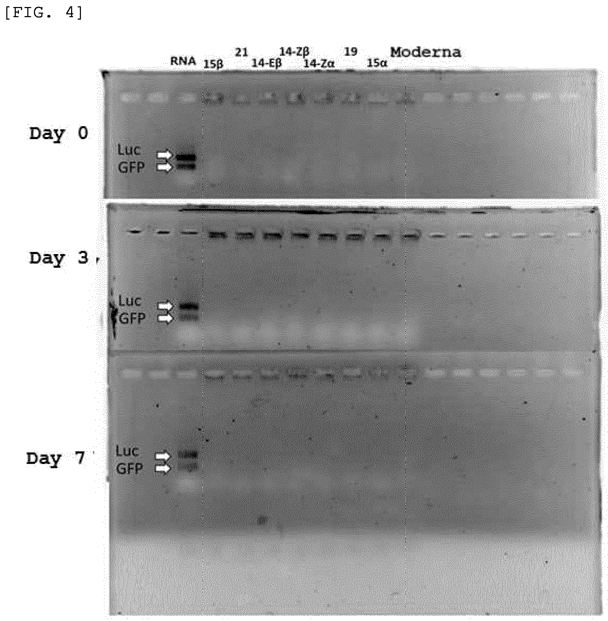

Various C-glycoside glycolipid-LNPs were left at room temperature to evaluate the stability of the LNPs. Luc (Luciferase mRNA) and GFP (green fluorescent protein mRNA) as decomposition products were simultaneously electrophoresed. Five µl of the various C-glycoside glycolipid-LNPs were injected into 2% agarose, which was then subjected to electrophoresis. The results are shown in Figure 4. Figure 4 shows that the two RNAs, which were the decomposition products of LNP, were not detected up to seven days.

The above results reveal that various C-glycoside glycolipid-LNPs are stable for seven days at room temperature, similarly to the Moderna type.

### Test Example 5

### Effects of various C-glycoside glycolipid-LNPs on expression of the genes related to adjuvant activity

The effects of various LNPs on the expression of the genes related to adjuvant activity that is important for antibody production after the LNP vaccine administration were evaluated.

The Moderna-type LNP was used as the basic model LNP to measure the effects of various LNPs. Raw 264.7 cells were seeded onto a 96-well plate at each cell number (50 µl), and various C-glycoside glycolipids-LNP (10 µl) were added thereto. After 16 hours, the cells were collected, RNA was extracted, and the expression levels of various genes were measured by real-time PCR.

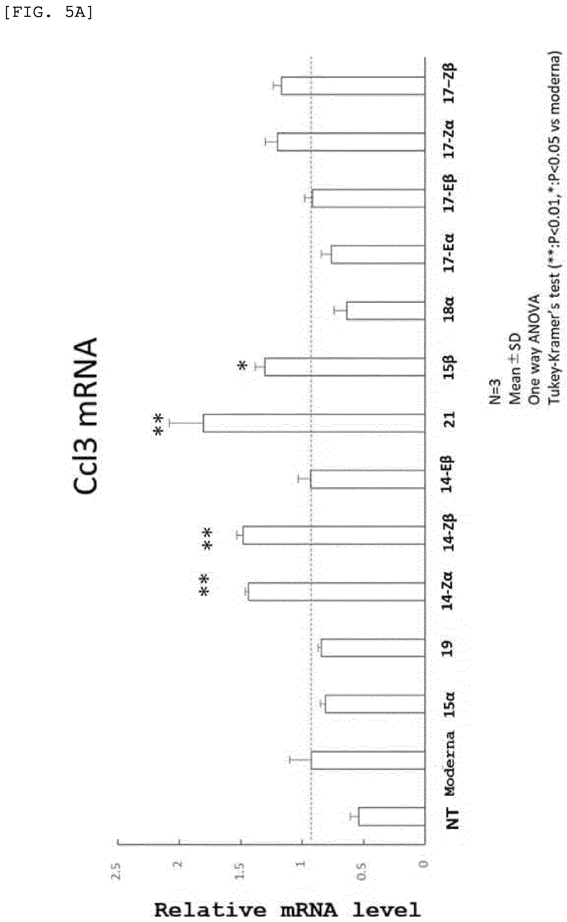

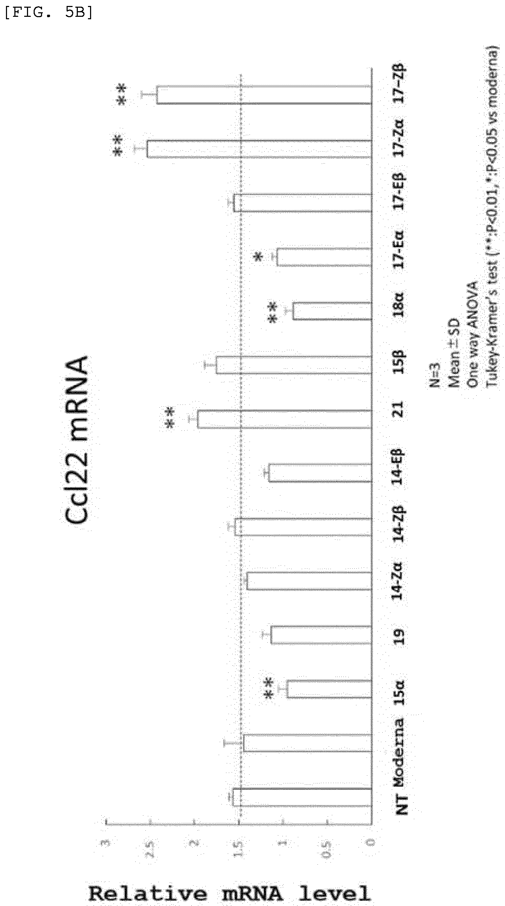

First, the expression level of the cytokine Ccl3, which promotes the migration of antigen-presenting cells in relation to adjuvant activity, was measured. The result is shown in Figure 5A. Figure 5A shows that the C-glycoside glycolipid-LNPs (compounds 14-Zα, 14-Zβ, 21, and 15β) significantly increased Ccl3 expressions in the cells treated with the LNPs in comparison with the Moderna-type LNPs. In addition, the expression level of the cytokine Ccl22, which suppresses adjuvant activity, was measured. The results are shown in Figure 5B. Figure 5B shows that the C-glycoside glycolipid LNPs (compounds 15α, 18α, and 17-Eα) significantly decreased Ccl22 expressions in the cells treated with the LNPs in comparison with the Moderna-type LNP, and that the C-glycoside glycolipid LNPs (compounds 21, 17-Zα, and 17-Zβ) significantly increased Ccl22 expressions in cells treated with the LNPs.

The above results suggested that various C-glycoside glycolipid LNPs have the adjuvant activity, and that 14-Zα and 17-Eα are useful due to their relationship between the mRNA intracellular uptake and the adjuvant activity.

### Test Example 6

### Effects of Galactose-type C-type lectin 2 (Macrophage galactose-type lectin 2: MGL2/CD301b) on intracellular uptake of various C-glycoside glycolipid-LNPs

In comparison with the Moderna-type LNP, compounds 15α and 14-Zα exhibit superior mRNA delivery into cells. Generally, LNPs are taken up into cells via the LDL receptor by binding the cholesterol of their constituent components to APO proteins. In the present invention, various C-glycoside glycolipid-LNPs were prepared by adding C-glycoside glycolipids instead of DSPC. Further, the structures of the C-glycoside glycolipids contain the bonded galactose. Therefore, it was hypothesized that lectin receptors that take up galactose should be important in intracellular uptake, and the uptake mechanism of various C-glycoside glycolipid-LNPs was verified.

First, upon investigation of the Raw 264.7 cells, focusing on the fact that the cells were macrophage-like cells, it was revealed that galactose-type C-type lectin 2 (macrophage galactose-type lectin 2: MGL2/CD301b) was expressed therein (American Society for Microbiology Journal of Virology Volume 88, Issue 3, 1 February 2014, Pages 1659-1672: https://doi.org/10.1128/JVI.)02014-13). Therefore, cells wherein Mgl2 was knocked down were prepared, and the amounts of various LNP intracellular uptakes were evaluated by luciferase activity.

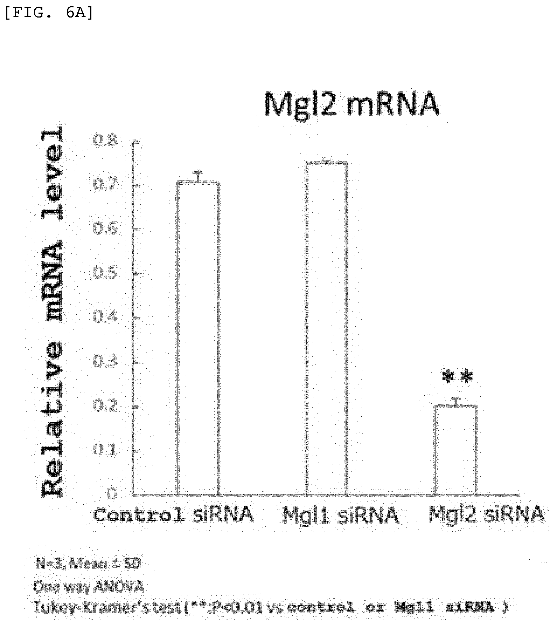

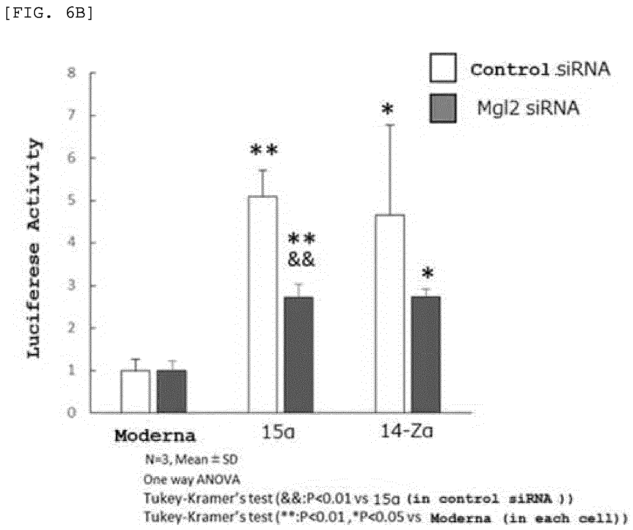

The results are shown in Figure 6. Figure 6A is a graph showing the results of knocking down the Mgl2 gene by transfecting Mgl2 siRNA listed in Table 2 into cells. Figure 6B shows that, in Mgl2 knockdown cells, there was no significant difference in the uptake of the Moderna-type LNP, whereas there were the significant decreases in the intracellular uptake of compound 15α and compound 14-Zα LNPs. The above results suggest that Mgl2 could play an important role in the intracellular uptake mechanism of C-glycoside glycolipid LNPs.

### Test Example 7

### Functional analysis of LNP using animals

The previous investigations made it clear that the C-glycoside glycolipid LNPs can more efficiently deliver genes to cultured cells in comparison with the Moderna-type LNP. Therefore, compound 14-Zα-LNP was administered to animals to measure the gene expression levels.

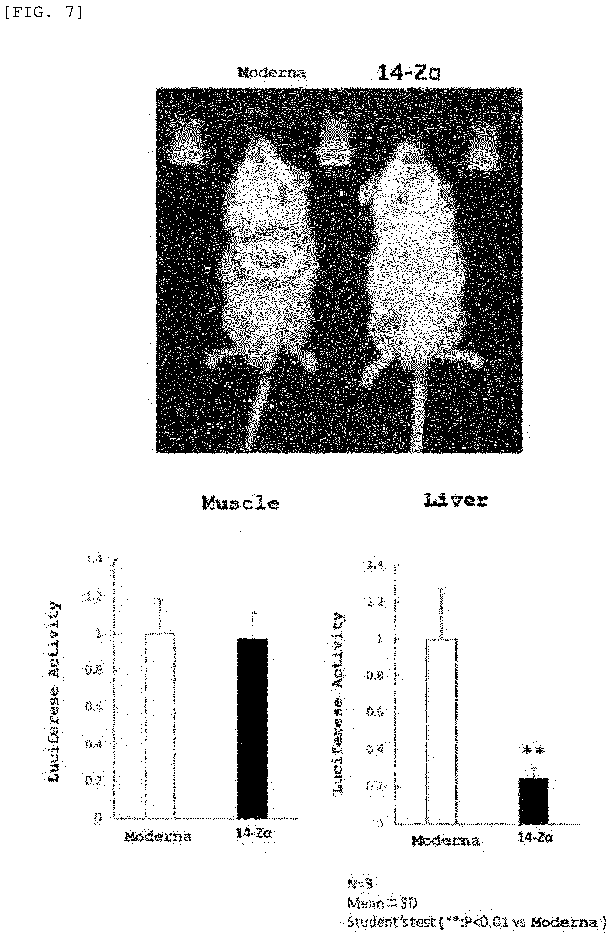

The LNP (10 µl) was administered to the calf muscle of the animal and the luciferase activity after 24 hours was analyzed using an in vivo imaging device. The results are shown in Figure 7. Figure 7 shows that compound 14-Zα-LNP exerted the stronger gene expression in the muscle in comparison with the Moderna-type LNP, whereas the Moderna-type LNP delivered the LNP not only to the muscles but also to the liver.

The above results reveal that compound 14-Zα delivers genes to cells at the site of intramuscular administration.

### Test Example 8

### Functional analysis of compound 30 using animals

The previous investigations revealed that compound 14-Zα-LNP could more efficiently deliver and express the genes in the cultured cells and the mouse tissues in comparison with the Moderna-type LNP. However, the Moderna-type LNP and compound 14-Zα-LNP exerted no significant difference in the gene expression in the calf muscle of animals. Thus, LNP (10 µl) containing compound 30 that enables stable gene expression in the cultured cells to the muscle, and after 24 hours, luciferase activity was analyzed using an in vivo imaging device.

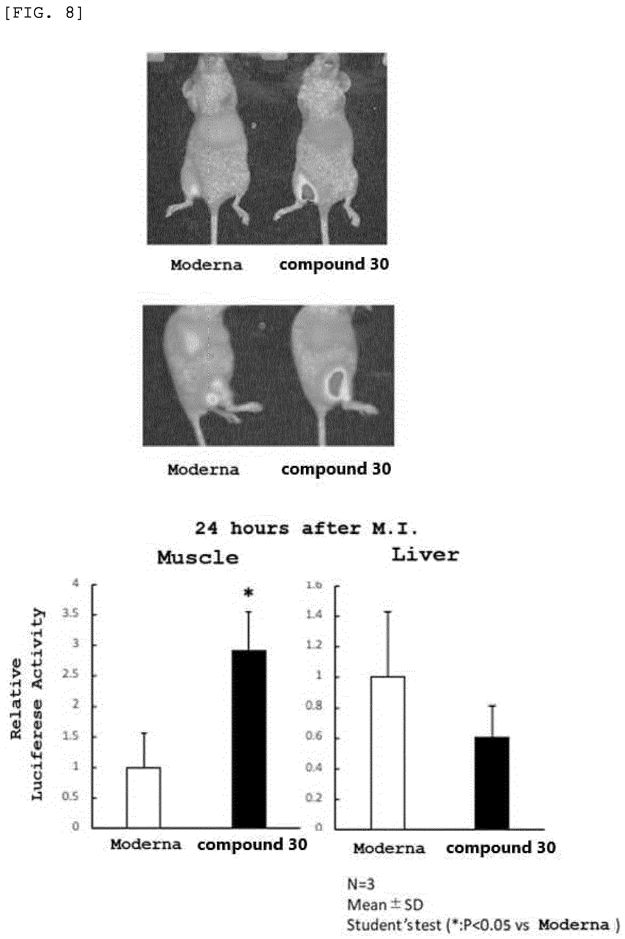

The results are shown in Figure 8. Figure 8 shows that the KGK020-LNP exerted significantly higher gene expression levels in the intramuscular administration group in comparison with the Moderna-type LNP, whereas the Moderna-type LNP exerted higher gene expression levels in the liver in comparison with compound 30-LNP.

### Test Example 9

### Functional analysis of compound 33 using animals

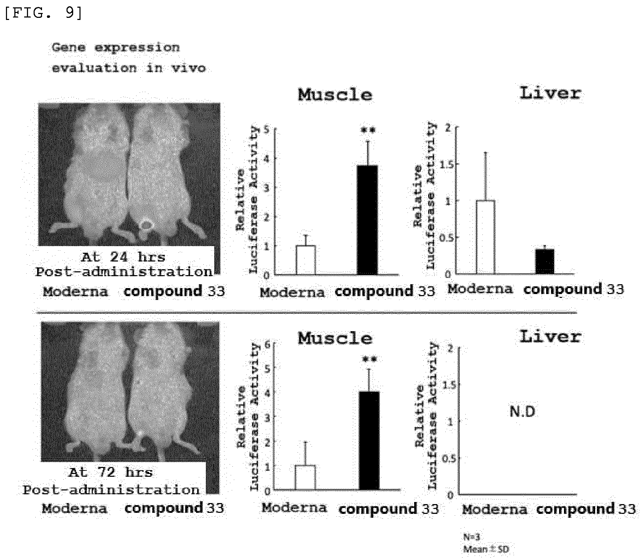

LNP containing compound 33, in which the disaccharide was bound similarly to compound 30, was administered to the muscle at a dose of 10 µl, and, after 24 and 72 hours, the luciferase activities were analyzed by an in vivo imaging device. The results are shown in Figure 9. Figure 9 shows that the KGK020-LNP exerted significantly higher gene expression in the intramuscular injection group in comparison with the Moderna-type LNP in both 24 and 72 hours, whereas the Moderna-type LNP at 24 hours after administration exerted higher gene expression in the liver in comparison with the compound 33-LNP, while observing no detection at 72 hours.

### Test Example 10

### Effect of Galactose-type C-type lectin 1 (Macrophage galactose-type lectin 1: MGL1/CD301a) on intracellular uptake of compound 14-Zα-LNP

In comparison with the Moderna-type LNP, compound 15α and compound 14-Zα exhibit superior mRNA delivery via MGL2. However, the effect was partial, and so MGL1, a subtype of MGL, was also examined for the effect. Specifically, cells wherein MGL1 was knocked down was prepared, and the amounts of various LNP intracellular uptakes were evaluated by luciferase activity.

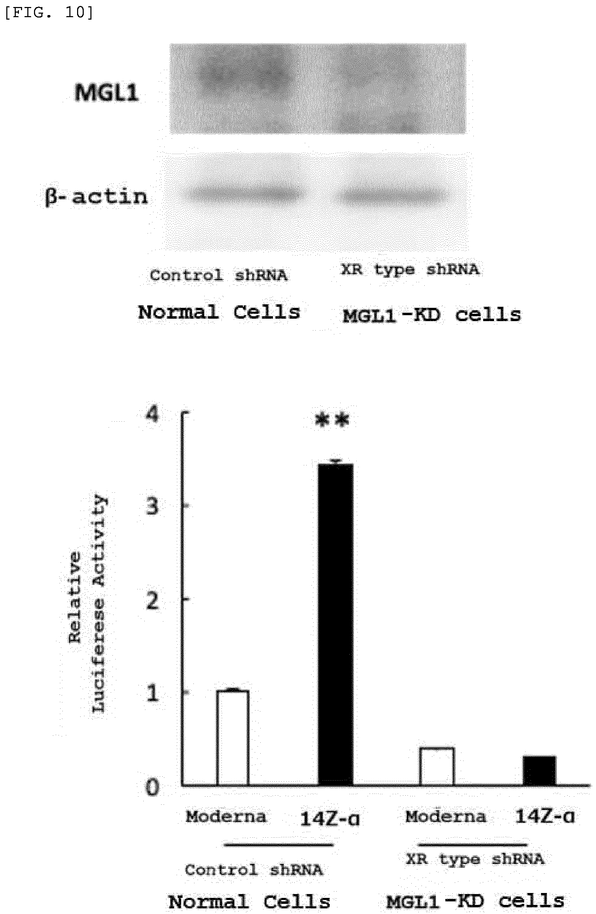

The results are shown in Figure 10. Figure 10 shows that the compound 14-Zα-LNP significantly decreased the amounts of the intracellular uptake in the MGL1 knockdown cells.

The above results suggest that MGL1 plays a more important role in the cellular uptake mechanism of C-glycoside glycolipid-LNP in comparison with MGL2.

### Test Example 11

### Effect of cell type on intracellular uptake of compound 14-Zα and compound 30-LNP

In comparison with the Moderna-type LNP, compound 14-Zα-LNP and compound 30-LNP exhibit superior mRNA deliver into cells via MGL1 and MGL2, which are highly expressed on the cell surface of immune cells. Thus, fibroblast cells (NIH3T3) and immune cells (Raw 264.7 cells) at a dose of 50 µl were seeded, and various LNPs (10 µl) were added thereto. After 16 hours, the luciferase activity was evaluated.

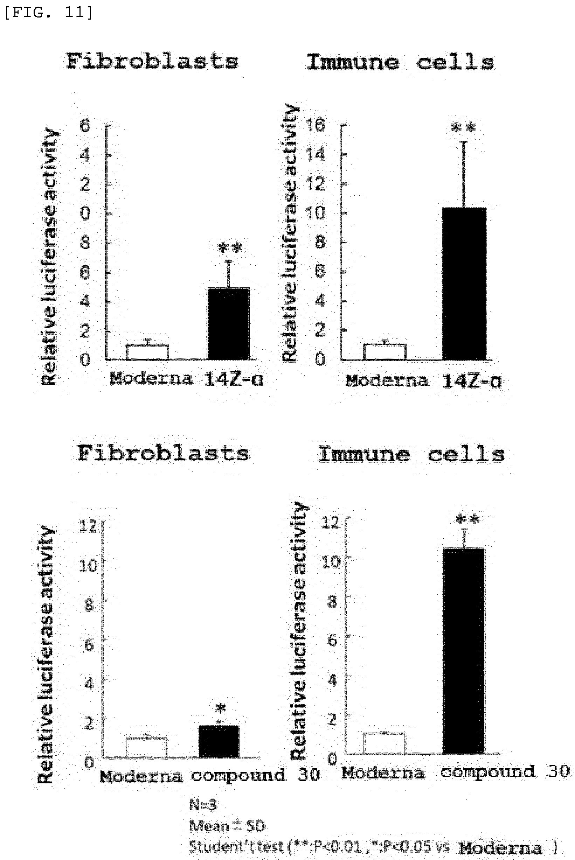

The results are shown in Figure 11. Figure 11 shows that the compound 14-Zα-LNP and the compound 30-LNP increased the luciferase activity in the immune cells in comparison with the Moderna type, whereas the increase rate in fibroblasts was lower than that in the immune cells.

### Test Example 12

### Intracellular mRNA uptake of compound 30-LNP in human immune cells

In comparison with the Moderna-type LNP, the C-glycoside glycolipid-LNPs according to the present invention more efficiently delivers mRNA into the cells in mouse immune cells. Thus, the levels of mRNA uptake by the compound 30-LNP were evaluated in human immune cells (human leukemia cells THP1).

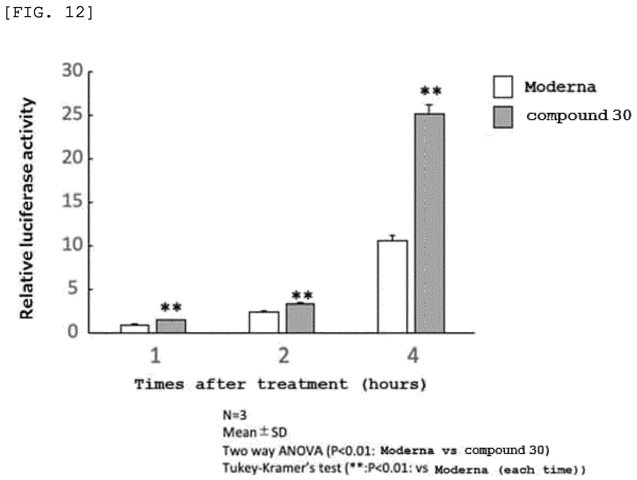

The results are shown in Figure 12. Figure 12 shows that, even in THP1 cells, the compound 30-LNP significantly increased the mRNA uptake over time in comparison with the Moderna-type LNP.

### Test Example 13

### Functional evaluation of compound 14-Zα, compound 30, and compound 33-LNP for mRNA intracellular delivery

The functions in delivery of various mRNA LNPs into cells were evaluated as indicated by luciferase activity. The Moderna-type LNP was used as the basic model LNP, the mRNA uptake levels of various LNPs were measured as indicated by luciferase activity. Raw 264.7 cells were seeded onto a 96-well plate at each cell number (50 µl), and various LNPs (10 µl) were added thereto. After 8 hours, the luciferase activity was measured.

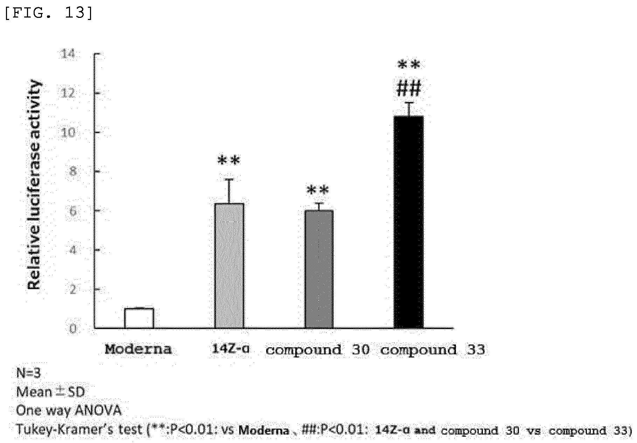

The results are shown in Figure 13. Figure 13 shows that, in comparison with the Moderna-type LNP, compound 30-LNP and compound 33-LNP, in which a disaccharide is attached like compound 14-Zα-LNP in which a monosaccharide is attached, more significantly increased the luciferase activity in the cells treated with the LNPs, and that the cells exposed with the compounds 33-LNP exerted the highest gene expression.

### Test Example 14

### Functional evaluation of compound 30-LNP for mRNA intracellular delivery as indicated by GFP protein

GFP mRNA was used instead of luciferase to evaluate the amount of intracellular uptake. Raw 264.7 cells were seeded onto a 24-well plate at each cell number (280 µl), and Moderna-type LNP and compound 30-LNP (20 µl) were added thereto. After 16 hours, GFP fluorescence activity was measured.

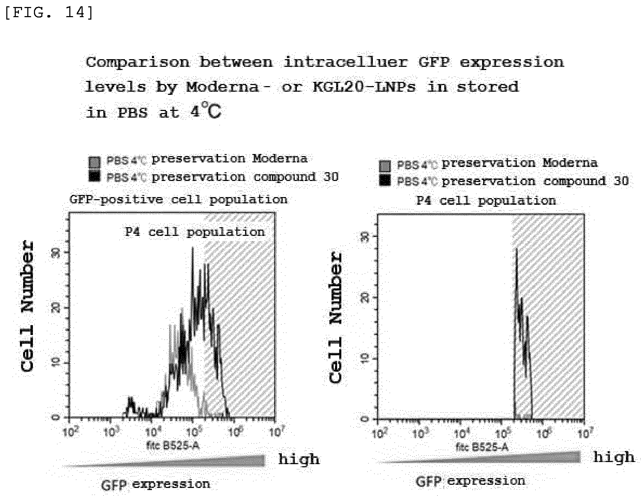

The results are shown in Figure 14. Figure 14 shows that the compound 30-LNP-LNP significantly increased the fluorescence intensity in the cells treated with the LNP in comparison with the Moderna-type LNP.

The above investigations reveal that the C-glycoside glycolipid-LNP020 according to the present invention increases the amount of intracellular uptake of a mRNA other than luciferase or the translation ability of the same, in comparison with the Moderna type.

### Test Example 15

### Effects of various C-glycoside glycolipid-LNPs on 4°C PBS preservation as indicated by GFP protein

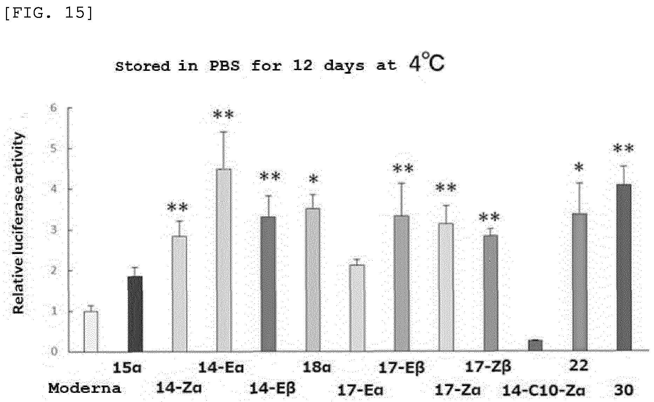

While GFP mRNA was used to evaluate the amount of intracellular uptake, the effects of various C-glycoside glycolipids-LNPs on preservation at 4°C in PBS were evaluated. The results are shown in Figure 15. Figure 15 suggests that various C-glycoside glycolipid-LNPs enable the longer-term preservation for 12 days or more in comparison with Moderna-type LNPs.

### Test Example 16

### Effect of compound 30-LNP on cryopreservation as indicated by GFP protein

Test Example 15 indicates that various C-glycoside glycolipid-LNPs enable the long-term preservation for 12 days or more during the storage at 4°C in PBS preservation. In order to investigate a more long-term and stable storage method, LNPs as used in this example were those which were suspended in a 20% sucrose solution, the suspension was frozen at -80°C, and then thawed to give the thawed LNP. Raw 264.7 cells were seeded onto a 24-well plate at each cell number (280 µl), and the Moderna-type LNP and compound 30-LNP (20 µl) were added thereto. After 16 hours, the GFP fluorescence activity was measured.

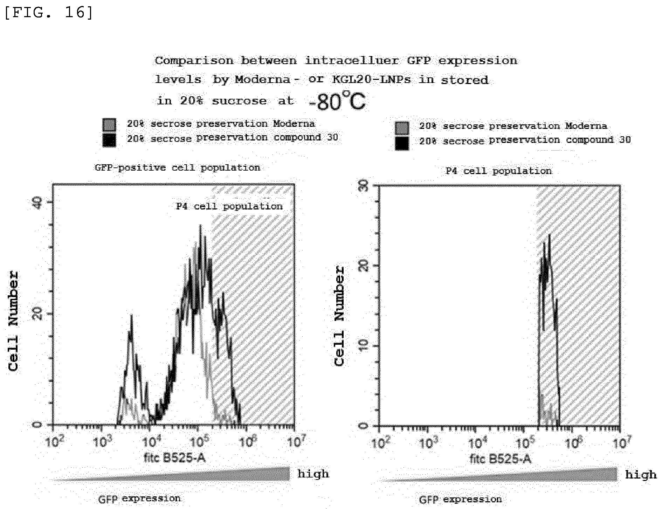

The results are shown in Figure 16. Figure 16 shows that the compound 30-LNP significantly increased the fluorescence activity in the cells treated with the LNPs in comparison with the Moderna-type LNP.

The above investigations reveal that the compound 30-LNP, which is suspended in a 20% sucrose solution, can be stored at -80°C, and its ability to deliver mRNA into cells is maintained.

### Test Example 17

### Evaluation of Moderna-type and compound 33-LNPs for antibody titers using receptor-binding domain (RBD) as an index

Covid-19 spike protein mRNA was encapsulated in compound 33-LNP, and the LNP (10 µl) was intramuscularly administered to mouse. After 5 weeks, the potency of antibodies against the spike protein was measured.

Specifically, Covid19 spike protein mRNA (Omicron type; OZ Biosciences) was encapsulated in compound 33-LNP and the LNP (10 µl) was administered to the thigh muscle of mouse. Blood was collected from the tail of the mouse, and the serum was separated to give an ample to be used for measurement. After immobilizing the spike protein (Omicron strain) or receptor-binding domain (RBD; Micron strain) at a concentration of 30 ng/µl on a 96-well plate and reacting it with the mouse serum, HRP-labeled mouse antibodies were reacted with the same. Finally, the chromogenic substrate, 2,2'-azino-bis(3-ethylbenzothiazoline-6-sulfonic acid) was reacted to measure the absorbance.

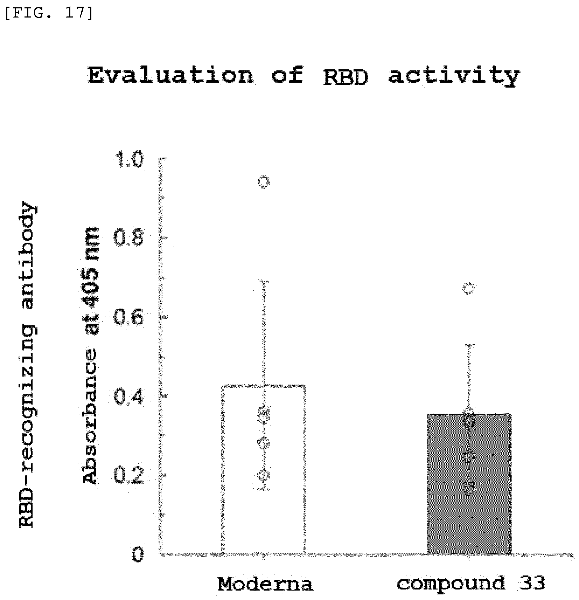

The results are shown in Figure 17. Figure 17 shows that, as the result of the evaluation by the antibody titer using RBD protein as an index, there was no significant difference in the RBD-targeting antibody titer by the compound 33-LNP in comparison with that by the Moderna-type LNP.

### Test Example 18

### Evaluation of Moderna-type and compound 33-LNPs for antibody production capacity

In accordance with the procedure of Test Example 17, Covid19 spike protein mRNA was encapsulated in the Moderna-type and Compound 33-LNPs, and the amounts of antibodies against the spike protein were measured in mouse muscle after intramuscular administration (10 µl) after the first and third weeks of the administration.

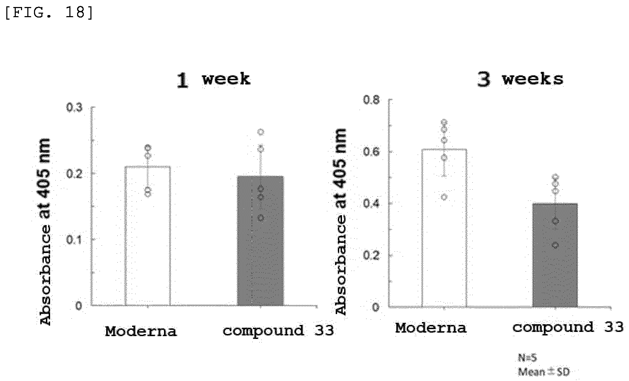

The results are shown in Figure 18. Figure 18 shows that the amount of antibody production by the compound 33-LNP decreased slightly in the third week in comparison with that by the Moderna-type LNP.

### Test Example 19

### Evaluation of Moderna-type and compound 33-LNPs for antibody titers using a full-length protein of spike protein as an index

In accordance with the procedure of Test Example 17, Covid19 spike protein mRNA was encapsulated in the Moderna-type and Compound 33-LNPs, and the amounts of antibodies against the spike protein were measured in mouse muscle after intramuscular administration (10 µl) after the fifth week of the administration.

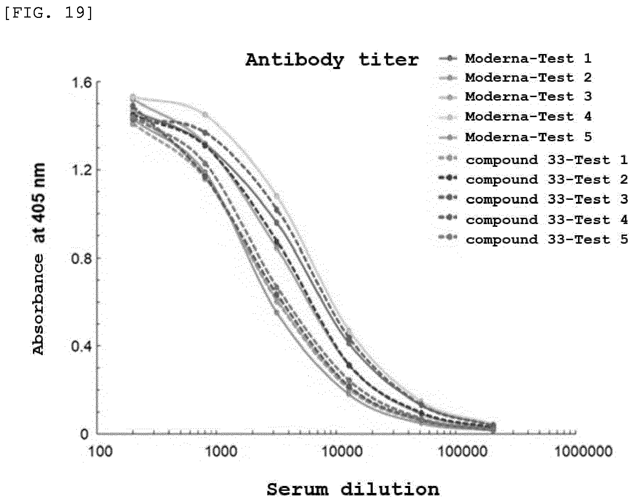

The results are shown in Figure 19. Figure 19 shows that, as the result of the evaluation by the antibody titer using the full-length protein of spike protein as an index, there was no significant difference in the antibody titer by the compound 33-LNP in comparison with that by the Moderna-type LNP.

The present invention can encompass the following embodiments.

### <C-glycoside glycolipid compounds>

[1] A C-glycoside glycolipid compound represented by formula (I): 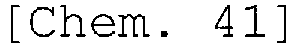 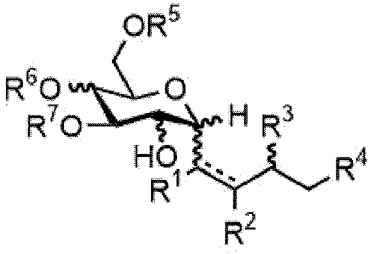 in which
   R1 is hydrogen, a halogen, or a hydroxy;
   R2 is a hydrogen atom, a hydroxy group that may be substituted, or a straight or branched, saturated or unsaturated C11-C15 alkyl group that may be substituted, wherein the alkyl group may be optionally interrupted by one or more ester bonds, amide bonds, and/or ether bonds;
   R3 is a hydrogen atom, a straight or branched, saturated or unsaturated C11-C15 alkyl group that may be substituted, wherein the alkyl group may be optionally interrupted by one or more ester bonds, amide bonds, and/or ether bonds, an amino group that may be secondarily amidated, or a hydroxy group that may be alkylated or esterified;
   R4 is a straight or branched, saturated or unsaturated C11-C15 alkyl group that may be substituted, wherein the alkyl group may be interrupted by one or more ester bonds, amide bonds, and/or ether bonds, an amino group that may be secondarily amidated, or a hydroxy group that may be alkylated or esterified;
   R5, R6 and R7 are each independent a hydrogen atom, or a sugar chain connected by glycosyl bonds;
   the wave lines each independently refer to a single stereochemistry of either S or R configuration when the bonded carbon is an asymmetric carbon atom; and
   the dotted lines each independently indicate the presence or absence of a bond, and refer to either the Z or E isomer when the bond exists as a double bond.
[2] The C-glycoside glycolipid compound according to [1], in which in formula (I),
   R1 is hydrogen, a halogen, or a hydroxy;
   R2 is a straight or branched, saturated C11-C15 alkyl group that may be substituted, wherein the alkyl group may be optionally interrupted by one or more ester bonds, amide bonds, and/or ether bonds;
   R3 is a straight or branched, saturated C11-C15 alkyl group that may be substituted, wherein the alkyl group may be optionally interrupted by one or more ester bonds, amide bonds, and/or ether bonds;
   R4 is a straight or branched, saturated C11-C15 alkyl group that may be substituted, wherein the alkyl group may be interrupted by one or more ester bonds, amide bonds, and/or ether bonds;
   R5, R6 and R7 are each independent a hydrogen atom, or a sugar chain connected by glycosyl bonds;
   the wave lines each independently refer to a single stereochemistry of either S or R configuration when the bonded carbon is an asymmetric carbon atom; and
   the dotted lines each independently indicate the presence or absence of a bond, and refer to either the Z or E isomer when the bond exists as a double bond.
[3] The C-glycoside glycolipid compound according to [1] or [2], in which in formula (I),
   R1 is hydrogen, a halogen, or a hydroxy;
   R2 is a straight or branched, saturated C11-C15 alkyl group that may be substituted, wherein the alkyl group is not interrupted by any bond;
   R3 is a straight or branched, saturated C11-C15 alkyl group that may be substituted, wherein the alkyl group is not interrupted by any bond;
   R4 is a straight or branched, saturated C11-C15 alkyl group that may be substituted, wherein the alkyl group is not interrupted by any bond;
   R5, R6 and R7 are each independent a hydrogen atom, or a sugar chain connected by glycosyl bonds;
   the wave lines each independently refer to a single stereochemistry of either S or R configuration when the bonded carbon is an asymmetric carbon atom; and
   the dotted lines each independently indicate the presence or absence of a bond, and refer to either the Z or E isomer when the bond exists as a double bond.
[4] The C-glycoside glycolipid compound according to any one of [1] to [3], represented by formula (I''): 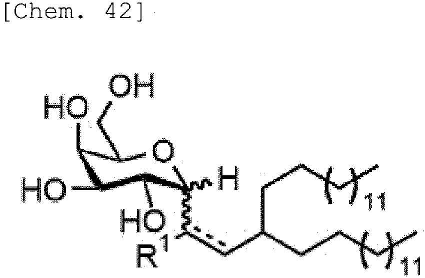 in which
   R1 is hydrogen, a halogen, or a hydroxy group;
   the wave lines each independently refer to a single stereochemistry of either S or R configuration when the bonded carbon is an asymmetric carbon atom; and
   the dotted lines each independently indicate the presence or absence of a bond, and refer to either the Z or E isomer when the bond exists as a double bond.
[5] The C-glycoside glycolipid compound according to any one of [1] to [4], in which R1 is fluoro.
[6] The C-glycoside glycolipid compound according to any one of [1] to [5], in which the dotted lines represent a double bond of Z isomer.

### <C-glycoside disaccharide compounds>

[7] A C-glycoside glycolipid compound represented by formula (II): 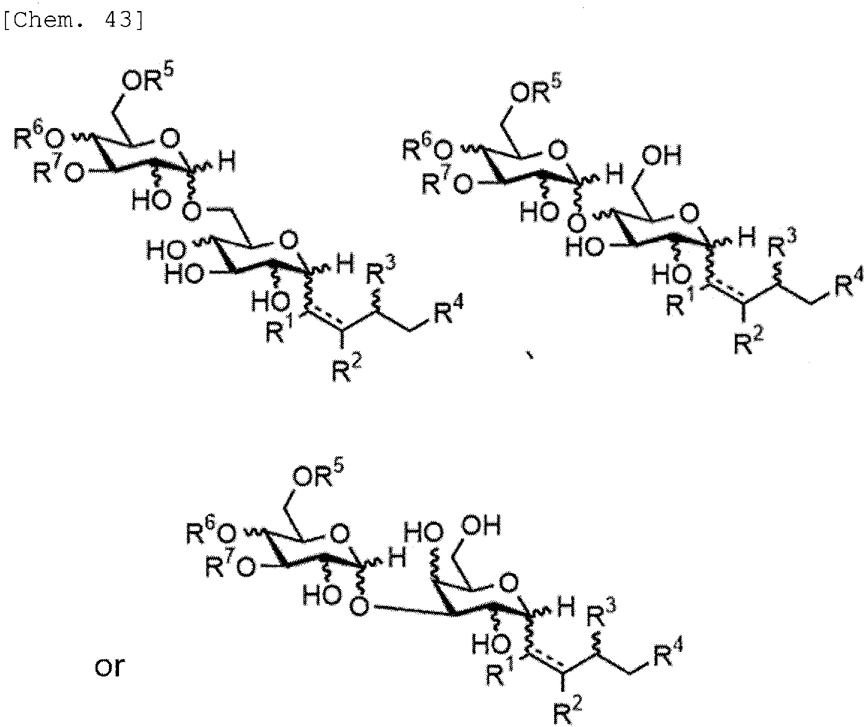 in which
   R1 is hydrogen, a halogen, or a hydroxy;
   R2 is a hydrogen atom, a hydroxy group that may be substituted, or a straight or branched, saturated or unsaturated C11-C15 alkyl group that may be substituted, wherein the alkyl group may be optionally interrupted by one or more ester bonds, amide bonds, and/or ether bonds;
   R3 is a hydrogen atom, a straight or branched, saturated or unsaturated C11-C15 alkyl group that may be substituted, wherein the alkyl group may be optionally interrupted by one or more ester bonds, amide bonds, and/or ether bonds, an amino group that may be secondarily amidated, or a hydroxy group that may be alkylated or esterified;
   R4 is a straight or branched, saturated or unsaturated C11-C15 alkyl group that may be substituted, wherein the alkyl group may be interrupted by one or more ester bonds, amide bonds, and/or ether bonds, an amino group that may be secondarily amidated, or a hydroxy group that may be alkylated or esterified;
   R5, R6 and R7 are each independent a hydrogen atom, or a sugar chain connected by glycosyl bonds;
   the wave lines each independently refer to a single stereochemistry of either S or R configuration when the bonded carbon is an asymmetric carbon atom; and
   the dotted lines each independently indicate the presence or absence of a bond, and refer to either the Z or E isomer when the bond exists as a double bond.
[8] The C-glycoside glycolipid compound according to [7], in which in formula (II),
   R1 is hydrogen, a halogen, or a hydroxy;
   R2 is a straight or branched, saturated C11-C15 alkyl group that may be substituted, wherein the alkyl group may be optionally interrupted by one or more ester bonds, amide bonds, and/or ether bonds;
   R3 is a straight or branched, saturated C11-C15 alkyl group that may be substituted, wherein the alkyl group may be optionally interrupted by one or more ester bonds, amide bonds, and/or ether bonds;
   R4 is a straight or branched, saturated C11-C15 alkyl group that may be substituted, wherein the alkyl group may be interrupted by one or more ester bonds, amide bonds, and/or ether bonds;
   R5, R6 and R7 are each independent a hydrogen atom, or a sugar chain connected by glycosyl bonds;
   the wave lines each independently refer to a single stereochemistry of either S or R configuration when the bonded carbon is an asymmetric carbon atom; and
   the dotted lines each independently indicate the presence or absence of a bond, and refer to either the Z or E isomer when the bond exists as a double bond.
[9] The C-glycoside glycolipid compound according to [7] or [8], in which in formula (II),
   R1 is hydrogen, a halogen, or a hydroxy;
   R2 is a straight or branched, saturated C11-C15 alkyl group that may be substituted, wherein the alkyl group is not interrupted by any bond;
   R3 is a straight or branched, saturated C11-C15 alkyl group that may be substituted, wherein the alkyl group is not interrupted by any bond;
   R4 is a straight or branched, saturated C11-C15 alkyl group that may be substituted, wherein the alkyl group is not interrupted by any bond;
   R5, R6 and R7 are each independent a hydrogen atom, or a sugar chain connected by glycosyl bonds;
   the wave lines each independently refer to a single stereochemistry of either S or R configuration when the bonded carbon is an asymmetric carbon atom; and
   the dotted lines each independently indicate the presence or absence of a bond, and refer to either the Z or E isomer when the bond exists as a double bond.
[10] The C-glycoside glycolipid compound according to any one of [7] to [9], represented by formula (II''): 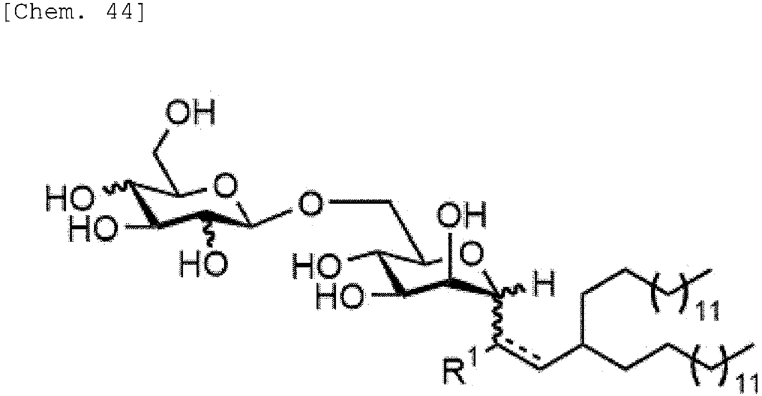 in which
   R1 is hydrogen, a halogen, or a hydroxy group;
   the wave lines each independently refer to a single stereochemistry of either S or R configuration when the bonded carbon is an asymmetric carbon atom; and
   the dotted lines each independently indicate the presence or absence of a bond, and refer to either the Z or E isomer when the bond exists as a double bond.
[11] The C-glycoside glycolipid compound according to any one of [7] to [10], in which R1 is fluoro.
[12] The C-glycoside glycolipid compound according to any one of [7] to [11], in which the dotted lines represent a double bond of Z isomer.

### <LNPs>

[13] A lipid nanoparticle comprising the C-glycoside glycolipid compound according to any one of [1] to [6] and [7] to [12], as well as (a) a nucleic acid molecule, (b) a cationic lipid, (c) an aggregation inhibitor (such as a polyethylene glycol (PEG) lipid or a PEG-modified lipid), and (d) a sterol.

### <Pharmaceutical compositions>

[14] A pharmaceutical composition comprising the lipid nanoparticle according to [13].
[15] The pharmaceutical composition according to [14], which is a vaccine.

## Claims

1. A C-glycoside glycolipid compound represented by formula (I): 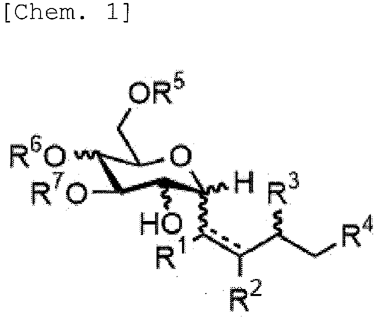 in which
R1 is hydrogen, a halogen, or a hydroxy;
R2 is a hydrogen atom, a hydroxy group that may be substituted, or a straight or branched, saturated or unsaturated C11-C15 alkyl group that may be substituted, wherein the alkyl group may be optionally interrupted by one or more ester bonds, amide bonds, and/or ether bonds;
R3 is a hydrogen atom, a straight or branched, saturated or unsaturated C11-C15 alkyl group that may be substituted, wherein the alkyl group may be optionally interrupted by one or more ester bonds, amide bonds, and/or ether bonds, an amino group that may be secondarily amidated, or a hydroxy group that may be alkylated or esterified;
R4 is a straight or branched, saturated or unsaturated C11-C15 alkyl group that may be substituted, wherein the alkyl group may be interrupted by one or more ester bonds, amide bonds, and/or ether bonds, an amino group that may be secondarily amidated, or a hydroxy group that may be alkylated or esterified;
R5, R6 and R7 are each independent a hydrogen atom, or a sugar chain connected by glycosyl bonds;
the wave lines each independently refer to a single stereochemistry of either S or R configuration when the bonded carbon is an asymmetric carbon atom; and
the dotted lines each independently indicate the presence or absence of a bond, and refer to either the Z or E isomer when the bond exists as a double bond.

2. The C-glycoside glycolipid compound according to claim 1, in which in formula (I),
R1 is hydrogen, a halogen, or a hydroxy;
R2 is a straight or branched, saturated C11-C15 alkyl group that may be substituted, wherein the alkyl group may be optionally interrupted by one or more ester bonds, amide bonds, and/or ether bonds;
R3 is a straight or branched, saturated C11-C15 alkyl group that may be substituted, wherein the alkyl group may be optionally interrupted by one or more ester bonds, amide bonds, and/or ether bonds;
R4 is a straight or branched, saturated C11-C15 alkyl group that may be substituted, wherein the alkyl group may be interrupted by one or more ester bonds, amide bonds, and/or ether bonds;
R5, R6 and R7 are each independent a hydrogen atom, or a sugar chain connected by glycosyl bonds;
the wave lines each independently refer to a single stereochemistry of either S or R configuration when the bonded carbon is an asymmetric carbon atom; and
the dotted lines each independently indicate the presence or absence of a bond, and refer to either the Z or E isomer when the bond exists as a double bond.

3. The C-glycoside glycolipid compound according to claim 2, in which in formula (I),
R1 is hydrogen, a halogen, or a hydroxy;
R2 is a straight or branched, saturated C11-C15 alkyl group that may be substituted, wherein the alkyl group is not interrupted by any bond;
R3 is a straight or branched, saturated C11-C15 alkyl group that may be substituted, wherein the alkyl group is not interrupted by any bond;
R4 is a straight or branched, saturated C11-C15 alkyl group that may be substituted, wherein the alkyl group is not interrupted by any bond;
R5, R6 and R7 are each independent a hydrogen atom, or a sugar chain connected by glycosyl bonds;
the wave lines each independently refer to a single stereochemistry of either S or R configuration when the bonded carbon is an asymmetric carbon atom; and
the dotted lines each independently indicate the presence or absence of a bond, and refer to either the Z or E isomer when the bond exists as a double bond.

4. The C-glycoside glycolipid compound according to claim 3, represented by formula (I''): 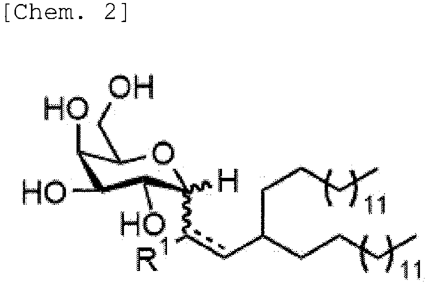 in which
R1 is hydrogen, a halogen, or a hydroxy group;
the wave lines each independently refer to a single stereochemistry of either S or R configuration when the bonded carbon is an asymmetric carbon atom; and
the dotted lines each independently indicate the presence or absence of a bond, and refer to either the Z or E isomer when the bond exists as a double bond.

5. The C-glycoside glycolipid compound according to claim 4, in which R1 is fluoro.

6. The C-glycoside glycolipid compound according to claim 5, in which the dotted lines represent a double bond of Z isomer.

7. A C-glycoside glycolipid compound represented by formula (II): 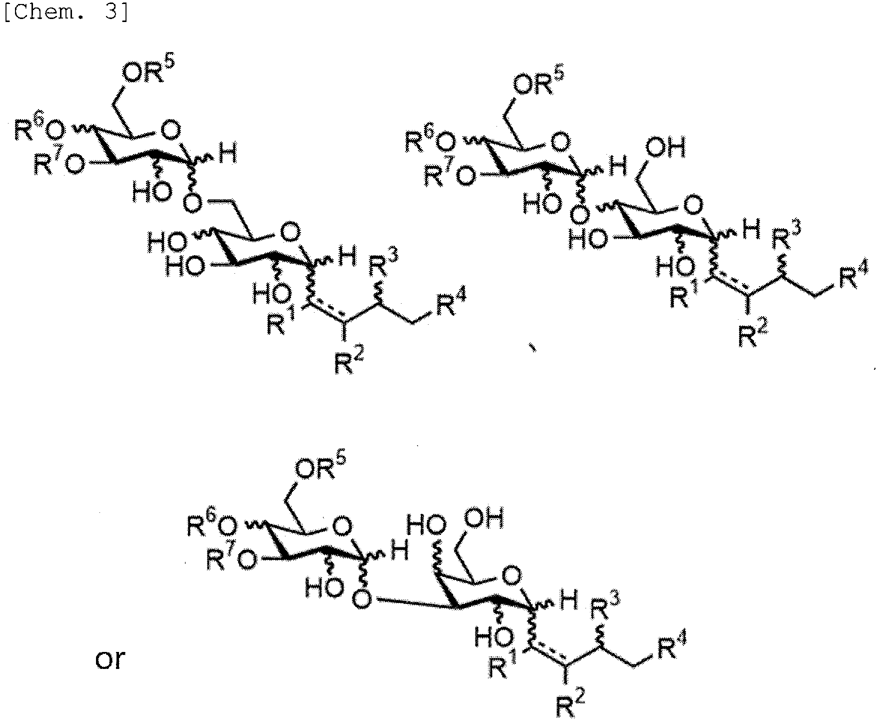 in which
R1 is hydrogen, a halogen, or a hydroxy;
R2 is a hydrogen atom, a hydroxy group that may be substituted, or a straight or branched, saturated or unsaturated C11-C15 alkyl group that may be substituted, wherein the alkyl group may be optionally interrupted by one or more ester bonds, amide bonds, and/or ether bonds;
R3 is a hydrogen atom, a straight or branched, saturated or unsaturated C11-C15 alkyl group that may be substituted, wherein the alkyl group may be optionally interrupted by one or more ester bonds, amide bonds, and/or ether bonds, an amino group that may be secondarily amidated, or a hydroxy group that may be alkylated or esterified;
R4 is a straight or branched, saturated or unsaturated C11-C15 alkyl group that may be substituted, wherein the alkyl group may be interrupted by one or more ester bonds, amide bonds, and/or ether bonds, an amino group that may be secondarily amidated, or a hydroxy group that may be alkylated or esterified;
R5, R6 and R7 are each independent a hydrogen atom, or a sugar chain connected by glycosyl bonds;
the wave lines each independently refer to a single stereochemistry of either S or R configuration when the bonded carbon is an asymmetric carbon atom; and
the dotted lines each independently indicate the presence or absence of a bond, and refer to either the Z or E isomer when the bond exists as a double bond.

8. The C-glycoside glycolipid compound according to claim 7, in which in formula (II),
R1 is hydrogen, a halogen, or a hydroxy;
R2 is a straight or branched, saturated C11-C15 alkyl group that may be substituted, wherein the alkyl group may be optionally interrupted by one or more ester bonds, amide bonds, and/or ether bonds;
R3 is a straight or branched, saturated C11-C15 alkyl group that may be substituted, wherein the alkyl group may be optionally interrupted by one or more ester bonds, amide bonds, and/or ether bonds;
R4 is a straight or branched, saturated C11-C15 alkyl group that may be substituted, wherein the alkyl group may be interrupted by one or more ester bonds, amide bonds, and/or ether bonds;
R5, R6 and R7 are each independent a hydrogen atom, or a sugar chain connected by glycosyl bonds;
the wave lines each independently refer to a single stereochemistry of either S or R configuration when the bonded carbon is an asymmetric carbon atom; and
the dotted lines each independently indicate the presence or absence of a bond, and refer to either the Z or E isomer when the bond exists as a double bond.

9. The C-glycoside glycolipid compound according to claim 8, in which in formula (II),
R1 is hydrogen, a halogen, or a hydroxy;
R2 is a straight or branched, saturated C11-C15 alkyl group that may be substituted, wherein the alkyl group is not interrupted by any bond;
R3 is a straight or branched, saturated C11-C15 alkyl group that may be substituted, wherein the alkyl group is not interrupted by any bond;
R4 is a straight or branched, saturated C11-C15 alkyl group that may be substituted, wherein the alkyl group is not interrupted by any bond;
R5, R6 and R7 are each independent a hydrogen atom, or a sugar chain connected by glycosyl bonds;
the wave lines each independently refer to a single stereochemistry of either S or R configuration when the bonded carbon is an asymmetric carbon atom; and
the dotted lines each independently indicate the presence or absence of a bond, and refer to either the Z or E isomer when the bond exists as a double bond.

10. The C-glycoside glycolipid compound according to claim 9, represented by formula (II''): 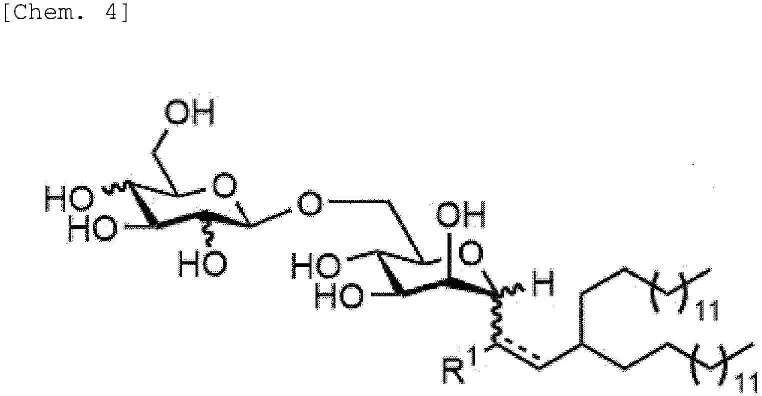 in which
R1 is hydrogen, a halogen, or a hydroxy group;
the wave lines each independently refer to a single stereochemistry of either S or R configuration when the bonded carbon is an asymmetric carbon atom; and
the dotted lines each independently indicate the presence or absence of a bond, and refer to either the Z or E isomer when the bond exists as a double bond.

11. The C-glycoside glycolipid compound according to claim 10, in which R1 is fluoro.

12. The C-glycoside glycolipid compound according to claim 11, in which the dotted lines represent a double bond of Z isomer.

13. A lipid nanoparticle comprising the C-glycoside glycolipid compound according to any one of claims 1 to 6 and claims 7 to 12, as well as (a) a nucleic acid molecule, (b) a cationic lipid, (c) an aggregation inhibitor (such as a polyethylene glycol (PEG) lipid or a PEG-modified lipid), and (d) a sterol.

14. A pharmaceutical composition comprising the lipid nanoparticle according to claim 13.

15. The pharmaceutical composition according to claim 14, which is a vaccine.
